(19) Europäisches Patentamt
European Patent Office
Office européen des brevets

(11) Publication number: **0 461 264 A1**

(12) **EUROPEAN PATENT APPLICATION published in accordance with Art. 158(3) EPC**

(21) Application number: **91900930.8**

(22) Date of filing: **21.12.90**

(86) International application number: **PCT/JP90/01682**

(87) International publication number: **WO 91/09847 (11.07.91 91/15)**

(51) Int. Cl.5: **C07D 211/90**, C07D 401/12, A61K 31/455, A61K 31/55

(30) Priority: **29.12.89 JP 339950/89**

(43) Date of publication of application: **18.12.91 Bulletin 91/51**

(84) Designated Contracting States: **AT BE CH DE DK ES FR GB GR IT LI LU NL SE**

(71) Applicant: **KAKEN PHARMACEUTICAL CO., LTD.**
**No. 28-8, 2-chome, Honkomagome**
**Bunkyo-Ku**
**Tokyo 113(JP)**

(72) Inventor: **NAKANISHI, Michio**
**393-1, Kitahorikawa**
**Nakatsu-shi Oita 871(JP)**
Inventor: **UCHIDA, Katsuhiro**
**336, Gojoagaru Kashiwaya-cho**
**Yanaginobanbadori**
**Shimogyo-ku Kyoto-shi Kyoto 600(JP)**
Inventor: **NAKANO, Jun**
**1180-5, Katsube-cho**
**Moriyama-shi Shiga 524(JP)**
Inventor: **NAGAHARA, Michiko**
**591, Oaza Nagahara Yasa-cho**
**Yasu-gun Shiga 520-23(JP)**
Inventor: **MURAI, Takeshi**
**6-26, Sekizawa 3-chome**
**Fujimi-shi Saitama 354(JP)**
Inventor: **OSADA, Eizaburo**
**3-19, Sizuka-machi**
**Koga-shi Ibaraki 306(JP)**

(74) Representative: **Türk, Gille, Hrabal**
**Brucknerstrasse 20**
**W-4000 Düsseldorf 13(DE)**

(54) **ETHYNYLPHENYL DERIVATIVE, PRODUCTION THEREOF, AND REMEDY FOR DISEASES OF CIRCULATORY ORGANS CONTAINING THE SAME AS ACTIVE INGREDIENT.**

(57) An ethynylphenyl derivative of general formula (I) and pharmacologically acceptable salts thereof: wherein $R^1$ represents hydrogen or halogen; $R^2$ represents lower alkyl or lower alkoxyalkyl; $R^3$ represents methyl, amino, cyano, formyl or dimethoxymethyl; $R^6$ and $R^7$ each represents hydrogen or lower alkyl; Y represents ($\alpha$), wherein $R^4$ and $R^5$ each represents hydrogen, halogen, lower alkyl or lower alkoxy; m is an integer of 2 to 4, provided that ($\beta$) groups may be the same or different from each other; n is an integer of 2 or 3; p is an integer of 0 to 2; and q is an integer of 0 or 1.

EP 0 461 264 A1

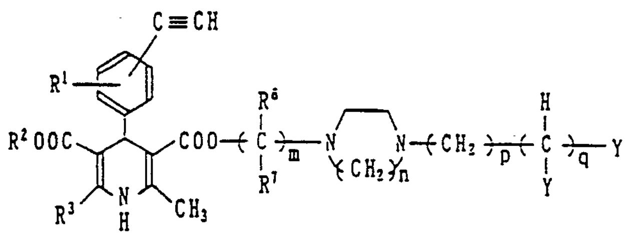

(I)

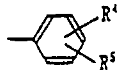 (α)

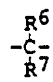 (β)

TECHNICAL FIELD

The present invention relates to ethynylphenyl derivative, process for preparing the same and medicament for circulatory disease containing the same as an effective ingredient.

BACKGROUND ART

Hitherto, many 1,4-dihydropyridine derivatives which are substituted by phenyl group at the 4-position have been known as compounds having pharmacological activities such as antihypertensive activity and vasodilative activity. For example, it is known that dimethyl 2,6-dimethyl-4-(2-nitrophenyl)-1,4-dihydropyridinedicarboxylate (hereinafter referred to as "nifedipine") has strong pharmacological activities such as antihypertensive activity and coronary vasodilative activity (USP 3644627). Also, 2-(N-benzyl-N-methylamino)ethyl, methyl 2,6-dimethyl-4-(3-nitrophenyl)-1,4-dihydropyridinedicarboxylate hydrochloride (hereinafter referred to as "nicardipine") (USP 3985758) and isopropyl, 2-methoxyethyl 2,6-dimethyl-4-(3-nitrophenyl)-1,4-dihydropyridinedicarboxylate (hereinafter referred to as "nimodipine") are widely known.

Most of well-known 1,4-dihydropyridine derivatives are compounds in which the phenyl group at the 4-position of pyridine ring is substituted by nitro group, a halogen atom, and the like. Examples of the compounds in which the phenyl group at the 4-position is substituted by acetylene are few. For example, in the process patent with respect to 4-(2-ethynylphenyl)-2,6-dialkyl-1,4-dihydropyridinedicarboxylic acid ester, an alkyne group is exemplified as the substituent (Japanese Examined Patent Publication No. 12632/1976). However, the compound is not concrete since no example as to the compound is shown. Of course, any physical property and pharmacological activity of the compound are unknown. Also, 4-[2-(2-aryl)ethynyl]-phenyl-2,6-dimethyl-1,4-dihydropyridine-3,5-dicarboxylic acid dialkyl ester is disclosed in Japanese Unexamined Patent Publication No. 252768/1987. However, detailed pharmacological activity is not described. So, it is found to be incomplete as a medicinal invention.

There were made many inventions concerning 1,4-dihydropyridine derivatives besides the above-mentioned. It is understood that the differences among peculiarities in drug efficacy of the compounds of those inventions depend on variation of substituents of the 1,4-dihydropyridine ring. Concretly, the peculiarities in drug efficacy have been found by substituting substituents of phenyl group substituted at the 4-position by substituents such as nitro group, a halogen atom and a haloalkyl group, substituting substituents at the 2-position or the 6-position by substituents such as cyano group, an aminoalkyl group and amino group or substituting substituents at the 3-position or the 5-position with a substituent such as an alkyl group, a substituted alkylester group and amido group. The peculiarities in drug efficacy have also been found by the combination of the above-mentioned positions for substitution with the above-mentioned substituents.

In fact, the differences among peculiarities in pharmacological action are found depending on the position at which nitro group is substituted in phenyl group at the 4-position or a slight change of an alkyl group of an ester side chain at the 3-position or the 5-position. With respect to nifedipine and nimodipine, the position at which nitro group is substituted in phenyl group substituted at the 4-position of dihydropyridine ring is the 2-position in nifedipine and the 3-position in nimodipine. Besides, ester side chains at the 3-position and the 5-position are methoxymethyl and isopropyl groups in nimodipine in contrast to dimethyl groups in nifedipine. However, a difference in central nervous action is observed between nimodipine and nifedipine (Neuropharmacology 28, No. 3 P229-233 (1989)).

On the other hand, as a report concerning a substituent at the 3-position of 1,4-dihydropyridine ring of the compound of the present invention, there is Japanese Unexamined Patent Publication No. 17562/1986 relating to the side chain of piperazylalkyl. For example, the group having the formula (1):

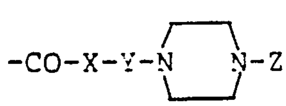

(1)

wherein Y is an alkylene chain having carbon atoms of 2 to 5, an alkyleneoxyalkylene, an alkylenethioalkylene or an alkyleneaminoalkylene chain, Z is phenyl, pyridinyl or pyrimidinyl which is not substituted or can be substituted by one or more substituents selected from a lower alkyl, a lower alkoxy, cyano, a halo- or trifluoromethyl and X is O or NH, is disclosed in Japanese Unexamined Patent Publication No. 17562/1986. However, the substituent of phenyl group substituted at the 4-position of 1,4-dihydropyridine ring is nitro group, halogen atom, cyano group, hydroxy group, alkoxy group, a haloalkyl group, acetoamide

group or methylsulfonyl group, so ethynylphenyl group of the compound of the present invention is not disclosed. Besides, the characteristic of pharmacological activity thereof is to have both calcium antagonistic activity and activity of blocking sympathetic α-adrenergic receptor. So the characteristic of pharmacological activity is different from that of the compound of the present invention.

Further, Japanese Unexamined Patent Publication No. 201765/1983 relating to a substituent of side chain similar to that of the compound of the present invention discloses the group having the formula (2):

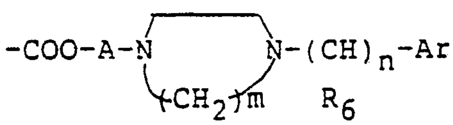 (2)

wherein A is an alkylene, Ar is an aryl or pyridyl, m is an integer of 1 to 3, respectively, and $R_6$ is hydrogen atom, an alkyl, a cycloalkyl, an aralkyl, an aryl or pyridyl.

However, the substituent of phenyl group substituted at the 4-position of 1,4-dihydropyridine ring is hydrogen atom, a halogen atom, nitro group, trifluoromethyl group, an alkyl group, a cycloalkyl group, an alkoxy group, cyano group, an alkoxycarbonyl group or an alkylthio group, so, ethynylphenyl group of the compound of the present invention is not disclosed. Besides, angiotensin antagonistic activity as a preferable characteristic of a medicament for circulatory disease is not disclosed as to a characteristic of pharmacological activity.

That is, there is nothing which allows expection or suggestion of the peculiarity of the pharmacological activity of the compounds of the present invention in prior arts.

Nifedipine and nimodipine which are representative compounds of the conventional 1,4-dihydropyridine compounds have remarkable antihypertensive activity, but there are defects in the persistence of the activity. Vasodepressor having activity of blocking α-adrenergic receptor has a defect of having orthostatic hypotension as a side effect in connection with antihypertensive activity. On the other hand, it is known that angiotensin acts directly on peripheral blood vein and also expresses hypertensive activity on central nervous system. Accordingly, the compound having angiotensin antagonistic activity may be expected to have preventive effect on cardiac failure as a preferable characteristic of a vasodepressor as well as antihypertensive activity. Generally, side effects come to be problems in case of using a compound of an invention as a medicament. Phenytoin which is a medicament of hydantoin group having anticonvulsive activity, calcium antagonist having anticonvulsive activity and the like are reported to express gingival hyperplasia as a side effect.

The compounds of the present invention have persistent antihypertensive activity and have no side effects such as bradycardia because they have small inhibitory action on atrioventricular conduction system. Also the compounds of the present invention don't express side effects such as gingival hyperplasia and have angiotensin antagonistic activity which is a preferable activity as a medicament for circulatory disease. The present invention is directed to provide ethynylphenyl derivative which is useful for treatment for circulatory disease, medicament for circulatory disease containing the said compound as an effective ingredient having treatment effect on hypertension, stenocardia, cardiac failure and/or peripheral circulatory disease or process for preparing the said compound.

DISCLOSURE OF THE INVENTION

As the result of the continuous effort and detailed investigation of the present inventors, an ethynylphenyl derivative having the formula (I):

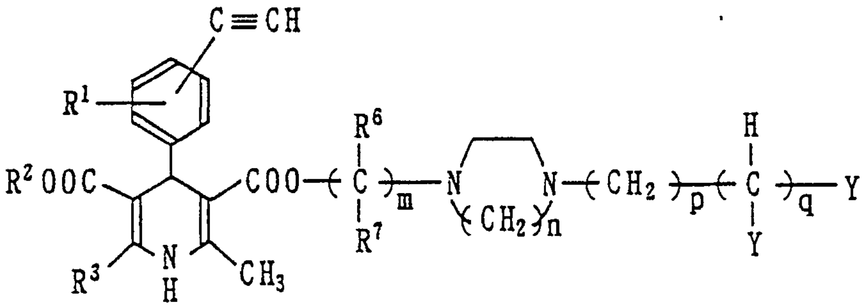

(I)

wherein $R^1$ is hydrogen atom or a halogen atom, $R^2$ is a lower alkyl group or a lower alkoxyalkyl group, $R^3$ is methyl group, amino group, cyano group, formyl group or dimethoxymethyl group, Y is the formula:

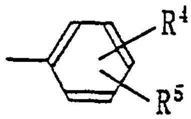

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

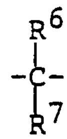

can be the same or different from each other, n is an integer of 2 or 3, p is an integer of 0 to 2 and q is an integer of 0 or 1, or a pharmacologically acceptable salt thereof was successfully synthesized. Besides, it was found that this derivative had calcium antagonistic activity, superior antihypertensive activity, activity of increasing cardiac coronary blood flow, activity of increasing cerebral blood flow and activity of inhibiting angiotensin II. Further it was found that this derivative was useful as a medicament of, for example, vasodepressor, coronary vasodilator, preventive and therapeutic agent for cardiac failure, cerebral blood flow increasing agent, phosphodiesterase inhibitor and the like, and the derivative was highly safe. Consequently, the present invention has been accomplished.

That is, the present invention relates to ① an ethynylphenyl derivative having the formula (I):

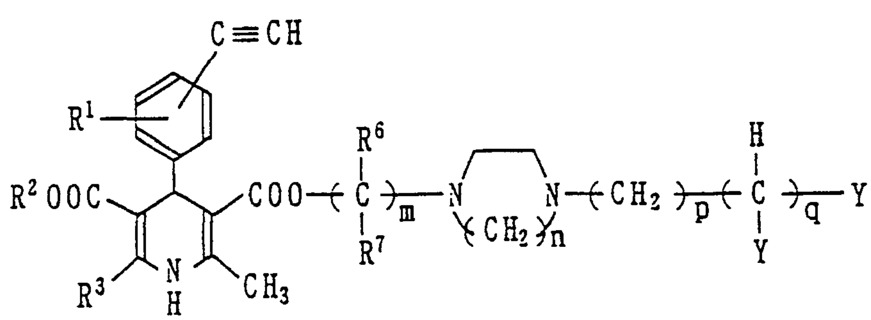

(I)

wherein $R^1$ is hydrogen atom or a halogen atom, $R^2$ is a lower alkyl group or a lower alkoxyalkyl group, $R^3$ is methyl group, amino group, cyano group, formyl group or dimethoxymethyl group, Y is the formula:

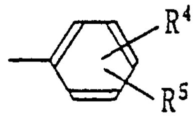

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

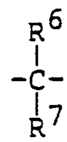

can be the same or different from each other, n is an integer of 2 or 3, p is an integer of 0 to 2 and q is an integer of 0 or 1, or a pharmacologically acceptable salt thereof, ② a process for preparing an ethynylphenyl derivative having the formula (Ia):

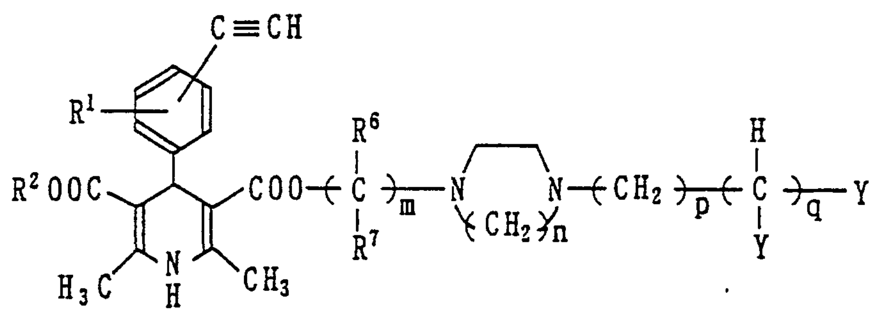

(I a)

wherein $R^1$ is hydrogen atom or a halogen atom, $R^2$ is a lower alkyl group or a lower alkoxyalkyl group, Y is the formula:

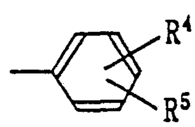

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

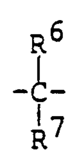

can be the same or different from each other, n is an integer of 2 or 3, p is an integer of 0 to 2 and q is an integer of 0 or 1, or a pharmacologically acceptable salt thereof, which comprises reacting an ethynylbenzaldehyde derivative having the formula (II):

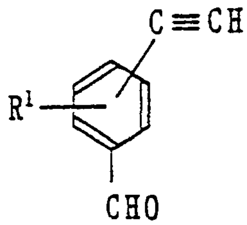

(II)

wherein $R^1$ is hydrogen atom or a halogen atom, a ketoester derivative having the formula (III):

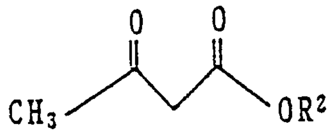

(III)

wherein $R^2$ is a lower alkyl group or a lower alkoxyalkyl group and an aminocrotonic acid derivative having the formula (IV):

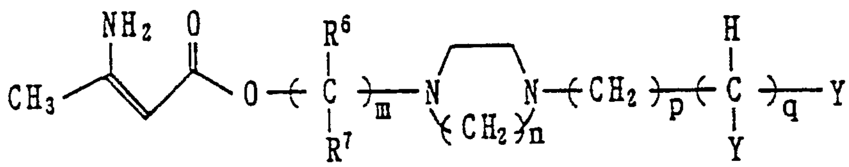

(IV)

wherein Y is the formula:

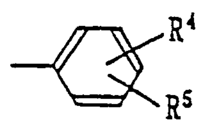

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

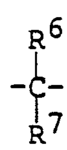

can be the same or different from each other, n is an integer of 2 or 3, p is an integer of 0 to 2 and q is an integer of 0 or 1, in an organic solvent, ③ a process for preparing an ethynylphenyl derivative having the formula (Ia):

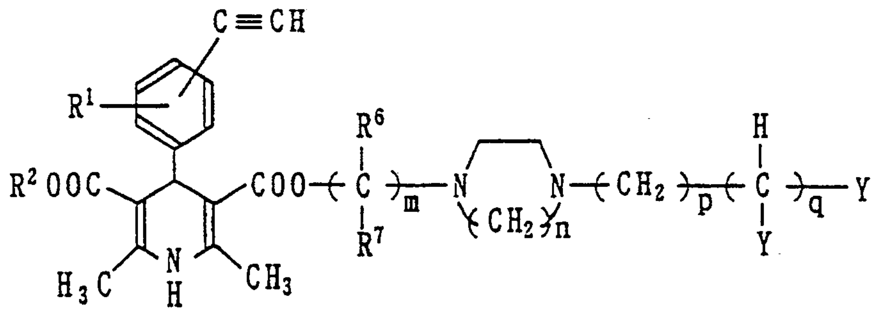

(I a)

wherein $R^1$ is hydrogen atom or a halogen atom, $R^2$ is a lower alkyl group or a lower alkoxyalkyl group, Y is the formula:

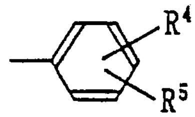

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

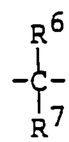

can be the same or different from each other, n is an integer of 2 or 3, p is an integer of 0 to 2 and q is an integer of 0 or 1, or a pharmacologically acceptable salt thereof, which comprises reacting an ethynylbenzaldehyde derivative having the formula (II):

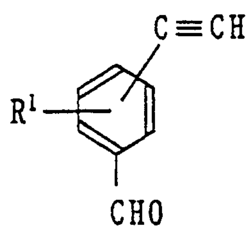

(II)

wherein $R^1$ is hydrogen atom or a halogen atom, an aminocrotonic acid derivative having the formula (V):

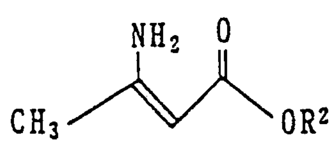

(V)

wherein $R^2$ is a lower alkyl group or a lower alkoxyalkyl group and a keto-ester derivative having the formula (VI):

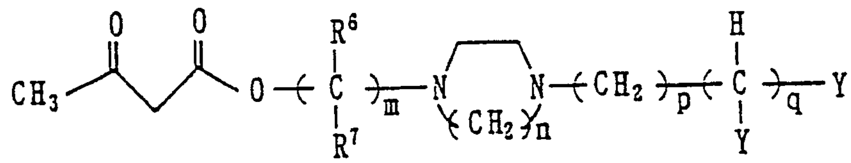

(VI)

wherein Y is the formula:

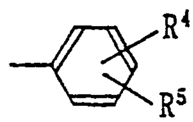

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

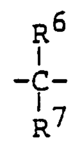

can be the same or different from each other, n is an integer of 2 or 3, p is an integer of 0 to 2 and q is an integer of 0 or 1, in an organic solvent, ④ a process for preparing an ethynylphenyl derivative having the formula (Ia):

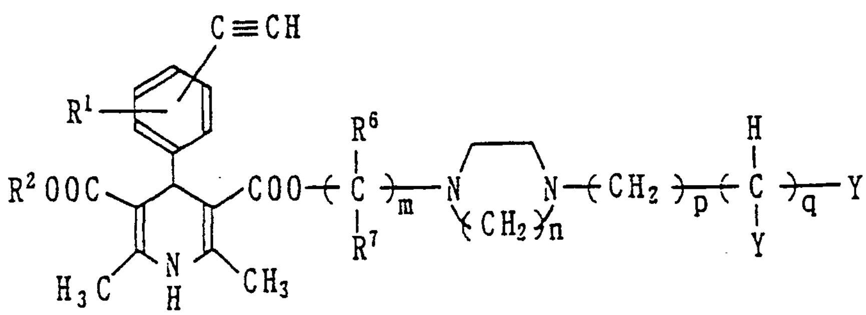

(I a)

wherein $R^1$ is hydrogen atom or a halogen atom, $R^2$ is a lower alkyl group or a lower alkoxyalkyl group, Y is the formula:

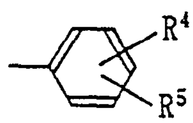

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

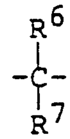

can be the same or different from each other, n is an integer of 2 or 3, p is an integer of 0 to 2 and q is an integer of 0 or 1, or a pharmacologically acceptable salt thereof, which comprises reacting a benzylidene derivative having the formula (VII):

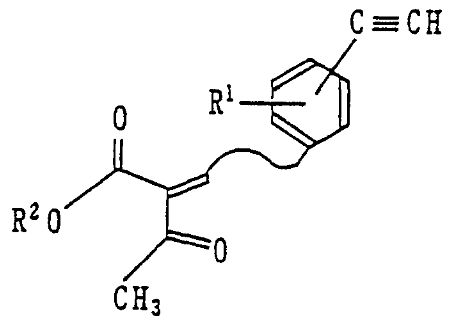

(VII)

wherein $R^1$ is hydrogen atom or a halogen atom and $R^2$ is a lower alkyl group or a lower alkoxyalkyl group and an aminocrotonic acid derivative having the formula (IV):

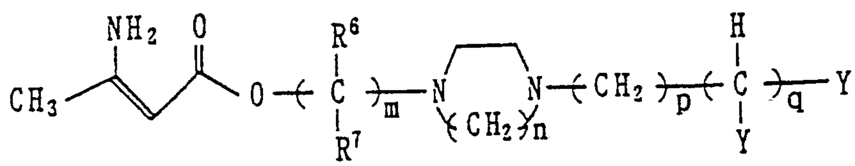

(IV)

wherein Y is the formula:

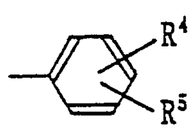

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

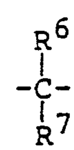

can be the same or different from each other, n is an integer of 2 or 3, p is an integer of 0 to 2 and q is an integer of 0 or 1, in a suitable organic solvent, ⑤ a process for preparing an ethynylphenyl derivative having the formula (Ia):

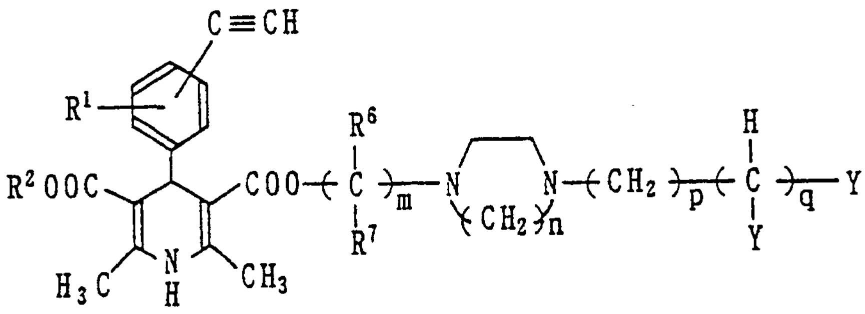

(I a)

wherein $R^1$ is hydrogen atom or a halogen atom, $R^2$ is a lower alkyl group or a lower alkoxyalkyl group, Y is the formula:

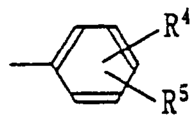

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

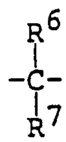

can be the same or different from each other, n is an integer of 2 or 3, p is an integer of 0 to 2 and q is an integer of 0 or 1, or a pharmacologically acceptable salt thereof, which comprises reacting an aminocrotonic acid derivative having the formula (V):

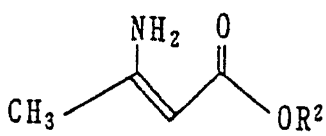

(V)

wherein $R^2$ is a lower alkyl group or a lower alkoxyalkyl group and a benzylidene derivative having the formula (VIII):

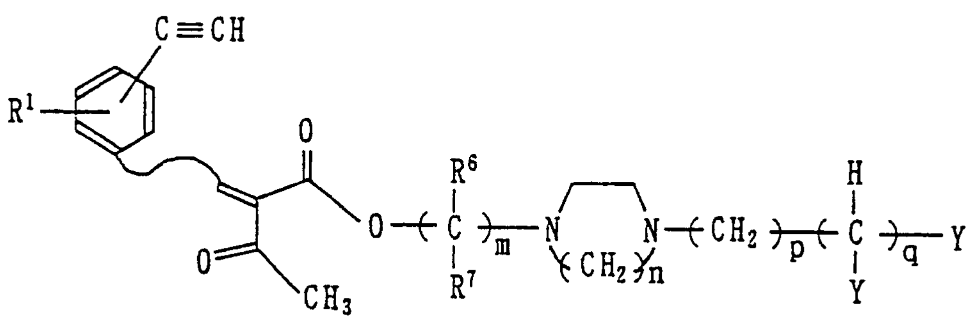

(VIII)

wherein $R^1$ is hydrogen atom or a halogen atom, Y is the formula:

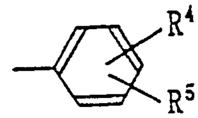

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

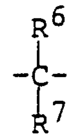

can be the same or different from each other, n is an integer of 2 or 3, p is an integer of 0 to 2 and q is an integer or 0 or 1, in a suitable organic solvent, ⑥ a process for preparing an ethynylphenyl derivative having the formula (Ib):

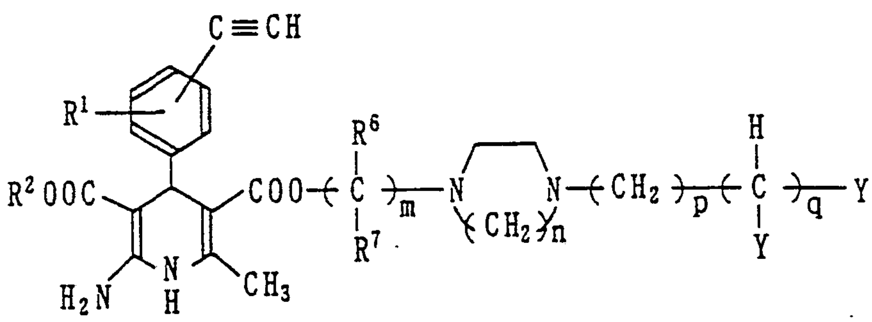

(I b)

wherein $R^1$ is hydrogen atom or a halogen atom, $R^2$ is a lower alkyl group or a lower alkoxyalkyl group, Y is the formula:

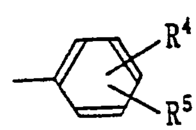

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

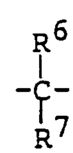

can be the same or different from each other, n is an integer of 2 or 3, p is an integer of 0 to 2 and q is an integer of 0 or 1, or a pharmacologically acceptable salt thereof, which comprises reacting an ethynylbenzaldehyde derivative having the formula (II):

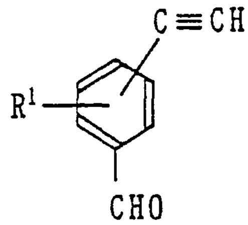

(II)

wherein $R^1$ is hydrogen atom or a halogen atom, an amidine derivative having the formula (IX):

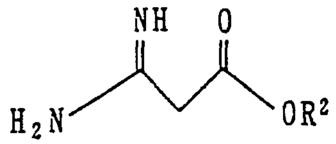

(IX)

wherein $R^2$ is a lower alkyl group or a lower alkoxyalkyl group and a keto-ester derivative having the formula (VI):

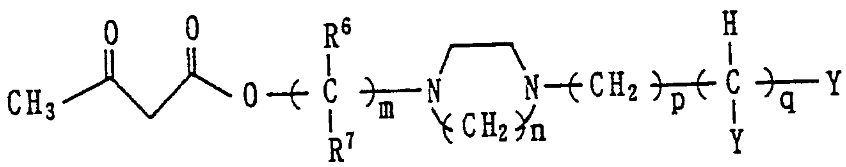

(VI)

wherein Y is the formula:

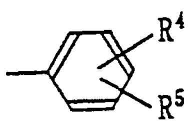

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

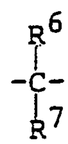

can be the same or different from each other, n is an integer of 2 or 3, p is an integer of 0 to 2 and q is an integer of 0 or 1, in an organic solvent, ⑦ a process for preparing an ethynylphenyl derivative having the formula (Ib):

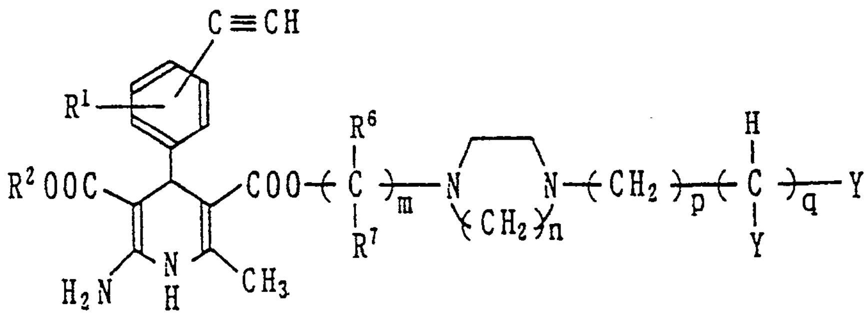

(I b)

wherein $R^1$ is hydrogen atom or a halogen atom, $R^2$ is a lower alkyl group or a lower alkoxyalkyl group, Y is the formula:

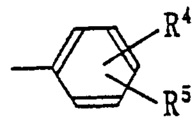

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

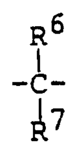

can be the same or different from each other, n is an integer of 2 or 3, p is an integer of 0 to 2 and q is an integer of 0 or 1, or a pharmacologically acceptable salt thereof, which comprises reacting an amidine derivative having the formula (IX):

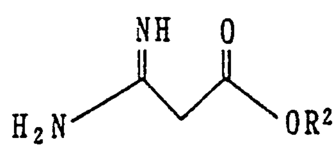

(IX)

wherein $R^2$ is a lower alkyl group or a lower alkoxyalkyl group and a benzylidene derivative having the formula (VIII):

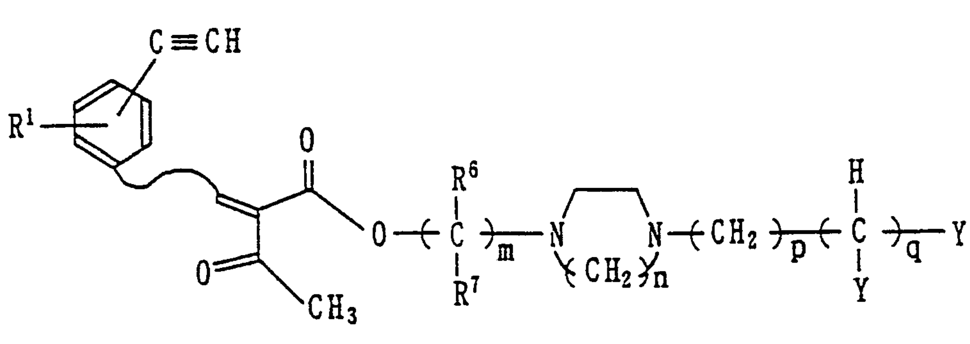

(VIII)

wherein $R^1$ is hydrogen atom or a halogen atom, Y is the formula:

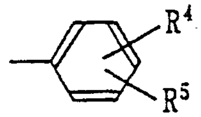

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

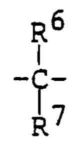

can be the same or different from each other, n is an integer of 2 or 3, p is an integer of 0 to 2 and q is an integer of 0 or 1, in a suitable organic solvent, ⑧ a process for preparing an ethynylphenyl derivative having the formula (Ic):

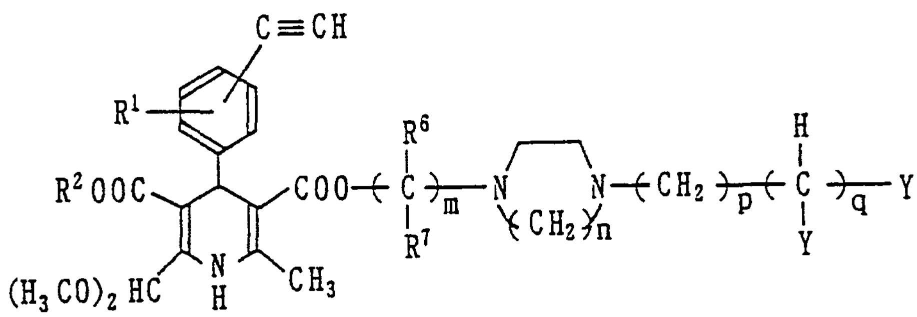

(I c)

wherein $R^1$ is hydrogen atom or a halogen atom, $R^2$ is a lower alkyl group or a lower alkoxyalkyl group, Y is the formula:

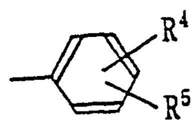

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

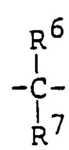

can be the same or different from each other, n is an integer of 2 or 3, p is an integer of 0 to 2 and q is an integer of 0 or 1, or a pharmacologically acceptable salt thereof, which comprises reacting an ethynylbenzaldehyde having the formula (II):

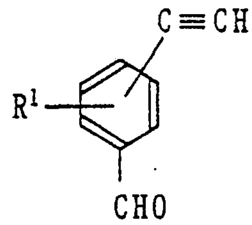

(II)

wherein $R^1$ is hydrogen atom or a halogen atom, an aminocrotonic acid derivative having the formula (IV):

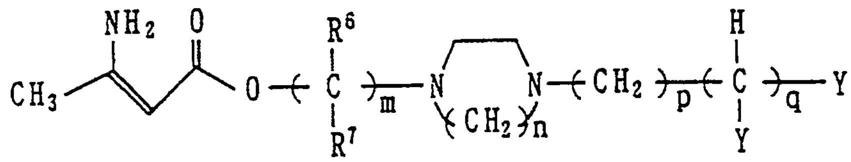

(IV)

wherein Y is the formula:

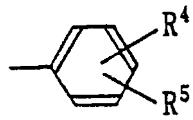

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

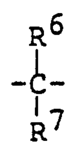

can be the same or different from each other, n is an integer of 2 or 3, p is an integer of 0 to 2 and q is an integer of 0 or 1, and an acetal keto-ester derivative having the formula (X):

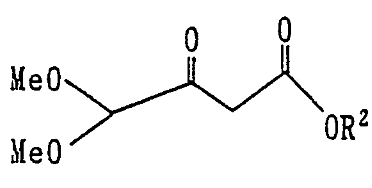

(X)

wherein $R^2$ is a lower alkyl group or a lower alkoxyalkyl group in a suitable organic solvent, ⑨ a process for preparing an ethynylphenyl derivative having the formula (Ic):

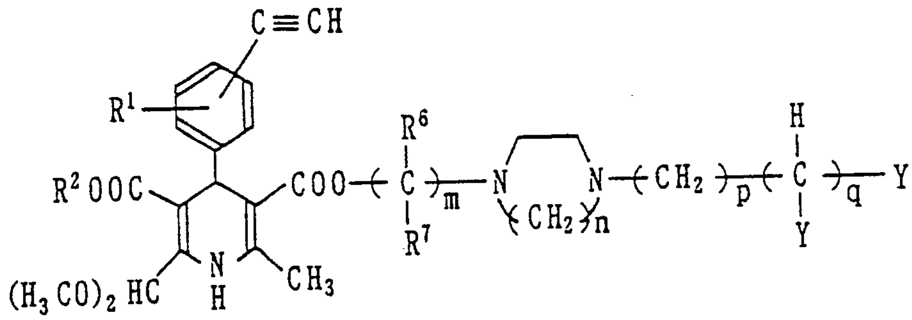

(I c)

wherein $R^1$ is hydrogen atom or a halogen atom, $R^2$ is a lower alkyl group or a lower alkoxyalkyl group, Y is the formula:

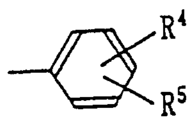

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

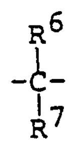

can be the same or different from each other, n is an integer of 2 or 3, p is an integer of 0 to 2 and q is an integer of 0 or 1, or a pharmacologically acceptable salt thereof, which comprises reacting a benzylidene derivative having the formula (XI):

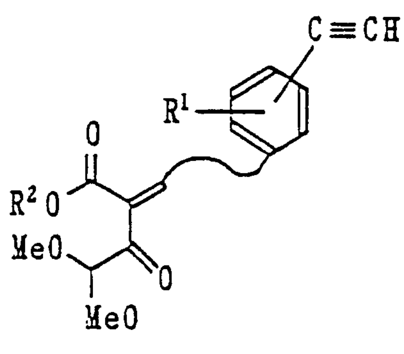

(XI)

wherein $R^1$ is hydrogen atom or a halogen atom and $R^2$ is a lower alkyl group or a lower alkoxyalkyl group and an aminocrotonic acid having the formula (IV):

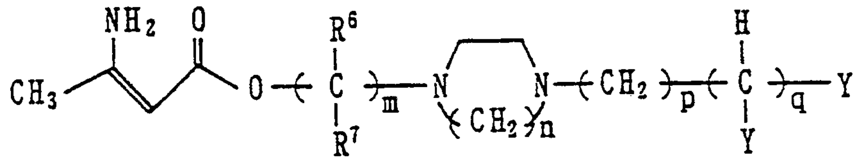

(IV)

wherein Y is the formula:

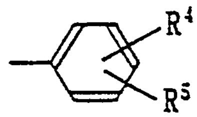

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

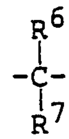

can be the same or different from each other, n is an integer of 2 or 3, p is an integer of 0 to 2 and q is an integer of 0 or 1, in a suitable organic solvent, ⑩ a process for preparing an ethynylphenyl derivative having the formula (Id):

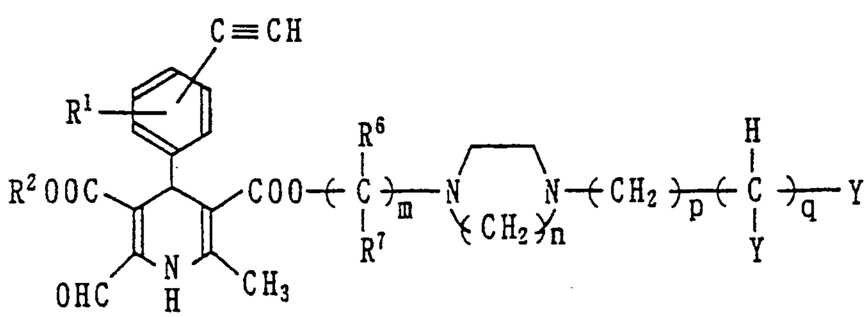

(I d)

wherein $R^1$ is hydrogen atom or a halogen atom, $R^2$ is a lower alkyl group or a lower alkoxyalkyl group, Y is the formula:

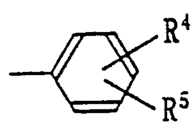

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

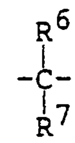

can be the same or different from each other, n is an integer of 2 or 3, p is an integer of 0 to 2 and q is an integer of 0 or 1, or a pharmacologically acceptable salt thereof, which comprises hydrolyzing an ethynylphenyl derivative having the formula (Ic):

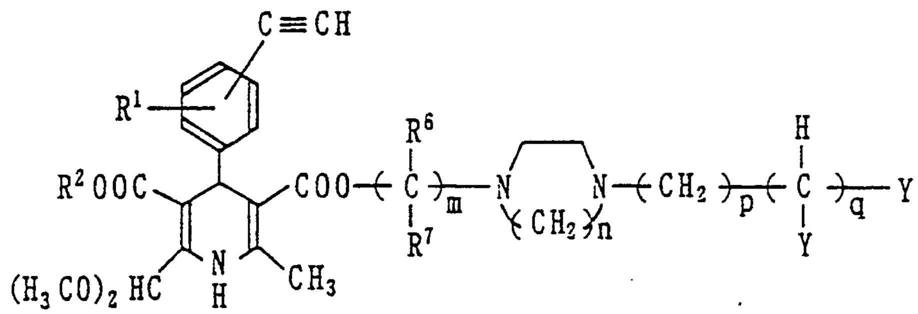

(I c)

wherein $R^1$ is hydrogen atom or a halogen atom, $R^2$ is a lower alkyl group or a lower alkoxyalkyl group, Y is the formula:

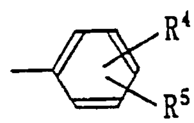

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

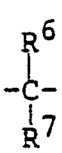

can be the same or different from each other, n is an integer of 2 or 3, p is an integer of 0 to 2 and q is an integer of 0 or 1, ⑪ a process for preparing an ethynylphenyl derivative having the formula (Ie):

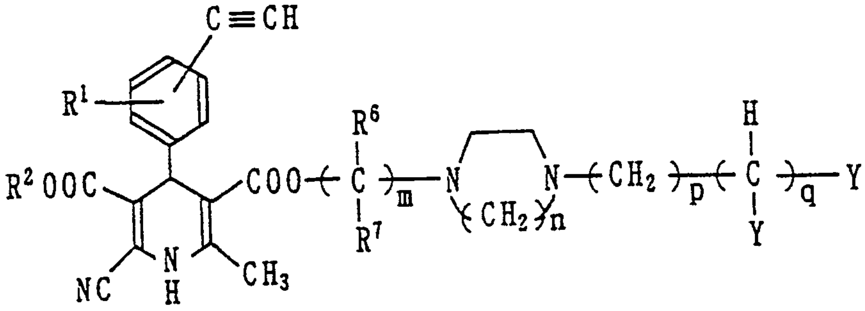

(I e)

wherein $R^1$ is hydrogen atom or a halogen atom, $R^2$ is a lower alkyl group or a lower alkoxyalkyl group, Y is the formula:

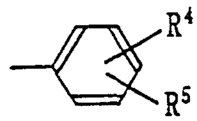

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

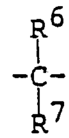

can be the same or different from each other, n is an integer of 2 or 3, p is an integer of 0 to 2 and q is an integer of 0 or 1, or a pharmacologically acceptable salt thereof, which comprises converting an ethynylphenyl derivative having the formula (Id):

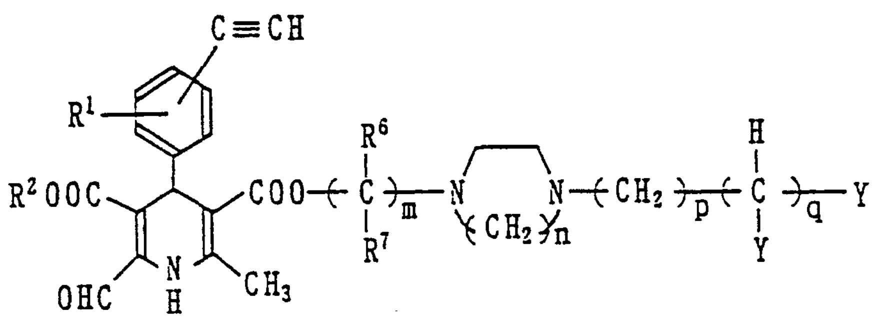

(I d)

wherein $R^1$ is hydrogen atom or a halogen atom, $R^2$ is a lower alkyl group or a lower alkoxyalkyl group, Y is the formula:

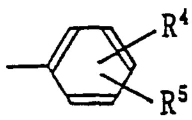

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

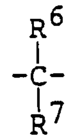

can be the same or different from each other, n is an integer of 2 or 3, p is an integer of 0 to 2 and q is an integer of 0 or 1, into an oxime and subsequently subjecting to dehydration, ⑫ a medicament for circulatory disease which comprises the above-mentioned ethynylphenyl derivative or a pharmacologically acceptable salt thereof as an effective ingredient and ⑬ a medicament for circulatory disease which comprises an effective amount of the above-mentioned ethynylphenyl derivative or a pharmacologically acceptable salt thereof and pharmacologically acceptable additives.

The ethynylphenyl derivative (I) of the present invention has a peculiar structure in comparison with calcium antagonists which are conventionally known in the concrete and has a peculiar activity due to its peculiarity of the structure. That is, since the compound having the formula (I) of the present invention and acid addition salt thereof show weak action against atrioventricular conduction system and increase coronary blood flow, they have great characteristics such as giving less stress on heart, continuing antihyperlensive activity, increasing cerebral blood flow and having no action against central nervous system. As a halogen atom represented by $R^1$ in the above-mentioned formula (I), either atom of F, Cℓ, Br, or I, in particular, preferably F or Cℓ, can be exemplified. The halogen atom can be substituted at any position of the ring, preferably at the 2, 3 or 6-position. The binding position of ethynyl group at phenyl ring substituted at the 4-position of dihydropyridine ring is any of the 2, 3, 5 or 6-position, preferably 3-position.

A lower alkyl group represented by $R^2$ is any of a straight chain, a branched chain and a ring, and number of carbon atom is preferably 1 to 6, for example, methyl, ethyl, propyl, isopropyl, buthyl, isobutyl, sec-butyl, t-butyl, pentyl, isopentyl, neopentyl, hexyl, cyclopropyl, cyclobutyl, cyclopentyl, cyclohexyl and the like. As to a lower alkoxyalkyl group, the group which consists of an alkoxy group having 1 to 3 carbon atoms and an alkyl group having 1 to 3 carbon atoms, for example, ethoxymethyl, methoxyethyl, ethoxyethyl, propoxymethyl, propoxyethyl or the like can be exemprefied. $R^4$ and $R^5$ can be the same or different, and can be substituted at any position of the ring, preferably at the 2- or 4-position. Halogen atoms represented by $R^4$ and $R^5$ are any atom of F, Cℓ, Br and I. And alkoxy groups represented by $R^4$ and $R^5$ are lower alkoxy groups having 1 to 5 carbon atoms, for example, methoxy, ethoxy, propoxy, isopropoxy, butyloxy, isobutyloxy, sec-butyloxy, t-butyloxy, pentyloxy, isopentyloxy, neopentyloxy and the like. Methoxy and ethoxy are, in particular, preferable.

Alkyl groups represented by $R^4$ and $R^5$ are lower alkyl groups having 1 to 4 carbon atoms, for example, methyl, ethyl, propyl, isopropyl, butyl, isobutyl, t-butyl and the like. Methyl and ethyl are, in particular, preferable.

Alkyl group represented by $R^6$ and $R^7$ are lower alkyl groups having 1 to 3 carbon atoms, for example, methyl, ethyl, propyl, isopropyl and the like. Methyl and ethyl are, in particular, prefereable.

The ring is piperazine ring when n is 2 and homopiperazine ring when n is 3.

When both p and q are 0, substituted phenyl group is directly bound to piperazine ring or homopiperazine ring. When p is 0 and q is 1, methyl group substituted with two substituted phenyl groups is directly bound to piperazine ring or homopiperazine ring.

When p is 1 or 2 and q is 0, one substituted phenyl group is bound to piperazine ring or homopiperazine ring via methylene group or ethylene group.

When p is 1 and q is 1, methyl group substituted with two substituted phenyl groups is bound to piperazine ring or homopiperazine ring via methylene group.

As the compound having the above-mentioned formula (I) obtained according to the present invention, the compounds listed in Table 1 can be exemplified.

Table 1

| Com. No. | position*1 | $R^1$ | $R^2$ | $R^3$ | m | n | p | q | Y | $R^6$ | $R^7$ |
|---|---|---|---|---|---|---|---|---|---|---|---|
| 1 | 3 | H | Me*2 | Me | 2 | 2 | 0 | 1 | 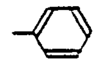 | H | H |
| 2 | 2 | 6-F*8 | Me | Me | 2 | 2 | 0 | 1 | 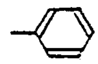 | H | H |
| 3 | 2 | H | Me | Me | 2 | 2 | 0 | 1 | 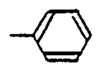 | H | H |

- continued -

| Com. No. | position$^{*1}$ | $R^1$ | $R^2$ | $R^3$ | m | n | p | q | Y | $R^6$ | $R^7$ |
|---|---|---|---|---|---|---|---|---|---|---|---|
| 4 | 3 | H | Me | Me | 2 | 2 | 0 | 1 | 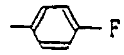 | H | H |
| 5 | 3 | H | Me | Me | 2 | 2 | 0 | 1 | 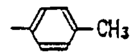 | H | H |
| 6 | 2 | 3-Cl | Me | Me | 2 | 2 | 0 | 1 | 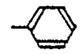 | H | H |
| 7 | 3 | H | Me | Me | 3 | 2 | 0 | 1 | 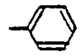 | H | H |
| 8 | 3 | H | Me | Me | 4 | 2 | 0 | 1 | 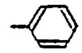 | H | H |
| 9 | 3 | H | iPr$^{*4}$ | Me | 2 | 2 | 0 | 1 | 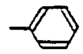 | H | H |
| 10 | 3 | H | nPr$^{*5}$ | Me | 2 | 2 | 0 | 1 | 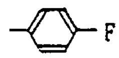 | H | H |
| 11 | 3 | H | iPr | Me | 3 | 2 | 0 | 1 | 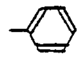 | H | H |
| 12 | 3 | H | cHe$^{*6}$ | Me | 2 | 2 | 0 | 1 | 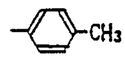 | H | H |
| 13 | 3 | H | Me | Me | 2 | 2 | 2 | 1 | 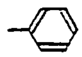 | H | H |
| 14 | 3 | H | $-CH_2CH_2OMe^{*7}$ | Me | 2 | 2 | 0 | 1 | 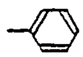 | H | H |
| 15 | 3 | H | Me | Me | 2 | 3 | 0 | 1 | 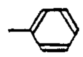 | H | H |
| 16 | 3 | H | Me | Me | 2 | 3 | 0 | 1 | 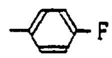 | H | H |
| 17 | 3 | 2-Cl | Me | Me | 2 | 2 | 0 | 1 | 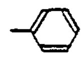 | H | H |
| 18 | 3 | H | Me | Me | 3 | 2 | 0 | 0 | 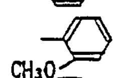 | H | H |
| 19 | 3 | H | Me | Me | 3 | 2 | 0 | 0 | 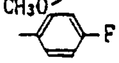 | H | H |
| 20 | 2 | 6-F | Me | Me | 3 | 2 | 0 | 0 | 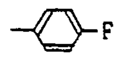 | H | H |
| 21 | 3 | H | Me | Me | 2 | 2 | 1 | 0 | 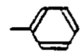 | H | H |
| 22 | 3 | H | Me | Me | 2 | 2 | 1 | 0 | 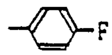 | H | H |
| 23 | 3 | H | Me | Me | 2 | 2 | 1 | 0 | 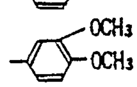 | H | H |
| 24 | 3 | H | Me | Me | 2 | 2 | 2 | 0 | 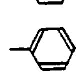 | H | H |
| 25 | 2 | 6-F | Me | Me | 3 | 2 | 1 | 0 | 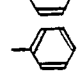 | H | H |
| 26 | 3 | H | Et$^{*3}$ | $NH_2$ | 2 | 2 | 0 | 1 | 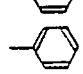 | H | H |

- continued -

| Com. No. | posi-tion*1 | $R^1$ | $R^2$ | $R^3$ | m | n | p | q | Y | $R^6$ | $R^7$ |
|---|---|---|---|---|---|---|---|---|---|---|---|
| 27 | 3 | H | nPr | $NH_2$ | 2 | 2 | 0 | 1 | 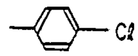 | H | H |
| 28 | 3 | H | $-CH_2CH_2OMe$ | $NH_2$ | 2 | 2 | 0 | 1 | 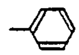 | H | H |
| 29 | 2 | 6-F | Me | $NH_2$ | 2 | 2 | 0 | 1 | 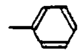 | H | H |
| 30 | 2 | 6-F | Me | $NH_2$ | 2 | 2 | 1 | 0 | 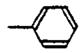 | H | H |
| 31 | 3 | H | Me | $-CH(OCH_3)_2$ | 2 | 2 | 0 | 1 | 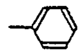 | H | H |
| 32 | 3 | H | Me | CHO | 2 | 2 | 0 | 1 | 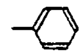 | H | H |
| 33 | 3 | H | Me | CN | 2 | 2 | 0 | 1 | 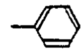 | H | H |
| 34 | 3 | H | iPr | $-CH(OCH_3)_2$ | 2 | 2 | 0 | 1 | 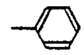 | H | H |
| 35 | 3 | H | iPr | CHO | 2 | 2 | 0 | 1 | 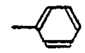 | H | H |
| 36 | 3 | H | iPr | CN | 2 | 2 | 0 | 1 | 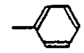 | H | H |
| 37 | 3 | H | Me | Me | 2*9 | 2 | 0 | 1 | 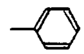 | *9 | *9 |
| 38 | 3 | H | Me | Me | 2*10 | 2 | 0 | 1 | 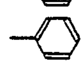 | *10 | *10 |

[Notes] *1: Position at which ethynyl group is substituted in phenyl ring at 4-position

*2: Methyl group

*3: Ethyl group

*4: Isopropyl group

*5: n-Propyl group

*6: Cyclohexyl group

*7: Methoxyethyl group

*8: A numeral described in front of a halogen atom of $R^1$: substituted position at phenyl ring at the 4-position

*9: The part of 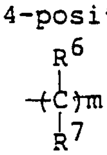 is 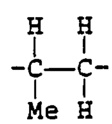

*10: The part of 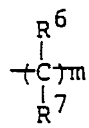 is 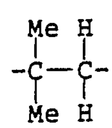

The ethynylphenyl derivative having the above-mentioned formula (I) of the present invention can be prepared by reacting an optional part and the residual part which form said ethynylphenyl derivative, according to a known means, in particular, subjecting to a reaction of dehydration and ring closure. For example, prepared as follows.

(Process for preparation)

Process A-1

The compound having the formula (Ia) wherein $R^3$ is methyl group can be obtained by Hantzsch synthesis method according to the reaction formula (a) or (a').

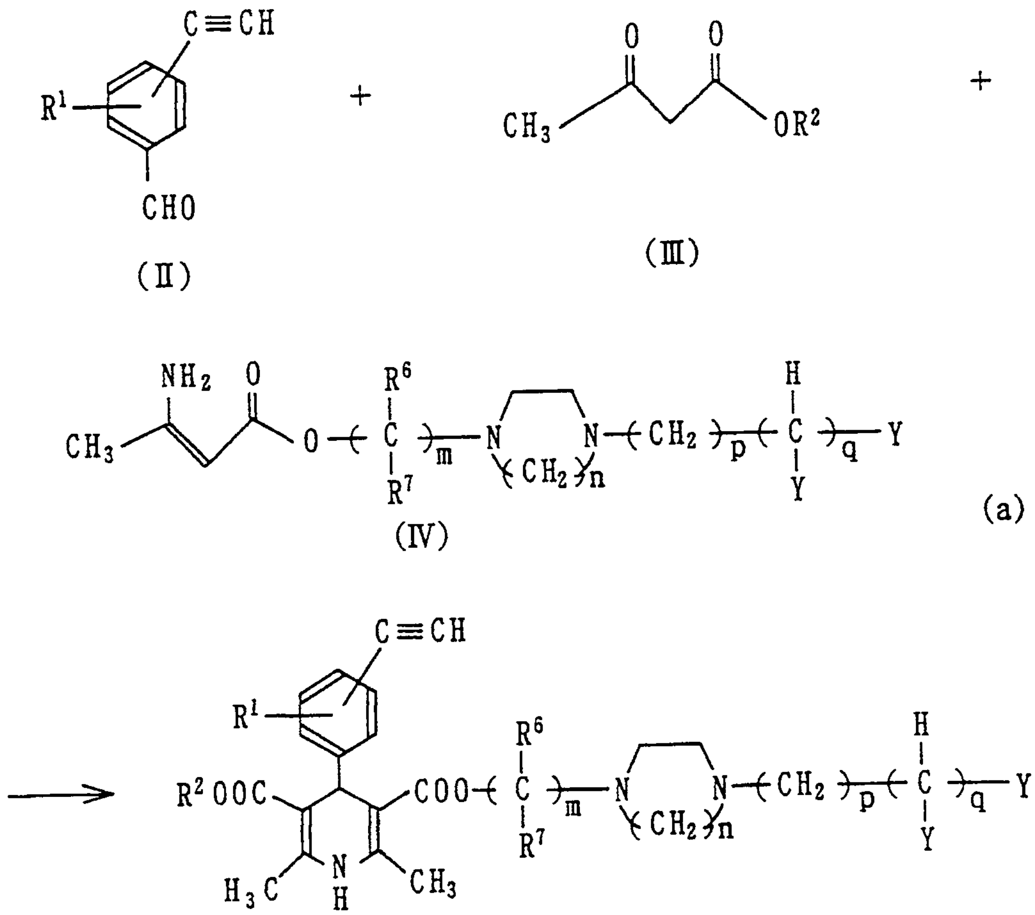

(II) (III) (IV) (a) (Ia)

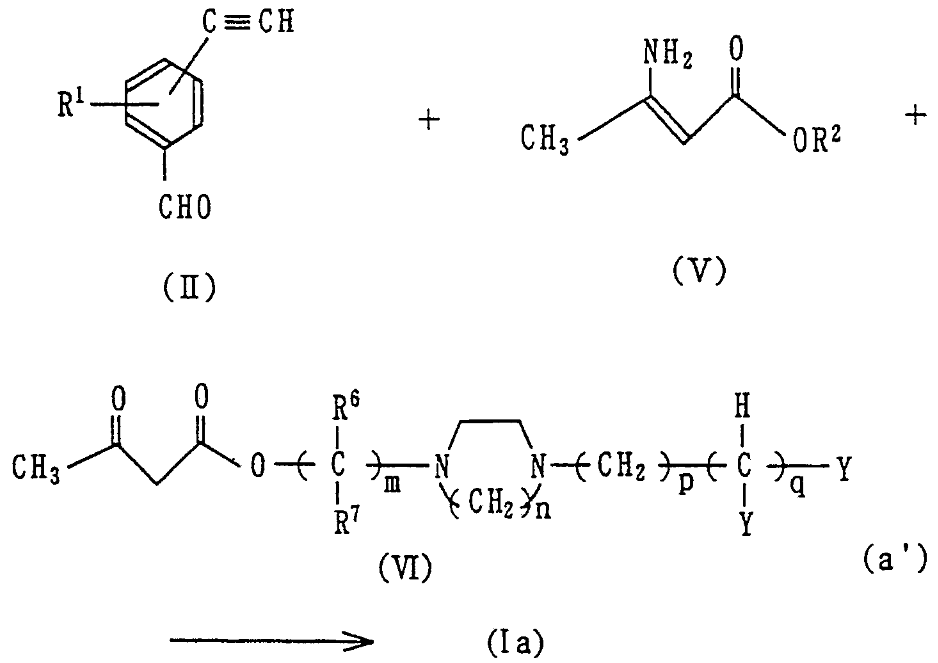

(II) (V) (VI) (a') (Ia)

In the reaction formula, $R^1$ is hydrogen atom or a halogen atom, $R^2$ is a lower alkyl group or a lower alkoxyalkyl group, Y is the formula:

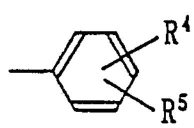

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

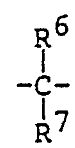

can be the same or different from each other, n is an integer of 2 or 3, p is an integer of 0 to 2 and q is an integer of 0 or 1.

In a typical process for preparation, the ethynylphenyl derivative having the formula (Ia) described in the reaction formula (a) and (a') can be prepared by adding an ethynylbenzaldehyde derivative (II), a keto-ester derivative (III) and an aminocrotonic acid derivative (IV) or an ethynylbenzaldehyde derivative (II), a keto-ester derivative (VI) and an aminocrotonic acid derivative (V) to a suitable organic soluent such as a lower alkanol, e.g. ethanol.

Alternatively, according to the reaction (b) or (b'), the ethynylphenyl derivative having the formula (Ia) can be also prepared by adding a keto-ester derivative (III) or (VI) and an ethynylbenzaldehyde derivative (II) to a solution of lower alkanol containing an aminocrotonic acid derivative (V) or (IV) which is previously derived from a keto-ester derivative (III) or (VI) and ammonium carbonate or ammonium acetate.

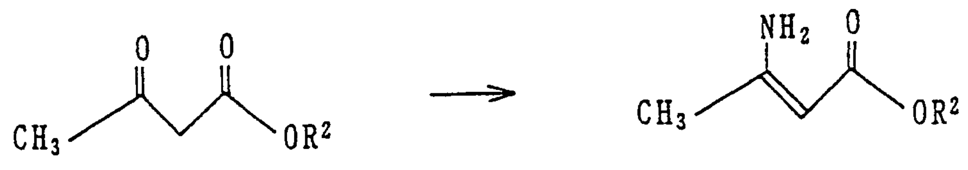

(III) (V) (b)

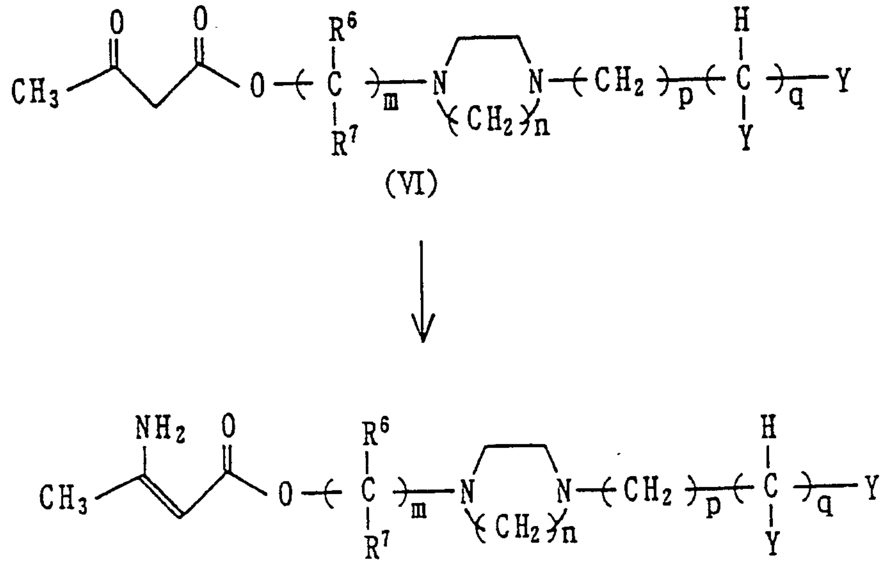

(VI)

(b')

(IV)

In the reaction formula, $R^2$, $R^6$, $R^7$, m, n, p, q and Y are as defined above.

With respect to the reaction formula (b) and (b'), a solution of a lower alkanol which is prepared by adding a keto-ester derivative (III) or (VI) and 1 to 1.5 equivalents of ammonium carbonate or ammonium acetate per equivalents of keto-ester derivative (III) or (VI) to a lower alkanol is heated, typically for 30 minutes to 5 hours, preferably at 30° to 120°C to substantially complete a conversion of (III) into (V) or (VI) into (IV).

Organic solvents which can be used in the present reaction are not particularly limited, if the solvents do not considerably inhibit this type of reaction. Examples of the suitable solvents are, for instance, lower alkanols such as ethanol, methanol, isopropyl alcohol and n-propyl alcohol.

With respect to the used amount of each reactant in the present reaction, preferably 1 to 1.5, in particular, preferably 1 to 1.3 equivalents of ammonium carbonate or ammonium acetate is used per equivalent of keto-ester derivative (III) or (VI).

In the present reaction, temperature is preferably 30° to 120°C, in particular, preferably 30° to 100°C and the reaction time is preferably 30 minutes to 5 hours, in particular, preferably 30 minutes to 4 hours.

The each amount of an aminocrotonic acid derivative (IV) and (V) and a keto-ester derivative (III) and (VI) used in the reaction formula (a) and (a') is usually an equal equivalent or a little excess, preferably 1 to 1.3 equivalents per equivalent of ethynylbenzaldehyde derivative (II).

Thus obtained reaction solution of a lower alkanol is stirred with heating for 1 to 24 hours, preferably at 20° to 120°C until the reaction is substantially completed. Subsequently the compound having the formula (Ia) can be purified and isolated by means of a conventional treatment method, for instance, recrystallization, chromatography or the like.

That is, organic solvents which can be used in the present reaction are not particularly limited, if the solvents do not considerably inhibit this type of reaction. Examples of the suitable solvents are, for instance, lower alkanols such as ethanol, methanol, isopropyl alcohol and n-propyl alcohol.

With respect to the used amount of each reactant in the present reaction, preferably 1 to 1.3, in particular, preferably 1 to 1.2 equivalents of an aminocrotonic acid derivative (IV) and a keto-ester derivative

(III) or a keto-ester derivative (VI) and an aminocrotonic acid deriative (V) are used respectively per equivalent of ethynylbenzaldehyde derivative (II).

In the present reaction, the reaction temperature is preferably 20° to 120° C, in particular, preferably 30° to 100° C and the reaction time is preferably 1 to 24 hours, in particular, preferably 1 to 20 hours.

Process A-2

The compound having the formula (Ia) wherein $R^3$ is methyl group can be also prepared according to the reaction formula (c) or (c').

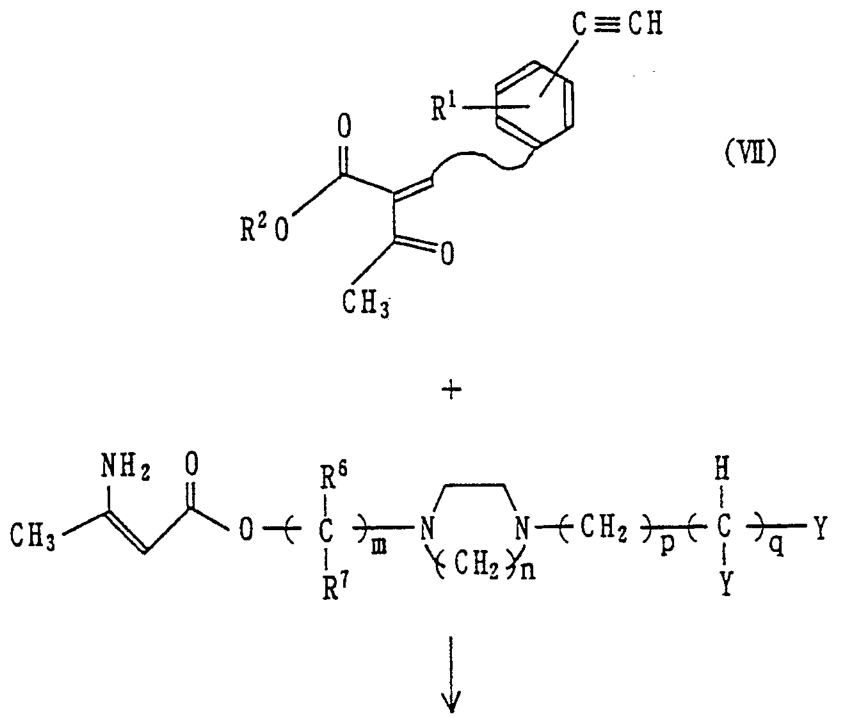

(VII)

(c)

(IV)

(Ia)

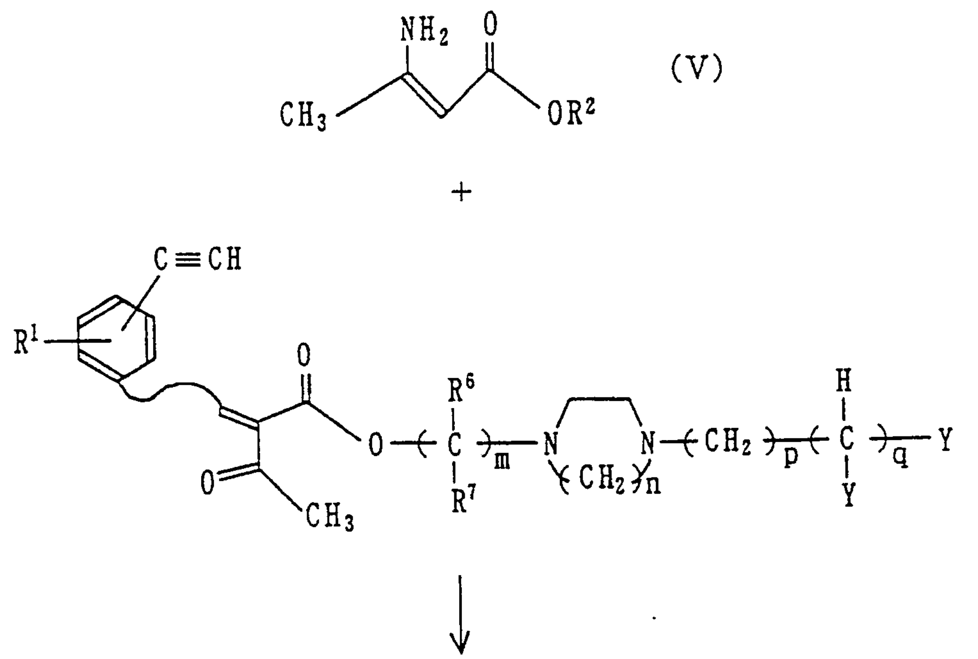

(V)

(c')

(VIII)

(Ia)

In the reaction formula, $R^1$, $R^2$, $R^6$, $R^7$, m, n, p, q and Y are as defined above.

In a typical process for preparation, the ethynylphenyl derivative having the formula (Ia) described in the reaction formula (c) and (c') can be prepared by adding a benzylidene derivative (VII) or (VIII) and an aminocrotonic acid derivative (IV) or (V) respectively to a suitable organic solvent such as a lower alkanol, e.g. ethanol. The amount of the aminocrotonic acid derivative (IV) or (V) used here is usually an equal

equivalent or a little excess, preferably 1 to 1.3 equivalents per equivalent of benzylidene derivative (VII) or (VIII).

Thus obtained reaction solution of a lower alkanol is stirred with heating for 1 to 24 hours, preferably at 20° to 120° C to substantially complete the reaction. Subsequently the compound having the formula (Ia) can be purified and isolated according to a conventional method, for instance, recrystallization, chromatography or the like.

That is, organic solvents which can be used in the present reaction are not particularly limited, if the solvents do not considerably inhibit this type of reaction. Examples of the suitable solvents are, for instance, lower alkanols such as ethanol, methanol, isopropyl alcohol and n-propyl alcohol.

With respect to the used amount of each reactant in the present reaction, preferably 1 to 1.3, in particular, preferably 1 to 1.2 equivalents of an aminocrotonic acid derivative (IV) or (V) is used per equivalent of a benzylidene derivative (VII) or (VIII).

In the present reaction, the reaction temperature is preferably 20° to 120° C, in particular, preferably 30° to 100° C and the reaction time is preferably 1 to 24 hours, in particular, preferably 1 to 20 hours.

The benzylidene derivative (VII) or (VIII) used in the reaction formula (c) and (c') can be prepared according to the reaction formula (d) and (d').

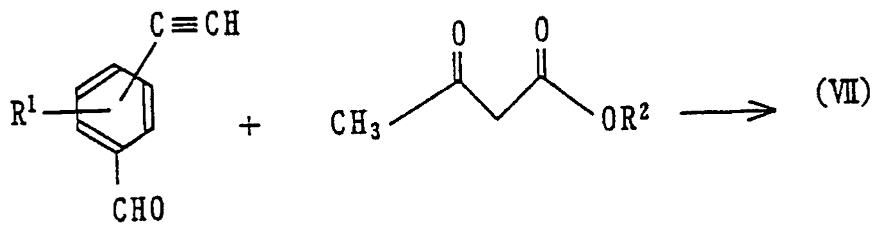

(II) (III) (VII) (d)

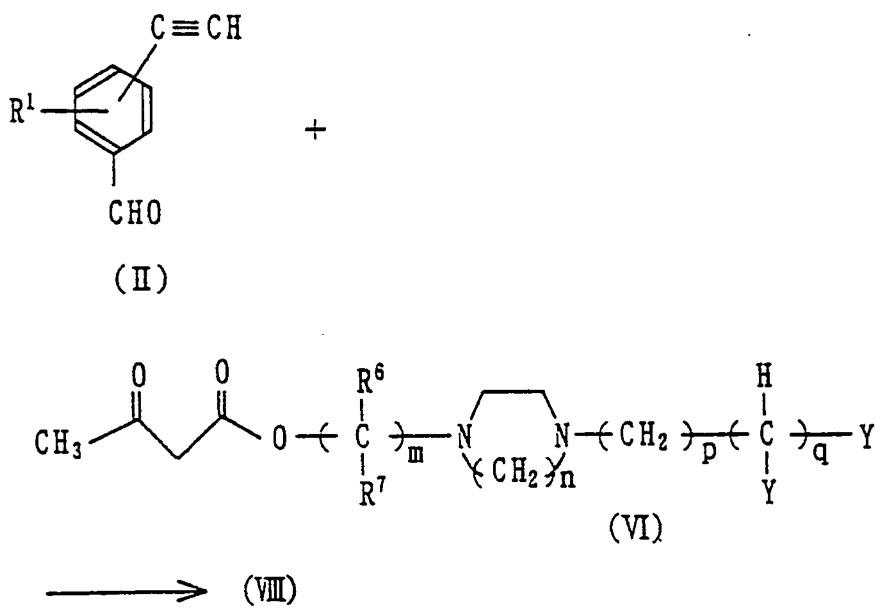

(II) (VI) (VIII) (d')

In the reaction formula, $R^1$, $R^2$, $R^6$, $R^7$, m, n, p, q and Y are as defined above.

In a typical process for preparation, an equal equivalent of ethynylbenzaldehyde derivative (II) and a keto-ester derivative (III) or (VI) are added to a suitable aromatic organic solvent such as toluene or benzene, and the mixture is reacted with using a suitable amine such as a cyclic secondary amine, e.g. piperidine, pyrrolidine or the like or a lower tertiary alkylamine, e.g. a triethylamine or the like as a base catalyst. The reaction solution is usually refluxed, and produced water is removed by an eliminator of water.

The reaction solution is stirred with heating for 2 to 100 hours to substantially complete the reaction.

The isolation and purification of the compound having the formula (VII) or (VIII) are carried out according to the method previously explained in Process A-1.

That is, organic solvents which can be used in the present reaction are not particularly limited, if the solvents do not considerably inhibit this type of reaction. Examples of the suitable solvents are, for instance, aromatic organic solvents such as toluene, benzene and xylene.

With respect to the used amount of each reactant in the present reaction, preferably 1 to 1.3, in particular, preferably 1 to 1.2 equivalents of a keto-ester derivative (III) or (VI) is used per equivalent of ethynylbenzaldehyde derivative (II).

In the present reaction, the reaction time is preferably 2 to 100 hours, in particular, preferably 3 to 80 hours.

Process B-1

The compound having the formula (Ib) wherein $R^3$ is amino group can be prepared according to the reaction formula (e).

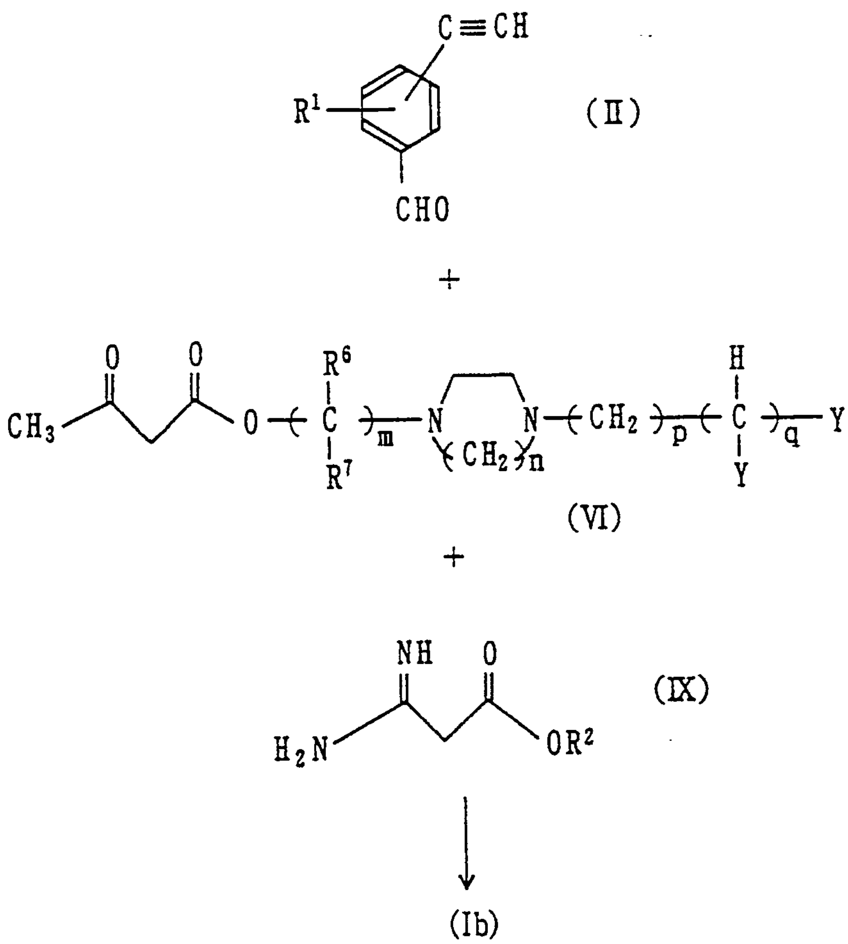

(II)

(VI)

(IX)

(Ib)

(e)

In the reaction formula, $R^1$, $R^2$, $R^6$, $R^7$, m, n, p, q and Y are as described above.

In a typical process for preparation, the ethynylphenyl derivative having the formula (Ib) described in the reaction formula (e) can be prepared by adding ethynylbenzaldehyde derivative (II), amidine derivative (IX) and a keto-ester derivative (VI) to a suitable organic solvent such as a lower alkanol, e.g. ethanol. The each amount of the amidine derivative (IX) and the keto-ester derivative (VI) used here is usually an equal equivalent or a little excess, preferably 1 to 1.3 equivalents per equivalent of ethynylbenzaldehyde (II). The obtained reaction solution of a lower alkanol is stirred with heating for 1 to 24 hours, preferably at 20° to 120° C to substantially complete the reaction. Subsequently the purification and isolation of the compound having the formula (Ib) are carried out according to the method previously explained in Process A-1.

That is, organic solvents which can be used in the present reaction are not particularly limited, if the solvents do not considerably inhibit this type of reaction. Examples of the suitable solvents are lower alkanols such as ethanol, methanol, isopropyl alcohol and n-propyl alcohol.

With respect to the used amount of each reactant in the present reaction, preferably 1 to 1.3, in particular, preferably 1 to 1.2 equivalents of a keto-ester derivative (VI) and an amidine derivative (IX) are used per equivalent of ethynylbenzaldehyde (II).

In the present reaction, the reaction temperature is preferably 20° to 120° C, in particular, preferably 30° to 100° C and the reaction time is preferably 1 to 24 hours, in particular, preferably 1 to 20 hours.

Process B-2

The compound having the formula (Ib) wherein $R^3$ is amino group can be prepared according to the reaction formula (f).

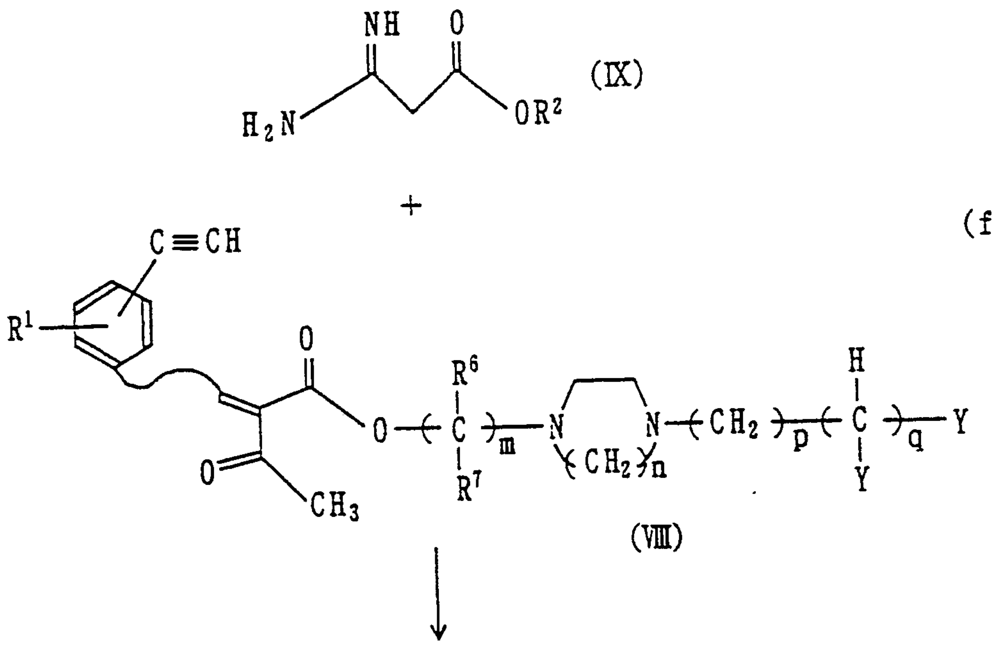
(IX)

(f)

(VIII)

(Ib)

In the reaction formula, $R^1$, $R^2$, $R^6$, $R^7$, m, n, p, q and Y are as defined above.

The benzylidene derivative (VIII) used here can be prepared from an ethynylbenzaldehyde derivative (II) and a keto-ester derivative (VI) according to the reaction formula (d') in the same manner as previously explained in Process A-2.

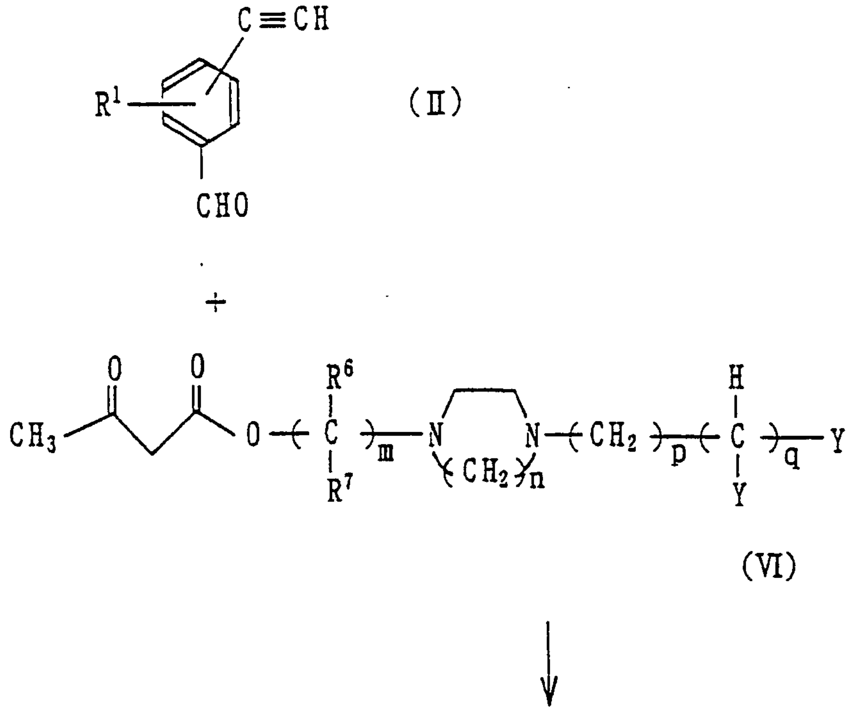

(II)

(d')

(VI)

(VIII)

In the reaction formula, $R^1$, $R^2$, $R^6$, $R^7$, m, n, p, q and Y are as defined above.

In a typical process for preparation, the ethynylphenyl derivative having the formula (Ib) described in the reaction formula (f) can be prepared by adding a benzylidene derivative (VIII) and an amidine derivative (IX) to a suitable organic solvent such as lower alkanol, e.g. ethanol. The amount of the amidine derivative (IX) used here is usually an equal equivalent or a little excess, preferably 1 to 1.3 equivalents per equivalent of benzylidene derivative (VIII). Thus obtained reaction solution of a lower alkanol is stirred with heating for 1 to 24 hours, preferably at 20° to 120° C to substantially complete the reaction. Subsequently the purification and isolation of the compound having the formula (Ib) are carried out according to the method previously explained in Process A-1.

That is, organic solvents which can be used in the present reaction are not particularly limited, if the solvents do not considerably inhibit this type of reaction. Examples of the suitable solvents are lower alkanols such as ethanol, methanol, isopropyl alcohol and n-propyl alcohol.

With respect to the used amount of each reactant in the present reaction, preferably 1 to 1.3, in particular, preferably 1 to 1.2 equivalents of an amidine derivative (IX) is used per equivalent of benzylidene derivative (VIII).

In the present reaction, the reaction temperature is preferably 20° to 120° C, in particular, preferably 30° to 100° C and the reaction time is preferably 1 to 24 hours, in particular, preferably 1 to 20 hours.

Process C-1

The compound having the formula (Ic) wherein $R^3$ is dimethoxymethyl group can be prepared according to the reaction formula (g).

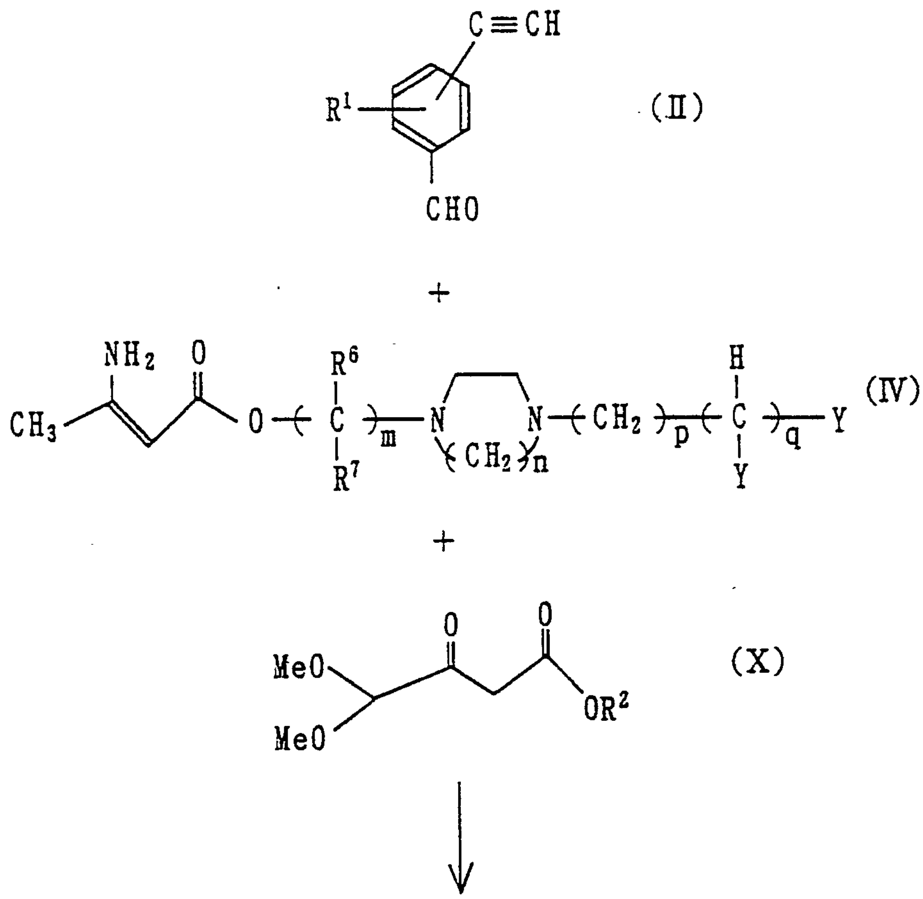

(II)

(IV)

(g)

(X)

(Ic)

In the reaction formula, $R^1$, $R^2$, $R^6$, $R^7$, m, n, p, q and Y are as defined above.

In a typical process for preparation, the ethynylphenyl derivative having the formula (Ic) described in the reaction formula (g) can be prepared by adding an ethynylbenzaldehyde derivative (II), an aminocrotonic acid derivative (IV) and an acetal keto-ester derivative (X) to a suitable organic solvent such as lower alkanol, e.g. ethanol. Alternatively, according to the reaction formula (b') as previously explained in Process A-1, the ethynylphenyl derivative (Ic) can be prepared by adding an ethynylbenzaldehyde derivative (II) and an acetal keto-ester derivative (X) to a solution of a lower alkanol containing an aminocrotonic acid derivative (IV) which is previously derived from a keto-ester derivative (VI).

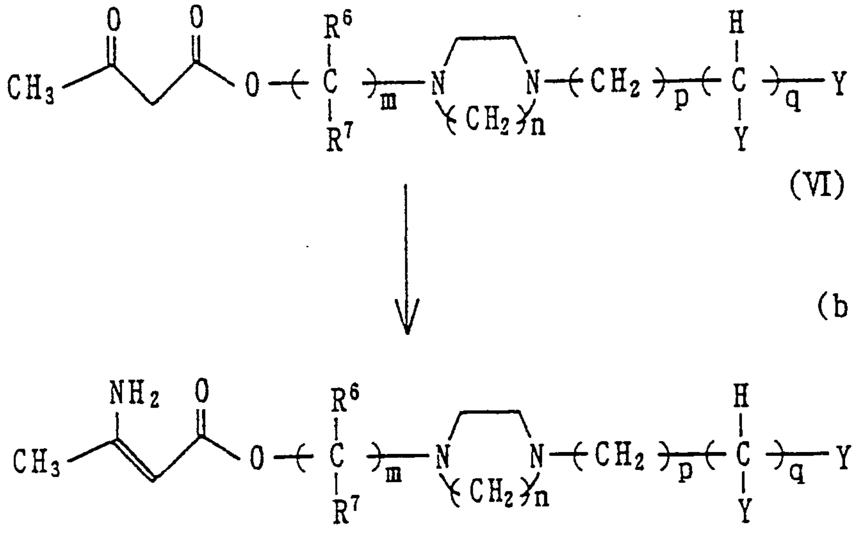

(VI)

(b')

(IV)

In the reaction formula, $R^6$, $R^7$, m, n, p, q and Y are as described above.

The each amount of the aminocrotonic derivative (IV) and the acetal keto-ester derivative (X) used here is usually an equal equivalent or a little excess, preferably 1 to 1.3 equivalents per equivalent of ethynylbenzaldehyde derivative (II). The obtained reaction solution of a lower alkanol is stirred with heating for 1 to 24 hours, preferably at 20° to 120°C to substantially complete the reaction. Subsequently the purification and isolation of the compound having the formula (Ic) are carried out according to the method previously explained in Process A-1.

That is, organic solvents which can be used in the present reaction are not particularly limited, if the solvents do not considerably inhibit this type of reaction. Examples of the suitable solvents are lower alkanols such as ethanol, methanol, isopropyl alcohol and n-propyl alcohol.

With respect to the used amount of each reactant in the present reaction, preferably 1 to 1.3, in particular, preferably 1 to 1.2 equivalents of an aminocrotonic acid derivative (IV) and an acetal keto-ester derivative (X) are used per equivalent of ethynylbenzaldehyde derivative (II).

In the present reaction, the reaction temperature is preferably 20° to 120°C, in particular, preferably 30° to 100°C and the reaction time is preferably 1 to 24 hours, in particular, preferably 1 to 20 hours.

Process C-2

The compound having the formula (Ic) wherein $R^3$ is dimethoxymethyl group can be prepared according to the reaction formula (h).

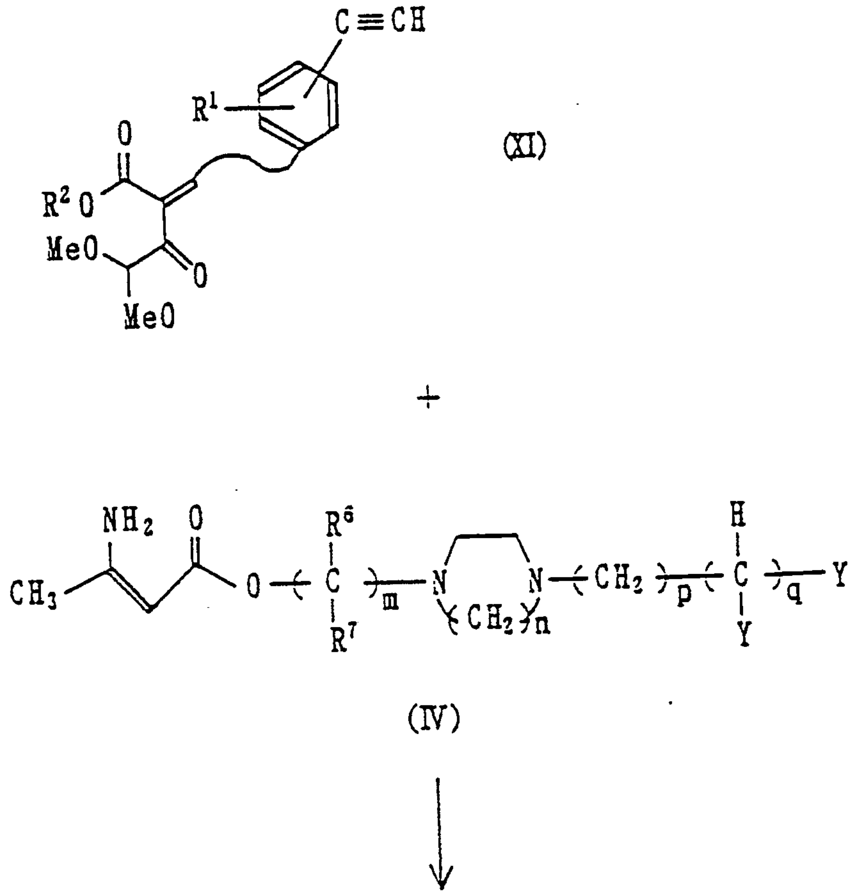

(XI)

(h)

+

(IV)

(Ic)

In the reaction formula, $R^1$, $R^2$, $R^6$, $R^7$, m, n, p, q and Y are as defined above.

The benzylidene derivative (XI) used here can be prepared from an ethynylbenzaldehyde derivative (II) and an acetal keto-ester derivative (X) according to the reaction formula (i) in the same manner as explained in Process A-2.

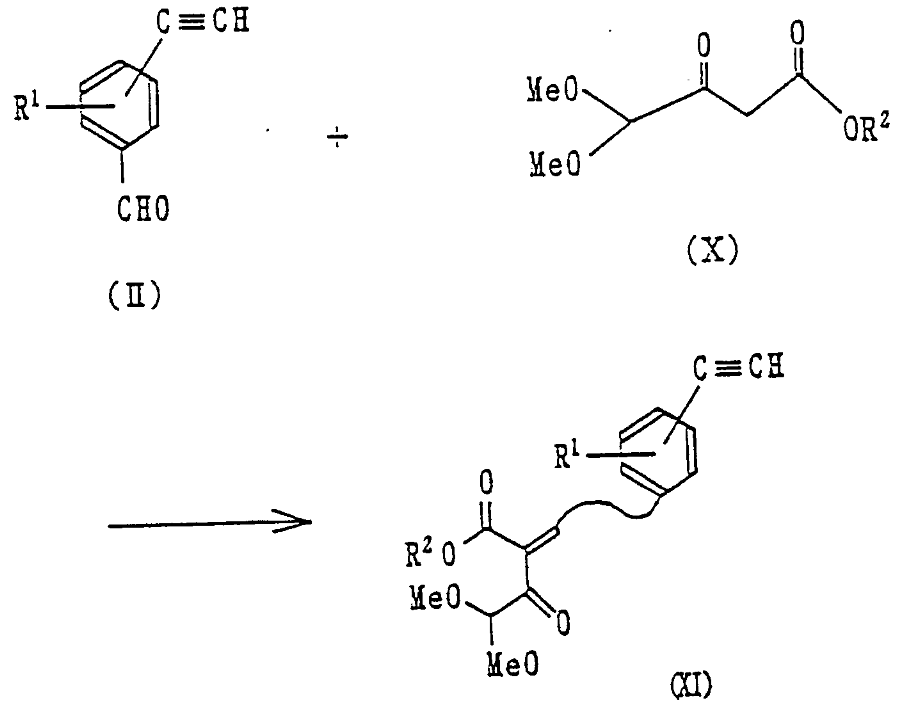

(II) (X) (XI) (i)

In the reaction formula, $R^1$ and $R^2$ are as defined above.

In a typical process for preparation, the ethynylphenyl derivative having the formula (Ic) described in the reaction formula (h) can be prepared by adding a benzylidene derivative (XI) and an aminocrotonic acid derivative (IV) to a suitable organic solvent such as a lower alkanol, e.g. ethanol.

The amount of the aminocrotonic acid derivative (IV) used here is usually an equal equivalent or a little excess, preferably 1 to 1.3 equivalents per equivalent of benzylidene derivative (XI). Thus obtained reaction solution of a lower alkanol is stirred with heating for 1 to 24 hours, preferably at 20° to 120°C to substantially complete the reaction. Subsequently the purification and isolation of the compound having the formula (Ic) are carried out according to the method previously explained in Process A-1.

That is, organic solvents which can be used in the present reaction are not particularly limited, if the solvents do not considerably inhibit this type of reaction. Examples of the suitable solvents are lower alkanols such as ethanol, methanol, isopropyl alcohol and n-propyl alcohol.

With respect to the used amount of each reactant in the present reaction, preferably 1 to 1.3, in particular, preferably 1 to 1.2 equivalents of the aminocrotonic acid derivative (IV) is used per equivalent of benzylidene derivative (XI).

In the present reaction, the reaction temperature is preferably 20° to 120°C, in particular, preferably 30° to 100°C and the reaction time is preferably 1 to 24 hours, in particular, preferably 1 to 20 hours.

Process D

The compound having the formula (Id) wherein $R^3$ is formyl group can be prepared according to the reaction formula (j), by hydrolyzing the compound having the formula (Ic) wherein $R^3$ is dimethoxymethyl group which can be obtained by Process C-1 and C-2.

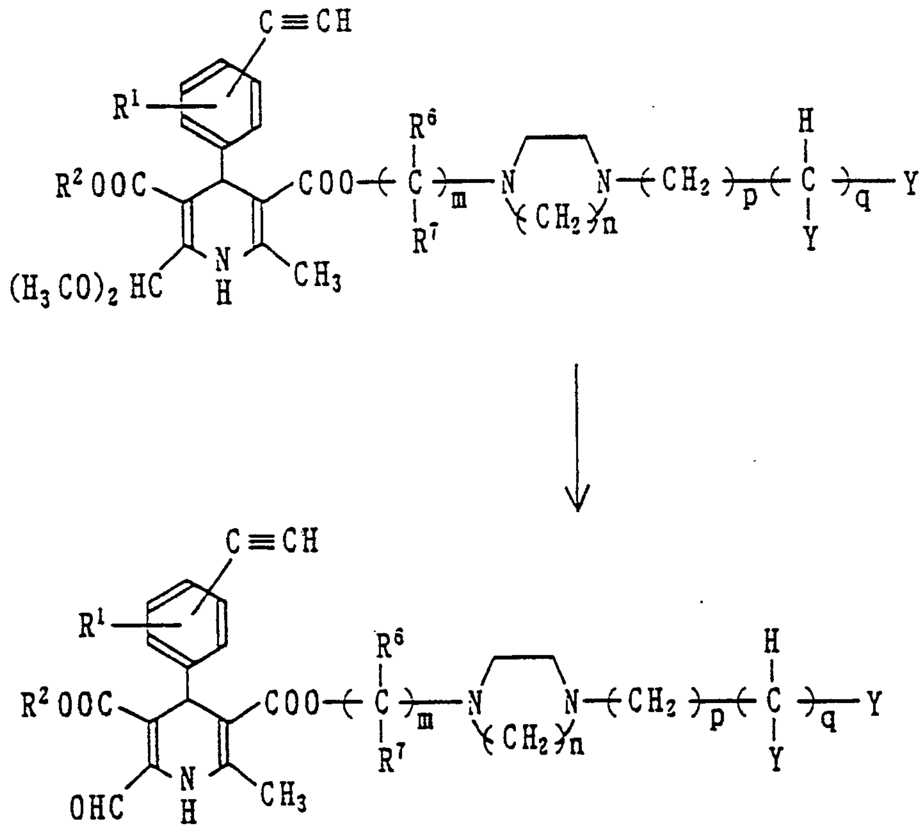

(Ic)

(j)

(Id)

In the reaction formula, $R^1$, $R^2$, $R^6$, $R^7$, m, n, p, q and Y are as defined above.

In a typical process for preparation, the ethynylphenyl derivative having the formula (Id) described in the reaction formula (j) can be prepared by adding the ethynylphenyl derivative (Ic) described in the reaction formula (j) to a suitable organic solvent such as acetone, dioxane, tetrahydrofuran, dimethylsulfoxide or, N,N-dimethylformamide and water, or an admixture thereof and sequentially adding an acid, for instance, an inorganic acid such as hydrochloric acid or sulfuric acid, an organic acid such as acetic acid, formic acid, trifluoroacetic acid or p-toluene sulfonic acid, or an acidic ion exchange resin or the like. The amount of the acid used is usually so called catalytic amount, preferably 0.01 to 0.05 equivalents per equivalent of the ethynylphenyl derivative (Ic) described in the reaction formula (j). Thus obtained reaction solution is stirred for 1 to 12 hours, preferably at 0° to 60°C to substantially complete the reaction. Subsequently the purification and isolation of the compound of the ethynylphenyl derivative having the formula (Id) described in the reaction formula (j), are carried out according to the method previously explained in Process A-1.

That is, in the present reaction, the reaction temperature is preferably 0° to 60°C, in particular, preferably 10° to 60°C and the reaction time is preferably 1 to 12 hours, in particular, preferably 1 to 10 hours.

Process E

The compound having the formula (Ie) wherein $R^3$ is cyano group can be prepared according to the reaction formula (k) by converting the compound having the formula (Id) wherein $R^3$ is formyl group which can be obtained by Process D into an oxime followed by dehydration reaction.

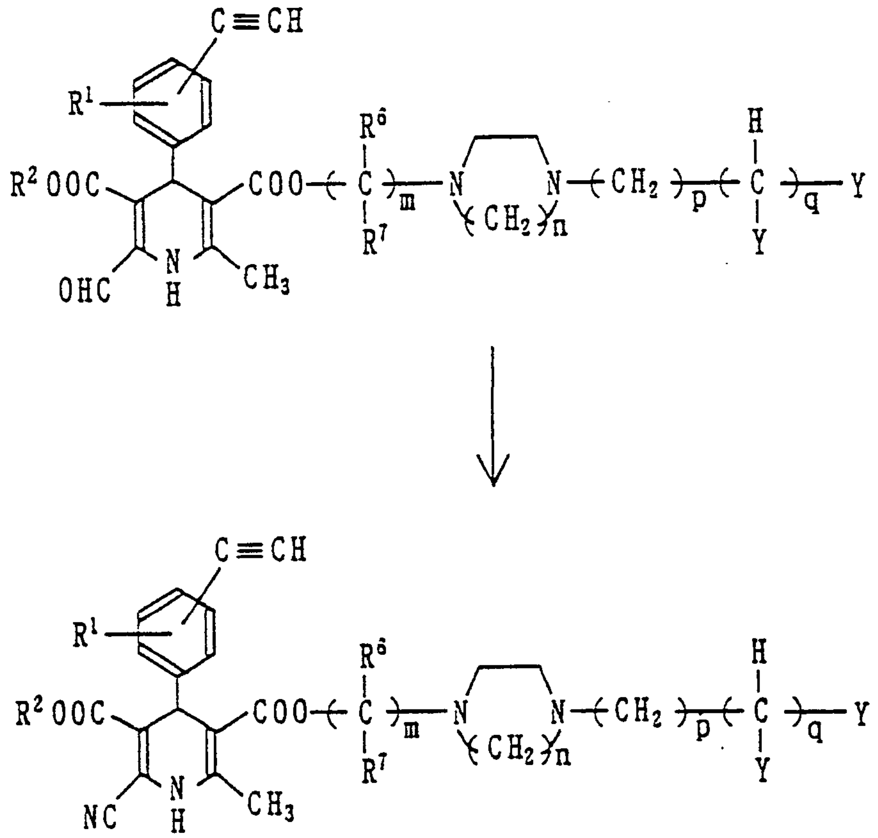

(Id)

(k)

(Ie)

In the reaction formula, $R^1$, $R^2$, $R^6$, $R^7$, m, n, p, q and Y are as defined above.

In a typical process for preparation, the following process is exemplified. The ethynylphenyl derivative having the formula (Id) and hydroxylamine or its salt (for instance, a salt of an inorganic acid such as hydrochloric acid or sulfuric acid, a salt of an organic acid such as acetic acid or formic acid, or the like) are added to a suitable organic solvent such as dioxane, ethanol or N,N-dimethylformamide and water, or an admixture thereof. Then, an acid or an inorganic weak base is added to the mixture. Examples of the acids are, for instance, an inorganic acid such as hydrochloric acid, hydrobromic acid or sulfuric acid, an organic acid such as acetic acid, formic acid, trifluoroacetic acid or p-toluenesulfonic acid, and the like. Examples of the inorganic weak base are, for instance, sodium acetate, potassium acetate, sodium formate, and the like. Hydroxylamine or its salt is used in an amount, generally, of an equal equivalent or a little excess, preferably 1 to 1.3 equivalents per equivalent of the ethynylphenyl derivative having the formula (Id). The used amount of the acid is from 20 to 50 equivalents per equivalent of the ethynylphenyl derivative (Id). When the acid is in the state of a liquid, the acid can be also used as the solvent. The amount of the inorganic weak base is generally from 1 to 1.5 equivalents per equivalent of the hydroxylamine or its salt. Thus obtained reaction solution is stirred until the reaction is substantially completed, for 1 to 5 hours, preferably at 0° to 50° C. That is to say, as to the used amount of each reactant in the conversion into an oxime of the present reaction, it is preferable that hydroxylamine is used in an amount of 1 to 1.3 equivalents per equivalent of the ethynylphenyl derivative (Id), which is described in the reaction formula (k), the acid is used in an amount of 20 to 50 equivalents per equivalent of the ethynylphenyl derivative, and if necessary, the inorganic weak base is used in an amount of 1 to 1.5 equivalents per equivalent of the hydroxylamine or its salt.

In the conversion into an oxime of the present reaction, it is preferable that the reaction temperature is from 0° to 50° C and reaction time is from 1 to 5 hours. The thus prepared oxime is isolated and purified, or the obtained reaction mixture is subjected to next dehydration as it is. In case of isolating and purifying, the same solvent as previously used in conversion into an oxime is usually used as the solvent used in next dehydration. Subsequetly, a dehydrating agent is added to the solution containing the oxime as an intermediate. Examples of the dehydrating agents are, for instance, an inorganic acid such as sulfuric acid, phosphoric acid or polyphosphoric acid, an organic acid, such as formic acid, acetic acid or p-toluenesulfonic acid, an organic acid anhydride such as benzoic anhydride, acetic anhydride or phthalic anhydride, an organic acid chloride such as acetyl chloride, benzoyl chloride, methanesulfonyl chloride or formyl chloride,

an inorganic chloride such as thionyl chloride, phosphorus pentachloride, phosphorus oxychloride or phosphorus tribromide, a carbodiimide such as N,N'-dicyclohexylcarbodiimide, and the like. The amount of the used dehydrating agent generally exceeds the amount of the ethynylphenyl derivative having the formula (Id). It is preferable that the dehydrating agent is used in an amount of 3 to 7 equivalents per equivalent of the ethynylphenyl derivative (Id). Thus obtained reacion solution is stirred until the reaction is substantially completed, for 1 to 10 hours, preferably at 20° to 130° C. The obtained compound having the formula (Ie) is isolated and purified in the same manner as explained in Process A-1.

That is, in dehydration of the present reaction, preferably 3 to 7, in particular, preferably 3 to 6 equivalents of the dehydrating agent per equivalent of the ethynylphenyl derivative (Id) described in the reaction formula (k) is used.

In the dehydration of the present reaction, a reaction temperature is preferably 20° to 130° C, in particular, preferably 30° to 120° C and the reaction time is preferably 1 to 10 hours, in particular, preferably 1 to 9 hours.

[Process for preparing an aminocrotonic acid derivative (IV) or (V)]

Each can be prepared by treating a keto-ester derivative such as a compound having the formula (III) or (VI) with liquid ammonia according to the reaction formula (1) or (1').

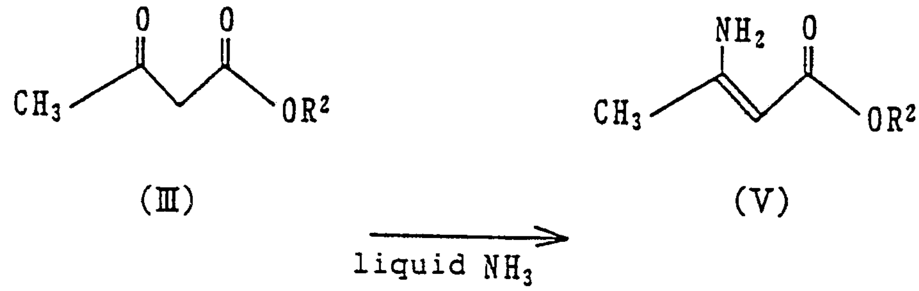


(III) (V) (ℓ)

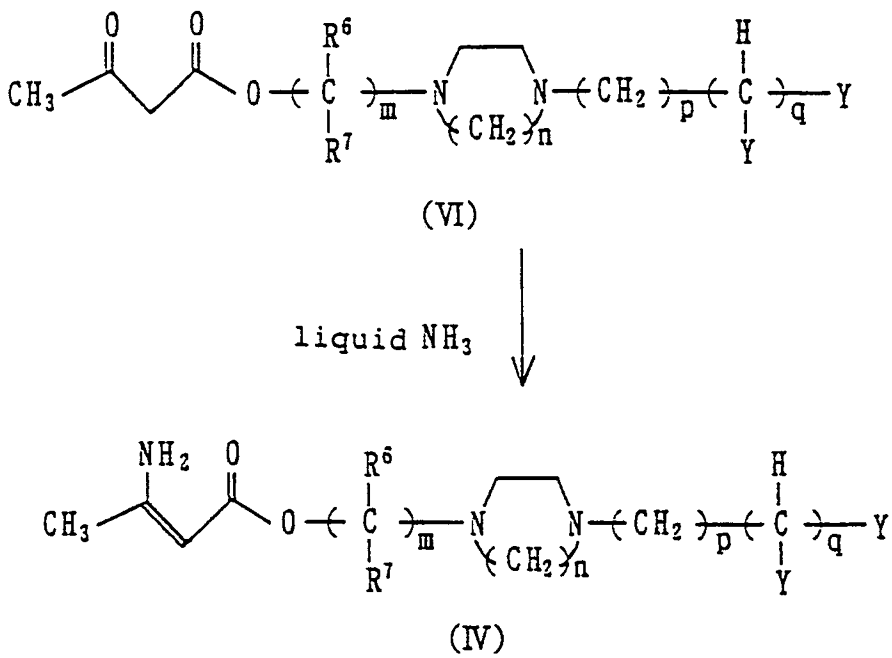


(VI) (IV) (ℓ')

In the reaction formula, $R^2$, $R^6$, $R^7$, m, n, p, q and Y are as defined above.

[Process for preparing a keto-ester derivative (III) or (VI)]

Each can be prepared by addition reaction of a corresponding alcohol derivative (XII) or (XIII) with a diketene (XIV) in the presence of a basic catalyst according to the reaction formula (m) or (m').

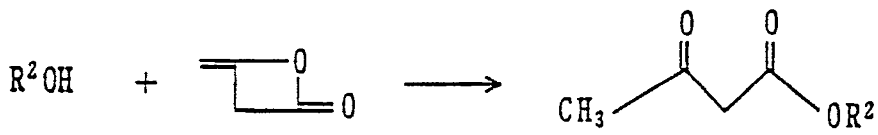

(XII) (XIV) (III) (m)

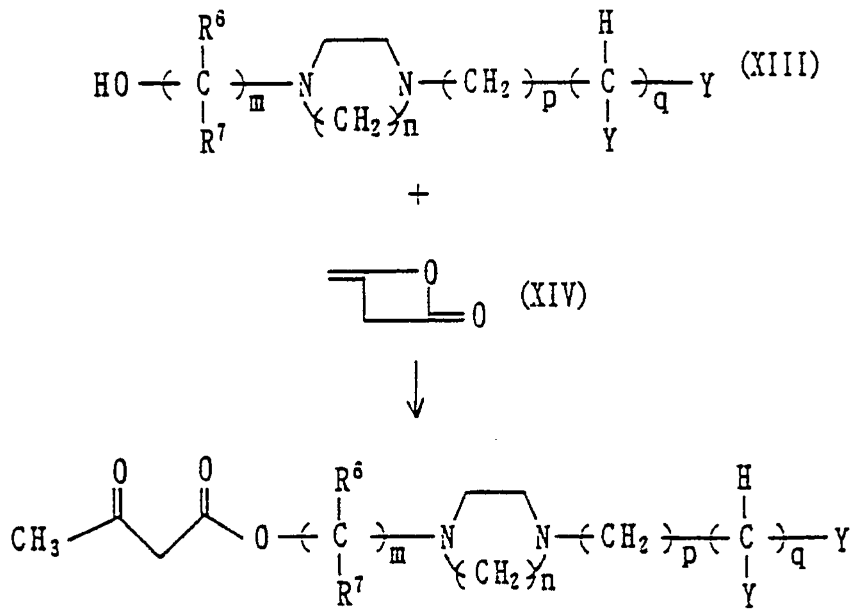

(XIII) (XIV) (VI) (m')

In the reaction formula, $R^2$, $R^6$, $R^7$, m, n, p, q and Y are as defined above.

[Process for preparing an amidine derivative (IX)]

According to the reaction formula (n), a cyanoacetic acid derivative (XV) is treated with hydrogen chloride in a lower alkanol e.g. ethanol to give an imidate derivative (XVI) [see S,A, Glickman and A,C, Cope, Journal of the American Chemical Society, 67, page 1017 (1945)], then the compound (XVI) is treated with liquid ammonia [see S,M,McElvain and B,E,Tate, Journal of the American Chemical Society, 73, page 2760 (1951)] to give the amidine derivative (IX).

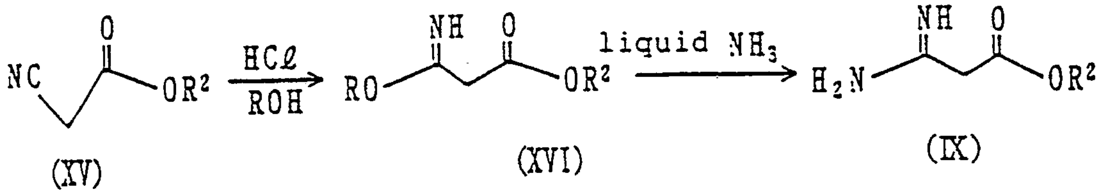


(XV) (XVI) (IX) (n)

In the reaction formula, R is a lower alkyl having carbon atoms of 1 to 3 and $R^2$ is as defined above.

[Process for preparing an acetal keto-ester derivative (X)]

This can be prepared by condensing pyruvic aldehyde dimethyl acetal (XVII) with a lower alcohol ester of carbonic acid (XVIII) according to the reaction formula (o) [see J.A.Secrist, C.J,Hickey and R.E.Norris, Journal of Organic Chmistry, 42, page 525 (1977)].

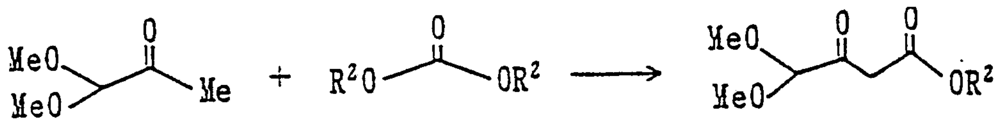

(XVII) (XVIII) (X) (o)

In the reaction formula, $R^2$ is as defined above.

The suitable reagents and reaction conditions for chemical changes in the process for preparing the raw material compounds as mentioned above are those well known in the art.

The compound having the formula (I) of the present invention has calcium antagonistic activity, superior antihypertensive activity, activity of increasing cardiac coronary blood flow and activity of inhibiting angiotensin II, and is useful as an effective ingredient of, for example, a vasodepressor, coronary vasodilator, preventive and trerapeutic agent for cardiac failure, cerebral blood flow increasing agent and phosphodiesterase inhibitor.

Among the compounds having the formula (I) of the present invention, the compounds having the formula (I) wherein ethynyl group is substituted at the 3 (meta)-position, $R^1$ is hydrogen atom, $R^1$ is a halogen atom, $R^2$ is an alkyl group, $R^2$ is an alkoxyalkyl group, $R^3$ is methyl group, $R^3$ is amino group, $R^3$ is cyano group, $R^3$ is dimethoxymethyl group, $R^3$ is formyl group, $R^4$ and $R^5$ are the same and hydrogen atom, $R^4$ and $R^5$ are the same halogen atom, $R^4$ and $R^5$ are the same lower alkoxy group, $R^4$ and $R^5$ are the same alkyl group, m is 2, n is 2, p is 0 and q is 1 are preferable.

Besides, since the ethynylphenyl derivative (I) has a basic group, it can be changed into the acid addition salt thereof by known means. These salts are not particularly limited if they are pharmacologically acceptable. Examples of these salts are, for instance, acid addition salt of mineral acid such as hydrochloric acid, hydrobromic acid, hydroiodic acid or sulfuric acid, acid addition salt of sulforic acid such as methanesulforic acid, p-toluenesulforic acid or benzenesulforic acid, acid addition salt of organic acid such as acetic acid, phosphoric acid, oxalic acid, maleic acid, tartaric acid, citric acid, gluconic acid or lactic acid, and the like.

The compound having the formula (I) may be in any preparation form for oral or parenteral adiministration. Examples of the preparation form are, for instance, preparations for oral administration such as tablets, capsules, granules, powders and syrups, preparations for parenteral administration such as injections containing subcutaneous injection and intravenous injection, suppositories and cataplasmata or emplastra and the like. These various preparations can be prepared in a usual method by using any conventional additives which are pharmaceutically accepted in the basis in accordance with the purpose, such as excipient, binder, disintegrator, lubricant, flavor, solubilizer or suspending agent.

Examples of the additives include gelatin, lactose, sucrose, titanium oxide, starch, crystalline cellulose, hydroxypropylmethylcellulose, carboxymethyl cellulose, corn starch, microcrystalline wax, white petrolatum, magnesium alumino meta silicate, anhydrous calcium phosphate, citric acid, trisodium citrate, hydroxypropyl cellulose, sorbitol, sorbitan esters of fatty acids, polyvinylpyrrolidone, magnesium stearate, light

anhydrous silicic acid, talc, vegetable oil, benzyl alcohol, gum arabic, propylene glycol or polyalkylene glycol. Although the dosage of the compound of the present invention is different according to symptom, age, body weight, route and times of administration, a usual dosage is about 2 to about 300 mg, on the basis of the compound (I) of the present invention per day for adults, and can be devided to 1 to several times.

And in cases of the compounds having the above-mentioned formula (I), asymmetric carbons exist in the molecules thereof. The present invention includes these optical isomers due to asymmetric carbons or a mixture thereof mixed in an optional rate.

The usefulness of the compound of the present invention as to drug efficacy is clear from the following Test Examples. As shown in Test Example 1-1, the compound of the present invention has greater antihypertensive activity and persistent effect thereof in comparison with nimodipine and nicardipine which are traditional calcium antagonists. Further, the persistant effect of antihypertensive activity of the compound of the present invention is shown in Test Example 1-2. As shown in Test Example 2, the compound of the present invention gives less influence on cardiac atrioventricular conduction system in comparison with nicardipine. Therefore, it has a characteristic as an excellent vasodepressor which gives less stress on heart. As shown in Examples 3 and 4, the compound of the present invention can be expected to have preventive and therapeutic effects on stenocardia by increasing coronary blood flow of ischemic heart, and these effects are superior to those of nifedipine or nicardipine. And the compound of the present invention has excellent activity of improving cerebrocirculation because it has superior activity of increasing vertebra blood flow in comparison with nifedipine or nicardipine as shown in Example 4. The compound of the present invention is expected to have preventive and therapeutic effects on cardiac failure because it has angiotensin II antagonistic activity as shown in Example 5. Besides, the compound of the present invention is regarded to cause no side effect such as gingival hyperplasia because it shows no anticonvulsive activity as shown in Example 6.

The ethynylphenyl derivative of the present invention shows greater antihypertensive activity than nicardipine, nifedipine and nimodipine which are carcium antagonists on the market, and further the activity persists. Besides, said ethynylphenyl derivative has activity of extending coronary artery and angiotensin II antagonistic activity as useful pharmacological characteristics, and has also activity of dilating peripheral blood vessel, activity of dilating cerebral blood vessel, activity of dilating nephric blood vessel and the like.

Therefore, the ethynylphenyl derivative of the present invention is useful as preventive and therapeutic agent for circulatory disease, for exmaple, hypertension, ischemic cardiac disease such as stenocardia and cardiac infarction, cardiac failure and the like in human.

BRIEF EXPLANATION OF THE DRAWINGS

Fig. 1 is a graph showing relationship between time and amount of change in blood pressure with respect to persistant activity as a vasodepressor of the compound of the present invention orally administered in rat. Fig. 2 is a graph showing relationship between intravenous dosage and change rate in PQ interval with respect to activity of the compound of the present invention against atrioventricular system in rat. Fig. 3 is a graph showing relationship between intravenous dosage and amount of ST wave with respect to activity of the compound of the present invention against model of vasopressin myocardial ischemia. And Fig. 4 is a graph showing relationship between intravenous dosage of the compound of the present invention and change rates in vertebral blood flow and coronary blood flow.

BEST MODE FOR CARRYING OUT THE INVENTION

The results of pharmacological tests which prove activities and pharmaceutical effects of the etynylphenyl derivatives and acid addition salts thereof, the compounds of the present invention, are shown in following Test Examples.

Test Example 1-1

[Activity against blood pressure]

Male spontaneously hypertensive rats (20 to 30 weeks) were employed. After being heated in a heating box at 45° C for about 5 minutes, the rats were put in holders for measuring blood pressure, and blood pressure cuffs were set on the rats. After the rat's heart rate was stabilized, blood pressure of each rat was measured by a sphygmomanometer for rat (8002e, made by W+W Co., Ltd.) before the administration, and

one hour, three hours and six hours after the administration. Each test compound dissolved in a 0.5 % solution of methyl cellulose was administered orally in a volume of 5 mℓ/kg.

Change of the blood pressure was calculated by the following formula.

Change rate (%) = [(Measured value after administration - Measured value before administration)/Measured value before administration] x 100

The results are shown in Table 2.

Table 2

| Test compound No. | 4 | 5 | 1 | 26 | 2 | 18 | 37 | Nimodipine | Nicardipine |
|---|---|---|---|---|---|---|---|---|---|
| Dose (mg/kg) | 10 | 10 | 10 | 30 | 30 | 10 | 30 | 10 | 10 |
| Change rate of blood pressure | | | | | | | | | |
| 1 hr. value | -41.2% | -41.5% | -39.0% | -38.8% | -25.0% | -5.3% | -38.1% | -11.0% | -35.0% |
| 3 hr. value | -40.9% | -41.5% | -45.2% | -43.8% | -22.5% | -12.4% | -44.7% | -11.0% | -3.5% |
| 6 hr. value | -35.7% | -29.2% | -43.3% | -41.5% | -10.0% | -14.3% | -48.7% | -10.2% | -2.0% |

Test Example 1-2

[Duration of blood pressure reduction activity]

Spontaneous hypertensive male rats (35 weeks) were employed. Before the day the test was carried out, rats were anesthetized with ether, and then, a polyethylene tube was inserted into an aorta abdominalis of the each rat. The other end of the polyethylene tube was led out of the rat's body, and the led polyethylene tube was connected with a pressure transducer. Blood pressure of each rat was measured by a polygraph (RM-85, made by Nihon Koden Kohgyo Co., Ltd.) without anesthesia before the administration, at intervals of one hour for eight hours after the administration and 24 hours after the administration.

Each test compound disolved in a 0.5 % solution of methyl cellulose was administered orally in a volume of 5 mℓ/kg and in a dose of 3 mg/kg or 10 mg/kg.

The results are shown in Figure 1. As is clear from Figure 1, blood pressure reduction activity of the compound No. 1 lasted for at least eight hours.

Test Example 2

[Activity against atrioventricular conduction system in rats]

Wistar rats weighing 400 to 500 g were employed. Rats were anesthetized by intraperitoneally administering 50 mg/kg of pentobarbital sodium, and electrocardiogram of each rat was recorded in lead II. PQ interval (interval from a begining point of P wave to a begining point of Q wave) in electrocardiogram was measured. Each test compound adjusted with a mixed solution containing 20 % of propylene glycol and 5 % of glucose, was administered additionally in a volume of 0.5 mℓ/kg in order through a polyethylene tube which was inserted into femoral vein. Electrocardiogram was recorded every one minute for five minutes after the administration at a chart speed of 100 mm/sec.

Activity against PQ interval of a test compound was calculated by the following formula.

Change rate (%) = [(PQ interval after administration - PQ interval before administration)/PQ interval before administration] x 100

The results are shown in Figure 2. As is clear from Figure 2, the compound No. 1 has a less influence on artrioventricular conduction system as compared with nicardipine.

Test Example 3

[Activity against a model of vasopressin myocardial ischemia]

In accordance with a method of Hiramatu et al. (Japanese Journal Pharmacology 20,313 to 324,1970), Donryu strain male rats weighing 142 to 265 g were employed. Rats were anesthetized by intraperitoneally administering 60 mg/kg of pentobarbital sodium. After intravenous administration of 0.3 µg/kg of vasopressin, electrocardiogram (lead II) was recorded every one minute for five minutes. The maximum depression of ST wave in electrocardiogram was measured as an indication of myocardial ischemia. Five minutes before the administration of vasoperessin, each test compound dissolved in a mixted solution containing 20 % of propylen glycol and 5 % of glucose, was administered intravenously in a dose of 1 mℓ/kg.

The results are shown in Figure 3. As is clear from Figure 3, the compound No. 1 showed greater antimyocardial ischemia activity than that of nicardipine or nifedipine.

Test Example 4

[Activity against blood flow in anesthetized dogs]

Beagle dogs weighing 7 to 14 kg were employed, irrespective of sex. Dogs were anesthetized by intravenously administering 30 mg/kg of pentobarbital sodium (Nembutal). During the test 5 mg/kg/min of pentobarbital was injected continually into each dog. A endotracheal tube was inserted into each trachea. Incision was performed along the midline of the chest and a heart was exposed. Probes of a electromagnetic flowmeter (made by Nihon Koden Kohgyo Co., Ltd.) were set on a left circumflex coronary artery and a right vertebral artery. Each parameter was recorded simultaneously by polygragh (made by Nihon Koden Kogyo Co., Ltd.). Each test compound, which was diluted and adjusted to 2 mg/mℓ with a mixed solution containing 20 % of propylen glycol and 5 % of glucose, was administered additionally in a volume of 0.5 mℓ in order through a polyethylene tube inserted into femoral vein. Change of blood flow was calculated by

the following Change rate.

Change rate (%) = [(Measured value after administration - Measured value before administration)/Measured value before administration] x 100

The results are shown in Figure 4. As is clear from Figure 4, the compound No. 1 showed greater activity for increasing coronary and vertebral blood flow as compared with nicardipine or nimodipine.

Test Example 5

[Inhibitory activity against angiotensin II]

From Hartrey male guinea pigs weighing about 500 g, ileum preparations were prepared. Ileum preparations were suspended with a load of 0.5 g in a Tyrode's solution (32° C) saturated with mixed gas (95% oxygen - 5 % carbon dioxide). Thereinto 3 x $10^{-8}$M of angiotensin II was added and constriction response of each preparation was measured through a isometoric transducer (made by Nihon Koden Kogyo Co., Ltd.). Before the addition of angiotensin II, a compound of the present invention or saralasin was previously added into a Magnus trough. Contractive responses in both cases were compared and Inhibition rate was calculated by the following formula.

Angiotensin II Inhibition rate = (1 - Tension in case of adding test compound/Tension of control) x 100

The results are shown in Table 3.

Table 3

| Test compound | Concentration (M) | Angiotensin II Inhibition rate (%) |
|---|---|---|
| Compound No. 1 | $10^{-8}$ | 6.5 |
| | $10^{-7}$ | 56.2 |
| | $10^{-6}$ | 71.1 |
| Saralasin | $10^{-7}$ | 47.3 |

As is clear from Table 3, the compound No. 1 showed the same extent of Inhibitory activity against angiotension II as that of saralasin which is a positive control drug.

Test Example 6

[Activity against convulsion caused by pentylenetetrazole]

Slc: ddy male mice weighing 24 to 32 g (5 weeks) were bred under an environment of 22° to 24° C room temperature and 60 % humidity for 1 week, and then the bred mice were subjected to a test. The test was carried out as the following procedure. One hour after the oral administration of a test compound, 40 mg/kg of pentylenetetrazole was administered in a caudal vein. Appearance of tonic extensive convulsion was measured as an indication, and anticonvulsive activity was examined. Each test compound was suspended in a 0.5 % solution of methyl cellulose, which was used for a control group. The results are

shown in Table 4.

Table 4

| Test compound | Dose (mg/kg) | Number of appearance of tonic extensive convulsion |
|---|---|---|
| Control (0.5% methyl cellulose) | - | 4 cases/4 cases |
| Compound No. 1 | 50 | 4 cases/4 cases |
| | 200 | 4 cases/4 cases |
| Nimodipine | 25 | 3 cases/4 cases |
| | 50 | 1 case /4 cases |
| | 100 | 1 case /4 cases |
| Nicardipine | 25 | 4 cases/4 cases |
| | 50 | 1 case /4 cases |
| | 100 | no cases /4 cases |
| Diphenylhidantoin | 6.25 | 2 cases/4 cases |
| | 12.5 | 1 case /4 cases |
| | 25 | 1 case /8 cases |
| | 50 | no cases/8 cases |
| | 100 | no cases/4 cases |

As is shown in Table 4, in contrast to nimodipine, nicardipine and dephenylhidantoin showing anticonvulsive activity, the compound No. 1 did not show the activity.

Test Example 7

[Acute toxicity]

In each group 5 Slc: Wistar male rats weighing 100 g to 114 g (4 weeks) were employed. The compound No. 1 was administered orally thereto and with death rate after 7 days a value of acute toxicity was measured.

Result

Rat $LD_{50}$ = 300 to 1000 mg/kg (oral administration)

It was demonstrated that the compound of the present invention has very low toxicity.

The present invention is more specifically described and explained by means of the following Examples. However, the present invention is not limited by these Examples.

Example 1

[Preparation of dihydrochloride salt of 4-(3-ethynylphenyl)-3-methoxycarbonyl-2,6-dimethyl-5-(N-diphenylmethylpiperazinylethoxycarbonyl)-1,4-dihydropyridine]

In 80 mℓ of 2-propanol were dissolved 7.8 g (0.06 mol) of 3-ethynylbenzaldehyde, 20 g (0.053 mol) of 2-(N-diphenylmethyl-piperazinyl) ethylacetoacetate and 6.8 g (0.06 mol) of methyl 3-aminocrotonate, and the solution was heated under reflux for 8.5 hours. After completing the reaction, the reaction solution was concentrated under reduced pressure. The residue was purified by subjecting to silica gel column chromatography [eluent: n-hexane-ethylacetate (1:1)] to give the free base of the desired compound as oil. The oil was dissolved in isopropylether, and the solution was added dropwise into cooled hexane over stirring to give 15.5 g of the free base of the desired compound as powder (yield: 50.0 %).

In 150 mℓ of ethanol and 40 mℓ of 2N-hydrochloric acid was dissolved 15.5 g of the obtained free base of the desired compound. The solution was concentrated under reduced pressure. To the residue was added 100 mℓ of ethanol, and further, the solution was concentrated under reduced pressure to give 14.0 g of the desired compound (yield: 90.1 %)

mp: 200° - 203° C
MS(m/z): 589 ($M^+$),

422 ($M^+$ − 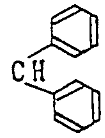 )

IR ($cm^{-1}$): 3360 (NH), 3100-2950 (CH), 1710 (COO)

Example 2

[Preparation of dihydrochloride salt of 4-(2-ethynyl-6-fluorophenyl)-3-methoxycarbonyl-2,6-dimethyl-5-(N-diphenylmethylpiperazinylethoxycarbonyl)-1,4-dihydropyridine]

In Example 2, 9.0 g (0.06 mol) of 2-ethynyl-6-fluorobenzaldehyde was employed instead of 3-ethynylbenzaldehyde employed in Example 1, and the reaction time was 6 hours. The procedure of reaction, treatment and purification of Example 1 were repeated except the above-mentioned conditions to give 13.2 g of the free base of the desired compound (yield: 41.0 %).

The procedure of reaction and treatment of Example 1 were repeated to give 12.5 g of the desired compound from the obtained free base of the desired compound (yield: 83.5 %).

mp: 149° - 152° C
MS(m/z): 607 ($M^+$),

440 ($M^+$ − 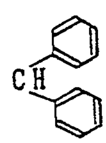 )

IR ($cm^{-1}$): 3410 (NH), 3100-2900 (CH), 1740 (COO), 1710 (COO)

Example 3

[Preparation of dihydrochloride salt of 4-(2-ethynylphenyl)-3-methoxycarbonyl-2,6-dimethyl-5-(N-diphenylmethylpiperazinylethoxycarbonyl)-1,4-dihydropyridine]

In Example 3, 0.91 g (0.007 mol) of 2-ethynylbenzaldehyde was employed instead of 3-ethynylbenzaldehyde employed in Example 1, ethanol was employed instead of 2-propanol, and the reflux time was 5 hours. The procedure of reaction, treatment and purification of Example 1 were repeated except the above-mentioned conditions to give the free base of the desired compound. Then, the procedure of reaction and treatment of Example 1 were repeated to give 1.4 g of the desired compound from the obtained free base of the desired compound (yield: 30.3 %).

mp: 157° - 161° C
MS(m/z): 589 ($M^+$),

422 ($M^+$ − 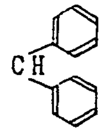 )

IR ($cm^{-1}$): 3300 (NH), 3100-2950 (CH), 1700 (COO)

Example 4

[Preparation of dihydrochloride salt of 4-(3-ethynylphenyl)-3-methoxycarbonyl-2,6-dimethyl-5-(N-di-4-fluorophenylmethylpiperazinylethoxycarbonyl)-1,4-dihydropyridine]

In Example 4, 2.54 g (0.006 mol) of 2-(N-di-4-fluorophenylmethylpiperazinyl)ethylacetoacetate was employed instead of 2-(N-diphenylmethylpiperazinyl)ethylacetoacetate employed in Example 1, ethanol was employed instead of 2-propanol, the reflux time was 6 hours, and chloroform-methanol (100:1) was employed as the eluent of silica gel column chromatography. The procedure of reaction, treatment and purification of Example 1 were repeated except the above-mentioned conditions to give the free base of the desired compound. Then, the procedure of reaction and treatment of Example 1 were repeated to give 1.6 g of the desired compound from the obtained free base (yield: 38 %).

mp: 142° - 146° C

MS(m/z): 625 ($M^+$),

422 ($M^+$ − 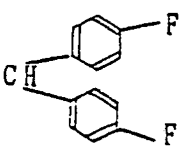 )

IR ($cm^{-1}$): 3350 (NH), 3150-2950 (CH), 1710 (COO)

Example 5

[Preparation of dihydrochloride salt of 4-(3-ethynylphenyl)-3-methoxycarbonyl-2,6-dimethyl-5-(N-di-4-methylphenylmethylpiperazinylethoxycarbonyl)-1,4-dihydropyridine]

In Example 5, 4.2 g (0.01 mol) of 2-(N-di-4-methylphenylmethylpiperazinyl)ethylacetoacetate was employed instead of 2-(N-diphenylmethylpiperazinyl)ethylacetoacetate employed in Example 1, ethanol was employed instead of 2-propanol, the reflux time was 5 hours, and chloroform-n-hexane (3:1) was employed as the eluent of silica gel column chromatography. The procedure of reaction, treatment and purification of Example 1 were repeated except the above-mentioned conditions to give the free base of the desired compound. Then, the procedure of reaction and treatment of Example 1 were repeated to give 2.2 g of the desired compound from the obtained free base (yield: 32.0 %).

mp: 149 - 152° C

MS(m/z): 617 ($M^+$),

422 ($M^+$ − 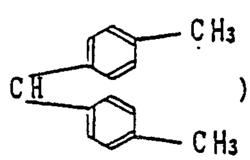 )

IR ($cm^{-1}$): 3400-3250 (NH), 3100-2900 (CH), 1700 - 1680 (COO)

Example 6

[Preparation of dihydrochloride salt of 4-(2-ethynyl-3-chlorophenyl)-3-methoxycarbonyl-2,6-dimethyl-5-(N-diphenylmethylpiperazinylethoxycarbonyl)-1,4-dihydropyridine]

In Example 6, 0.96 g (0.006 mol) of 2-ethynyl-3-chlorobenzaldehyde was employed instead of 3-ethynylbenzaldehyde employed in Example 1, and the reflux time was 7 hours. The procedure of reaction, treatment and purification of Example 1 were repeated except the above-mentioned conditions to give the free base of the desired compound. Then, the procedure of reaction and treatment of Example 1 were repeated to give 1.4 g of the desired compound from the obtained free base (yield: 35.0 %).

mp: 152° - 155° C

MS(m/z): 623 ($M^+$),

456 ($M^+$ − 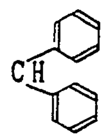 )

IR ($cm^{-1}$): 3400 (NH), 3050-2900 (CH), 1690 (COO)

Example 7

[Preparation of dihydrochloride salt of 4-(3-ethynylphenyl)-3-methoxycarbonyl-2,6-dimethyl-5-(N-diphenylmethylpiperazinylpropoxycarbonyl)-1,4-dihydropyridine]

In Example 7, 3.9 g (0.01 mol) of 2-(N-diphenylmethylpiperazinyl)propylacetoacetate was employed instead of 2-(N-diphenylmethylpiperazinyl)ethylacetoacetate employed in Example 1, ethanol was employed instead of 2-propanol, the reflux time was 12 hours, and chloroform to chloroform-ethylacetate (4:1) was employed as the eluent of silicagel column chromatography. The procedure of reaction, treatment and purification of Example 1 were repeated except the above-mentioned conditions to give 3.9 g of the free base of the desired compound (yield: 64 %).

Then, the procedure of reaction and treatment of Example 1 were repeated to give 3.8 g of the desired compound from 3.9 g of the obtained free base of the desired compound (yield: 56 %).

mp: 145° - 147° C

MS(m/z): 603 ($M^+$),

436 ($M^+$ − 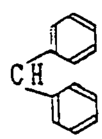 )

IR ($cm^{-1}$): 3350 (NH), 3100-2900 (CH), 1700 - 1690 (COO)

Example 8

[Preparation of dihydrochloride salt of 4-(3-ethynyphenyl)-3-methoxycarbonyl-2,6-dimethyl-5-(N-diphenylmethylpiperazinylbutoxycarbonyl)-1,4-dihydropyridine]

In Example 8, 3.5 g (0.008 mol) of 2-(N-diphenylmethylpiperazinyl)butylacetoacetate was employed instead of 2-(N-diphenylmethylpiperazinyl)ethylacetoacetate employed in Example 1, ethanol was employed instead of 2-propanol, the reflux time was 12 hours, and chloroform to chloroform-ethylacetate (4:1) was employed as the eluent of silica gel column chromatography. The procedure of reaction, treatment and purification of Example 1 were repeated except the above-mentioned conditions to give the free base of the desired compound. Then, the procedure of reaction and treatment of Example 1 were repeated to give 0.73 g of the desired compound from the obtained free base (yield: 14 %).

mp: 128° - 131° C

MS(m/z): 617 ($M^+$),

516 ($M^+$ - 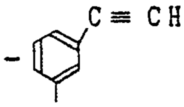 )

450 ($M^+$ - 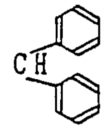 )

IR ($cm^{-1}$): 3400 (NH), 3050-2850 (CH), 1690 (COO)

Example 9

[Preparation of dihydrochloride salt of 4-(3-ethynylphenyl)-3-isopropoxycarbonyl-2,6-dimethyl-5-(N-diphenylmethylpiperazinylethoxycarbonyl)-1,4-dihydropyridine]

The procedure of reaction, treatment and purification of Example 1 were repeated except that 2.9 g (0.02 mol) of isopropyl-3-aminocrotonate was employed instead of 3-aminocrotonate employed in Example 1 to give the free base of the desired compound. Then, the procedure of reaction and treatment of Example 1 were repeated to give 6.0 g of the desired compound from the obtained free base (yield: 43.5 %).

mp: 148° - 151° C

MS(m/z): 617 ($M^+$),

450 ($M^+$ - 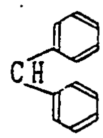 )

IR ($cm^{-1}$): 3350 (NH), 3100-2900 (CH), 1700 (COO)

Example 10

[Preparation of dihydrochloride salt of 4-(3-ethynylphenyl)-3-n-propoxycarbonyl-2,6-dimethyl-5-(N-di-4-fluorophenylmethylpiperazinylethoxycarbonyl)-1,4-dihydropyridine]

In Example 10, 1.4 g (0.01 mol) of n-propyl 3-aminocrotonate was employed instead of 3-aminocrotonate employed in Example 1, 4.2 g (0.01 mol) of 2-(N-di-4-fluorophenylmethylpiperazinyl)-ethylacetoacetate was employed instead of 2-(N-diphenylmethylpiperazinyl)ethyl acetoacetate employed in Example 1, and chloform-methanol (100:1) was employed as the eluent of silica gel colum chromatography. The procedure of reaction, treatment and purification of Example 1 were repeated except the above-mentioned conditions to give the free base of the desired compound. Then, the procedure of reaction and treatment of Example 1 were repeated to give 2.1 g of the desired compound from the obtained free base (yield: 28.8 %).

mp: 140° - 143° C

MS(m/z): 653 ($M^+$)

450 ($M^+$ - 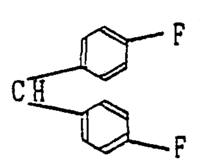 )

IR ($cm^{-1}$): 3400 (NH), 3100-2900 (CH), 1700 (COO)

Example 11

[Preparation of dihydrochloride salt of 4-(3-ethynylphenyl)-3-isopropoxycarbonyl-2,6-dimethyl-5-(N-diphenylmethylpiperazinylpropoxycarbonyl)-1,4-dihydropyridine]

In Example 11, 1.4 g (0.01 mol) of isoprpyl 3-aminocrotonate was employed instead of 3-aminocrotonate employed in Example 1, 3.9 g (0.01 mol) of 2-(N-diphenylmethylpiperazinyl)-propylacetoacetate was employed instead of 2-(N-diphenylmethylpiperazinyl)ethylacetoacetate employed in Example 1, ethanol was employed instead of 2-propanol, and chloroform to chloroformethylacetate (4:1) was employed as the eluent of silica gel column chromatography. The procedure of reaction, treatment and purification of Example 1 were repeated except the above-mentioned conditions to give the free base of the desired compound. Then, the procedure of reaction and treatment of Example 1 were repeated to give 1.5 g of the desired compound from the obtained free base (yield: 22.8 %).

mp: 150 - 153 °C
MS(m/z): 631 ($M^+$),
IR ($cm^{-1}$): 3350 (NH), 3100-2900 (CH), 1700 (COO)

Example 12

[Preparation of dihydrochloride salt of 4-(3-ethynylphenyl)-3-cyclohexyloxycarbonyl-2,6-dimethyl-5-(N-di-4-methylphenylmethylpiperazinylethoxycarbonyl)-1,4-dihydropyridine]

In Example 12, 1.8 g (0.01 mol) of cyclohexyl 3-aminocrotonate was employed instead of 3-aminocrotonate employed in Example 1, 4.2 g (0.01 mol) of 2-(N-di-4-methylphenylmethylpiperazinyl)-ethylacetoacetate was employed instead of 2-(N-diphenylmethylpiperazinyl)ethylacetoacetate employed in Example 1, and chloroform-n-hexane (3:1) was employed as the eluent of silica gel column chromatography. The procedure of reaction, treatment and purification of Example 1 were repeated except the above-mentioned conditions to give the free base of the desired compound. Then, the procedure of reaction and treatment of Example 1 were repeated to give 2.7 g of the desired compound from the obtained free base (yield: 35 %).

mp: 213° - 215 °C
MS(m/z): 685 ($M^+$),

490 ($M^+$ - 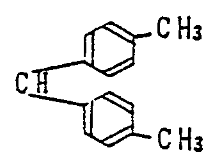 )

IR ($cm^{-1}$): 3400 - 3350 (NH), 3100-2850 (CH), 1710 (COO)

Example 13

[Preparation of dihydrochloride salt of 4-(3-ethynylphenyl)-3-methoxycarbonyl-2,6-dimethyl-5-(N-3-diphenylpropylpiperazinylethoxycarbonyl)-1,4-dihydropyridine]

In Example 13, 5.6 g (0.013 mol) of 2-(N-3-diphenylpropylpiperazinyl) ethylacetoacetate was employed instead of 2-(N-diphenylmethylpiperazinyl)ethylacetoacetate employed in Example 1, ethanol was employed instead of 2-propanol, the reflux time was 13 hours, and n-hexane-dichloromethane (1:1) to dichloromethane-ether (7:3) was employed as the eluent of silica gel column chromatography. The procedure of reaction, treatment and purification of Example 1 were repeated except the above-mentioned conditions to give 1.75 g of the free base (yield 21.9 %). Then, the procedure of reaction and treatment of Example 1 were repeated to give 1.7 g of the desired compound from the obtained free base of the desired compound (yield: 85 %).

mp: 133° - 136 °C
MS(m/z): 617 ($M^+$),

450 ($M^+$ − 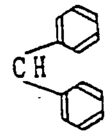 )

IR ($cm^{-1}$): 3400 - 3350 (NH), 3050-2900 (CH), 1690 - 1680 (COO)

Example 14

[Preparation of dihydrochloride salt of 4-(3-ethynylphenyl)-3-methoxyethoxycarbonyl-2,6-dimethyl-5-(N-diphenylmethylpiperazinylethoxycarbonyl)-1,4-dihydropyridine]

In 10 mℓ of ethanol were dissolved 0.8 g (0.005 mol) of methoxyethylacetoacetate and 0.5 g (0.0065 mol) of ammoniumacetate, and the solution was reacted at 80°C for 3 hours. Then, 30 mℓ of ethanol solution of 0.845 g (0.0065 mol) of 3-ethynylbenzaldehyde and 2.47 g (0.0065 mol) of 2-(N-diphenylmethylpiperazinyl)ethylacetoacetate was added to the reaction solution. Further the solution was reacted at 80°C for 16 hours. The reaction solution was concentrated under reduced pressure. The residue was purified by subjecting to silica gel column chromatography [eluent: n-hexane-ethylacetate (1:1)] to give the free base of the desired compound. The procedure of reaction and treatment of Example 1 were repeated to give 0.83 g of the desired compound from the obtained free base (yield: 23 %).

mp: 141° - 143°C

MS(m/z): 633 ($M^+$),

466 ($M^+$ − 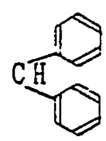 )

IR ($cm^{-1}$): 3250 (NH), 3050-2900 (CH), 1690 (COO)

Example 15

[Preparation of dihydrochloride salt of 4-(3-ethynylphenyl)-3-methoxycarbonyl-2,6-dimethyl-5-(N-diphenylmethylhomopiperazinylethoxycarbonyl)-1,4-dihydropyridine]

In Example 15, 4.0 g (0.0101 mol) of 2-(N-diphenylmethylhomopiperazinyl)ethylacetoacetate was employed instead of 2-(N-diphenylmethylpiperazinyl)ethylacetoacetate employed in Example 1, the reflux time was 3 hours, and n-hexane-ethylacetate (1:1 to 1:2) was employed as the eluent of silica gel column chromatography. The procedure of reaction, treatment and purification of Example 1 were repeated except the above-mentioned conditions to give 2.4 g of the free base of the desired compound (yield: 39.3 %). Then, the procedure of reaction and treatment of Example 1 were repeated to give 2.2 g of the desired compound from the obtained free base (yield: 81.8 %).

mp: 142° - 144.5°C

MS(m/z): 603 ($M^+$),

436 ($M^+$ − 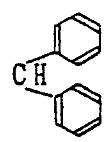 )

IR ($cm^{-1}$): 3400 (NH), 3100-2900 (CH), 1720 (COO)

Example 16

[Preparation of dihydrochloride salt of 4-(3-ethynylphenyl)-3-methoxycarbonyl-2,6-dimethyl-5-(N-di-4-fluorophenylmethylhomopiperazinylethoxycarbonyl)-1,4-dihydropyridine]

The procedure of reaction, treatment and purification of Example 15 were repeated except that 4.3 g (0.0101 mol) of 2-(N-di-4-fluorophenylmethylhomopiperazinyl)ethylacetoacetate employed instead of 2-(N-diphenylmethylhomopiperazinyl)ethylacetoacetate employed in Example 15, to give the free base of the desired compound. Then, the procedure of reaction and treatment of Example 1 were repeated to give 2.6 g of the desired compound from the obtained free base (yield: 35.8 %).

mp: 137° - 140° C

MS(m/z): 639 ($M^+$),

436 ($M^+$ – 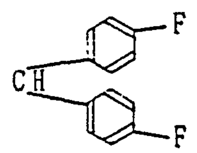 )

IR ($cm^{-1}$): 3400 (NH), 3100-2900 (CH), 1700 (COO)

Example 17

[Preparation of dihydrochloride salt of 4-(2-chloro-3-ethynylphenyl)-3-methoxycarbonyl-2,6-dimethyl-5-(N-diphenylmethylpiperazinylethoxycarbonyl)-1,4-dihydropyridine]

The procedure of reaction, treatment and purification of Example 6 were repeated except that 0.8 g (0.005 mol) of 2-chloro-3-ethynylbenzaldehyde was employed instead of 2-ethynyl-3-chlorobenzaldehyde employed in Example 6, to give the free base of the desired compound. Then, the procedure of reaction and treatment of Example 1 were repeated to give 1.1 g of the desired compound from the otained free base (yield: 30.7 %).

mp: 158° - 161.5° C

MS(m/z): 623 ($M^+$),

456 ($M^+$ – 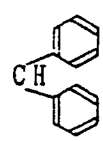 , )

IR ($cm^{-1}$): 3400 (NH), 3100-2900 (CH), 1690 (COO)

Example 18

[Preparation of dihydrochloride salt of 4-(3-ethynylphenyl)-3-methoxycarbonyl-5-[3-(N-2-methoxyphenyl-piperazinyl)propoxycarbonyl]-2,6-dimethyl-1,4-dihydropyridine]

In Example 18, 1.6 g (0.0048 mol) of 3-[N-(2-methoxyphenyl)piperazinyl]propylacetoacetate was employed instead of 2-(N-diphenylmethylpiperazinyl)ethylacetoacetate employed in Example 1, ethanol was employed instead of 2-propanol, and the reflux time was 10 hours, and n-hexane-ethylacetate (1:2) was employed as the eluent of silica gel column chromatography. The procedure of reaction, treatment and purification of Example 1 were repeated except the above-mentioned conditions to give the free base of the desired compound. Then, the procedure of reaction and treatment of Example 1 were repeated to give 1.2 g of the desired compound from the obtained free base (yield: 36.1 %).

mp: 139° - 141° C

MS(m/z): 543 ($M^+$), 528 ($M^+$ -$CH_3$), 512 ($M^+$ -$OCH_3$)

IR ($cm^{-1}$): 3300 - 3250 (NH), 3100-2850 (CH), 1710 - 1690 (COO)

Example 19

[Preparation of dihydrochloride salt of 4-(3-ethynylphenyl)-3-methoxycarbonyl-5-[3-(N-4-fluorophenyl-

piperazinyl)propoxycarbonyl]-2,6-dimethyl-1,4-dihydroxypyridine]

In Example 19, 1.61 g (0.005 mol) of 3-[N-(4-fluorophenyl)piperazinyl]propylacetoacetate was employed instead of 2-(N-diphenylmethylpiperazinyl)ethylacetoacetate employed in Example 1, ethanol was employed instead of 2-propanol, the reflux time was 24 hours, and dichloromethane-ethanol (100:1) was employed as the eluent of silica gel column chromatography. The procedure of reaction, treatment and purification of Example 1 were repeated except the above-mentioned conditions to give 1.6 g of the free base of the desired compound. Then, the procedure of reaction and treatment of Example 1 were repeated to give 1.2 g of the desired compound from the obtained free base (yield: 40 %).

mp: 159.5° - 162° C
MS(m/z): 531 ($M^+$), 516 ($M^+$ -$CH_3$), 500 ($M^+$ -$OCH_3$)
IR ($cm^{-1}$): 3250 (NH), 3100-2850 (CH), 1690 - 1670 (COO)

Example 20

[Preparation of dihydrochloride salt of 4-(2-ethynyl-6-fluorophenyl)-3-methoxycarbonyl-5-[3-(N-4-fluorophenylpiperazinyl)propoxycarbonyl]-2,6-dimethyl-1,4-dihydropyridine]

In Example 20, 0.9 g (0.006 mol) of 2-ethynyl-6-flurorbenzaldehyde was employed instead of 3-ethynylbenzaldehyde employed in Example 1, 1.61 g (0.0053 mol) of 3-[N-(4-fluorophenyl)piperazinyl]-propylacetoacetate was employed instead of 2-(N-diphenylmethylpiperazinyl)-ethlacetoacetate employed in Example 1, the reflux time was 4 hours, and n-hexane-ethylacetate (1:1) to ethylacetate was employed as the eluent of silicagel column chromatography. The procedure of reaction, treatment and purification of Example 1 were repeated except the above-mentioned conditions to give 1.0 g of the free base of the desired compound (yield: 34.6 %). Then, the procedure of reaction and treatment of Example 1 were repeated to give 0.905 g of the desired compound from the obtained free base (yield: 90.5 %).

mp: 105° - 108° C
MS(m/z): 549 ($M^+$), 518 ($M^+$ -$OCH_3$), 490 ($M^+$ -$COOCH_3$)
IR ($cm^{-1}$): 3400 (NH), 3050-2900 (CH), 1730 (COO), 1710 (COO)

Example 21

[Preparation of dihydrochloride salt of 4-(3-ethynylphenyl)-3-methoxycarbonyl-2,6-dimethyl-5-(N-benzyl-piperazinylethoxycarbonyl)-1,4-dihydropyridine]

In Example 21, 3 g (0.01 mol) of 2-(N-benzylpiperazinyl)ethylacetoacetate was employed instead of 2-(N-diphenylmethylpiperazinyl)ethylacetoacetate employed in Example 1, ethanol was employed instead of 2-propanol, the reflux time was 24 hours, and dichloromethane-ethanol (100:1) was employed as the eluent of silica gel column chromatography. The procedure of reaction, treatment and purification of Example 1 were repeated except the above-mentioned conditions to give the free base of the desired compound. Then, the procedure of reaction and treatment of Example 1 were repeated to give 2.6 g of the desired compound from the obtained free base (yield: 50 %).

mp: 162° - 164.5° C
MS(m/z): 513 ($M^+$), 498 ($M^+$ -$CH_3$), 482 ($M^+$ -$OCH_3$)
IR ($cm^{-1}$): 3300 - 3200 (NH), 3100-2900 (CH), 1680 - 1670 (COO)

Example 22

[Preparation of dihydrochloride salt of 4-(3-ethynylphenyl)-3-methoxycarbonyl-2,6-dimethyl-5-(N-4-fluorobenzylpiperazinylethoxycarbonyl)-1,4-dihydropyridine]

The procedure of reaction, treatment and purification of Example 21 were repeated except that 3.2 g (0.01 mol) of 2-(N-4-fluorobenzylpiperazinyl)ethylacetoacetate was employed instead of 2-(N-benzyl-piperazinyl)ethylacetoacetate employed in Example 21 to give the free base of the desired compound. Then, the procedure of reaction and treatment of Example 1 were repeated to give 2.9 g of the desired compound from the obtained free base (yield: 50 %).

mp: 165° - 168° C
MS(m/z): 531 ($M^+$), 516 ($M^+$ -$CH_3$), 500 ($M^+$ -$OCH_3$)

IR ($cm^{-1}$): 3350 - 3200 (NH), 3100-2900 (CH), 1700 (COO)

Example 23

[Preparation of dihydrochloride salt of 4-(3-ethynylphenyl)-3-methoxycarbonyl-2,6-dimethyl-5-(N-3,4-dimethoxybenzylpiperazinylethoxycarbonyl)-1,4-dihydropyridine]

The procedure of reaction, treatment and purification of Example 21 were repeated except that 3.6 g (0.01 mol) of 2-(N-3,4-dimethoxybenzylpiperazinyl)ethylacetoacetate was employed instead of 2-(N-benzylpiperazinyl)ethylacetoacetate employed in Example 21 to give the free base of the desired compound. Then, the procedure of reaction and treatment of Example 1 were repeated to give 2.6 g of the desired compound from the obtained free base (yield: 45 %).

mp: 144° - 147° C
MS(m/z): 573 ($M^+$), 558 ($M^+$ -$CH_3$), 542 ($M^+$ -$OCH_3$)
IR ($cm^{-1}$): 3400 - 3300 (NH), 3100-2850 (CH), 1700 (COO)

Example 24

[Preparation of dihydrochloride salt of 4-(3-ethynylphenyl)-3-methoxycarbonyl-2,6-dimethyl-5-(N-phenylethylpiperazinylethoxycarbonyl)-1,4-dihydropyridine]

The procedure of reaction, treatment and purification of Example 21 were repeated except that 3.2 g (0.01 mol) of 2-(N-phenylethylpiperazinyl)ethylacetoacetate was employed instead of 2-(N-benzylpiperazinyl)ethylacetoacetate employed in Example 21 to give the free base of the desired compound. Then, the procedure of reaction and treatment of Example 1 were repeated to give 2.6 g of the desired compound from the obtained free base (yield: 55 %).

mp: 134° - 137° C
MS(m/z): 527 ($M^+$), 512 ($M^+$ -$CH_3$), 496 ($M^+$ -$OCH_3$)
IR ($cm^{-1}$): 3300 - 3200 (NH), 3100-2950 (CH), 1700 - 1690 (COO)

Example 25

[Preparation of dihydrochloride salt of 4-(2-ethynyl-6-fluorophenyl)-3-methoxycarbonyl-2,6-dimethyl-5-(N-benzylpiperazinylpropoxycarbonyl)-1,4-dihydropyridine]

In Example 25, 1.3 g (0.01 mol) of 2-ethynyl-6-fluorobenzaldehyde was employed instead of 3-ethynylbenzaldehyde employed in Example 21, and 3.2 g (0.01 mol) of 2-(N-benzylpiperazinyl)-propylacetoacetate was employed instead of 2-(N-benzylpiperazinyl)ethylacetoacetate employed in Example 21. The procedure of reaction, treatment and purification of Example 21 were repeated except the above-mentioned conditons to give the free base of the desired compound. Then, the procedure of reaction and treatment of Example 1 were repeated to give 2.5 g of the desired compound from the obtained free base (yield: 40 %).

mp: 121.5° - 124.5° C
MS(m/z): 545 ($M^+$), 530 ($M^+$ -$CH_3$), 514 ($M^+$ -$OCH_3$)
IR ($cm^{-1}$): 3300 - 3200 (NH), 3100-2900 (CH), 1700 (COO)

Example 26

[Preparation of dihydrochloride salt of 4-(3-ethynylphenyl)-3-ethoxycarbonyl-2-amino-6-methyl-5-(N-diphenylmethylpiperazinylethoxycarbonyl)-1,4-dihydropyridine]

In 80 mℓ of ethanol were dissolved 3.2g (0.007 mol) of 2-(3-ethynylbenzylidene)acetoacetate (2-N-diphenylmethylpiperazinyl)ethylester and 1.27 g (0.01 mol) of ethoxycarbonylacetoamidine, and the solution was heated under reflux for 8 hours. After completing the reaction, the reaction solution was concentrated under reduced pressure. The residue was dissolved in ethylacetate, washed with water, dehydrated and then, concentrated. The residue was purified by subjecting to silica gel column chromatography [eluent: chloroform-ethanol (100:1)] to give 1.1 g of the free base of the desired compound (yield: 28 %). In 10 mℓ of ethanol and 2 equivalent of 1.0 mℓ of 2N hydrochloric acid was dissolved 1.1 g of the obtained free base

of the desired compound, then the procedure of reaction and treatment of Example 1 were repeated to give 1.01 g of the desired compound (yield: 91.7 %).

mp: 167° - 168° C

MS(m/z): 604 ($M^+$), 531 ($M^+$ -$COOC_2H_5$),

437 ($M^+$ - 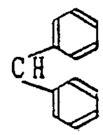 )

IR ($cm^{-1}$): 3300 - 3200 ($NH_2$, NH), 3100-2900 (CH), 1740 - 1720 (COO)

Example 27

[Preparation of dihydrochloride salt of 4-(3-ethynylphenyl)-3-n-propoxycarbonyl-2-amino-6-methyl-5-(N-di-4-chlorophenylmethylpiperazinylethoxycarbonyl)-1,4-dihydropyridine]

In Example 27, 3.9 g (0.007 mol) of 2-(3-ethynylbenzylidene)acetoacetate (2-N-di-4-chlorophenylmethyl-piperazinyl)ethylester was employed instead of 2-(3-ethynylbenzylidene)acetoacetate (2-N-diphenylmethyl-piperazinyl)ethylester employed in Example 26, and 1.4 g (0.01 mol) of n-propoxycarbonylacetoamidine instead of ethoxycarbonylacetoamidine employed in Example 26. The procedure of reaction, treatment and purification of Example 26 were repeated except the above-mentioned conditions to give 1.0 g of the desired compound (yield: 20.3 %).

mp: 172° - 174° C

MS(m/z): 686 ($M^+$), 688 ($M^+$ +2), 671 ($M^+$ -$CH_3$), 655 ($M^+$ -$OCH_3$)

IR ($cm^{-1}$): 3350 - 3200 ($NH_2$, NH), 3100-2900 (CH), 1740 - 1720 (COO)

Example 28

[Preparation of dihydrochloride salt of 4-(3-ethynylphenyl)-3-methoxyethoxycarbonyl-2-amino-6-methyl-5-(N-diphenylmethylpiperazinylethoxycarbonyl)-1,4-dihydropyridine]

The procedure of reaction, treatment and purification of Example 26 were repeated except that 1.6 g (0.01 mol) of methoxyethoxycarbonylacetoamidine was employed instead of ethxycarbonylacetoamidine employed in Example 26 to give 1.3 g of the desired compound (yield: 30 %).

mp: 178° - 180° C

MS(m/z): 634 ($M^+$), 619 ($M^+$ -$CH_3$), 603 ($M^+$ -$OCH_3$)

IR ($cm^{-1}$): 3300 - 3200 ($NH_2$, NH), 3100 - 2850 (CH), 1730 - 1710 (COO)

Example 29

[Preparation of dihydrochloride salt of 4-(2-ethynyl-6-fluorophenyl)-3-methoxycarbonyl-2-amino-6-methyl-5-(N-diphenylmethylpiperazinylethoxycarbonyl)-1,4-dihydropyridine]

In Example 29, 3.6 g (0.007 mol) of 2-(2-ethynyl-6-fluorobenzylidene)acetoacetate (2-N-diphenylmethyl-piperazinyl)ethylester was employed instead of 2-(3-ethynylbenzylidene)acetoacetate (2-N-diphenylmethyl-piperazinyl)ethylester employed in Example 26, and 1.1 g (0.01 mol) of methoxycarbonylacetoamidine was employed instead of ethoxycarbonylacetoamidine employed in Example 26. The procedure of reaction, treatment and purification of Example 26 were repeated except the above-mentioned conditions to give 1.1 g of the desired compound (yield: 26.5 %).

mp: 162° - 164° C

MS(m/z): 608 ($M^+$), 593 ($M^+$ -$CH_3$), 577 ($M^+$ -$OCH_3$)

IR ($cm^{-1}$): 3300 - 3200 ($NH_2$, NH), 3100 - 2850 (CH), 1735 - 1720 (COO)

Example 30

[Preparation of dihydrochloride salt of 4-(2-ethynyl-6-fluorophenyl)-3-methoxycarbonyl-2-amino-6-methyl-5-(N-benzylpiperazinylethoxycarbonyl)-1,4-dihydropyridine]

In Example 30, 3.0 g (0.007 mol) of 2-(2-ethynyl-6-fluorobenzylidene)acetoacetate (2-N-benzylpiperazinyl)ethylester was employed instead of 2-(3-ethynylbenzylidene)acetoacetate (2-N-diphenylmethylpiperazinyl)ethylester employed in Example 26, and 1.1 g (0.01 mol) of methoxycarbonylacetoamidine instead of ethoxycarbonylacetoamidine employed in Example 26. The procedure of reaction, treatment and purification of Example 26 were repeated except the above-mentioned conditions to give 1.2 g of the desired compound (yield: 31.1 %).

mp: 180° - 182° C
MS(m/z): 532 ($M^+$), 517 ($M^+$ $-CH_3$), 501 ($M^+$ $-OCH_3$)
IR ($cm^{-1}$): 3300 - 3200 ($NH_2$, NH), 3100 - 2900 (CH), 1740 - 1720 (COO)

Example 31

[Preparation of dihydrochloride salt of 4-(3-ethynylphenyl)-3-methoxycarbonyl-2-dimethoxymethyl-6-methyl-5-(N-diphenylmethylpiperazinylethoxycarbonyl)-1,4-dihydropyridine]

Into a vessel cooled with dry ice and acetone, about 30 mℓ of liquid ammonia was introduced. Thereinto 0.9 g (0.0024 mol) of acetoacetate (2-N-diphenylmethylpiperazinyl)ethylester was added, and the solution was stirred for 1 hour under cooling condition. After reacting at room temperature over night, ammonia was removed therefrom. Thereto 0.6 g (0.0024 mol) of 4,4-dimethoxy-2-(3-ethynylbenzylidene)-acetoacetatemethyl was added, and the solution was reacted at 70° C for 1 hour, then, at 120° C for 2.5 hours. After completing the reaction, ethylacetate was added to the reaction solution. The organic layer was washed with water, dehydrated and concentrated to give 1.6 g of the desired compound as oil (yield: 94 %)

MS(m/z): 649 ($M^+$)
IR ($cm^{-1}$): 3300 - 3200 (NH), 3100 - 2900 (CH), 1700 (COO)

Example 32

[Preparation of dihydrochloride salt of 4-(3-ethynylphenyl)-3-methoxycarbonyl-2-formyl-6-methyl-5-(N-diphenylmethylpiperazinylethoxycarbonyl)-1,4-dihydropyridine]

In mixture of 1.2 mℓ of 6N-hydrochloride solution and 12 mℓ of acetone was dissolved 1.6 g (0.0025 ml) of 4-(3-ethynylphenyl)-3-methoxycarbonyl-2-dimethoxymethyl-6-methyl-5-(N-diphenylmethylpiperazinylethoxycarbonyl)-1,4-dihydropyridine synthesized in Example 31. The solution was reacted at room temperature for 4 hours. After completing the reaction, acetone was distilled away therefrom. The pH of the solution was adjusted to pH 7.5 with saturated aqueous solution of sodium bicarbonate. The solution was extracted with ethylacetate. The organic layer was dryed, and concentrated. The residue was purified by subjecting to silicagel column chromatography [eluent: n-hexane-ethylacetate (1:1)] to give 0.96 g of the desired compound (yield: 64 %).

mp: 101° - 102° C
MS(m/z): 603 ($M^+$)
IR ($cm^{-1}$): 2100 (C≡CH), 1700 (COO), 1685 (CHO)

Example 33

[Preparation of dihydrochloride salt of 4-(3-ethynylphenyl)-3-methoxycarbonyl-2-cyano-6-methyl-5-(N-diphenylmethylpiperazinylethoxycarbonyl)-1,4-dihydropyridine]

In 12 mℓ of ethylacetate was dissolved 0.8 g (0.0013 mol) of 4-(3-ethynylphenyl)3-methoxycarbonyl-2-formyl-6-methyl-5-(N-diphenylmethylpiperazinylethoxycarbonyl)-1,4-dihydropyridine obtained in Example 32. Thereinto 120 mg of hydroxylamine hydrochloride salt and then 180 mg of sodium acetic anhydride were added. The solution was reacted at room temperature for 2.5 hours. Then 520 mg of acetic anhydride was added thereinto, and the solution was reacted for 1.5 hours. Further the solution was reacted at 95° to 100° C for 4 hours. After completing the reaction, acetic anhydride was distilled away therefrom under redused pressure. Water was added to the residue. The solution was neutralized with saturated aqueous solution of sodium bicarbonate, extracted with ethylacetate. The solution of ethylacetate was washed with water, dehydrated and concentrated. The residue was purified by subjecting to silica gel column chromatography [eluent: n-hexaneethylacetate (4:1)] to give 0.32 g of the desired compound (yield: 40 %).

mp: 115° C

MS(m/z): 600 ($M^+$), 573 ($M^+$ -HCN),

433 ($M^+$ − 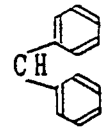 )

IR ($cm^{-1}$): 3260 (NH), 2240 (CN), 2100 (C≡CH), 1700 (COO)

Example 34

[Preparation of dihydrochloride salt of 4-(3-ethynylphenyl)-3-isopropoxycarbonyl-2-dimethoxymethyl-6-methyl-5-(N-diphenylmethylpiperazinylethoxycarbonyl)-1,4-dihydropyridine]

The procedure of reaction, treatment and purification of Example 31 were repeated except that 0.68 g (0.0024 mol) of 4,4-dimethoxy-2-(3-ethynylbenzylidene)acetate methyl ester was employed instead of 4,4-dimethoxy-2-(3-ethynylbenzylidene)acetoacetate methyl ester employed in Example 31 to give 1.2 g of the desired compound as oil (yield: 90.5 %).

MS(m/z): 677 ($M^+$)

IR ($cm^{-1}$): 3300 -3250 (NH), 3100 - 2900 (CH), 1700 (COO)

Example 35

[Preparation of dihydrochloride salt of 4-(3-ethynylphenyl)-3-isopropoxycarbonyl-2-formyl-6-methyl-5-(N-diphenylmethylpiperazinylethoxycarbonyl)-1,4-dihydropyridine]

The procedure of reaction, treatment and purification of Example 32 were repeated except that 1.7 g (0.0025 mol) of 4-(3-ethynylphenyl)-3-isopropoxycarbonyl-2-dimethoxymethyl-6-methyl-5-(N-diphenylmethylpiperazinylethoxycarbonyl)-1,4-dihydropyridine synthesized in Example 34 was employed instead of 4-(3-ethynylphenyl)-3-methoxycarbonyl-2-dimethoxymethyl-6-methyl-5-(N-diphenylmethylpiperazinylethoxycarbonyl)-1,4-dihydropyridine employed in Example 32 to give 1.1 g of the desired compound (yield: 67 %).

mp: 108° - 110° C

MS(m/z): 631 ($M^+$)

IR ($cm^{-1}$): 2100 (C≡CH), 1700 (COO), 1690 (CHO)

Example 36

[Preparation of dihydrochloride salt of 4-(3-ethynylphenyl)-3-isopropoxycarbonyl-2-cyano-6-methyl-5-(N-diphenylmethylpiperazinylethoxycarbonyl)-1,4-dihydropyridine]

The procedure of reaction, treatment and purification of Example 33 were repeated except that 0.82 g (0.0013 mol) of 4-(3-ethynylphenyl)-3-isopropoxycarbonyl-2-formyl-6-methyl-5-(N-diphenylmethylpiperazinylethoxycarbonyl)-1,4-dihydropyridine obtained in Example 35 was employed instead of 4-(3-ethynylphenyl)-3-methoxycarbonyl-2-formyl-6-methyl-5-(N-diphenylmethylpiperazinylethoxycarbonyl)-1,4-dihydropyridine employed in Example 33 to give 0.32 g of the desired compound (yield: 39.7 %).

mp: 121° - 123° C

MS (m/z): 628 ($M^+$), 601 ($M^+$ -HCN),

461 ($M^+$ − 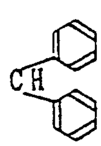 )

IR ($cm^{-1}$): 3260 (NH), 2240 (CN), 2100 (C CH), 1710 (COO)

Example 37

[Preparation of dihydrochloride salt of 4-(3-ethynylphenyl)-3-methoxycarbonyl-2,6-dimethyl-5-[2-(N-diphenylmethylpiperazinyl)-1-methylethoxycarbonyl]-1,4-dihydropyridine]

The procedure of reaction, treatment and purification of Example 1 were repeated except that 4.0 g (0.0101 mol) of 2-(N-diphenylmethylpiperazinyl)-1-methylethylacetoacetate was employed instead of 2-(N-diphenylmethylpiperazinyl)ethylacetoacetate empolyed in Example 1 to give 2.5 g of the free base of the desired compound (yield: 41.0 %). Then, the procedure of reaction and treatment of Example 1 were repeated to give 2.25 g of the desired compound from the obtained free base (yield: 90.1 %).

mp: 177° - 180° C

MS (m/z): 603 ($M^+$),

436 ($M^+$ – 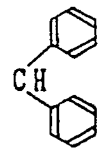 )

IR ($cm^{-1}$): 3360 (NH), 3100 - 2950 (CN), 1710 (COO)

Example 38

[Preparation of dihydrochloride salt of 4-(3-ethynylphenyl)-3-methoxycarbonyl-2,6-dimethyl-5-[2-(N-diphenylmethylpiperazinyl)-1-dimethylethoxycarbonyl)]-1,4-dihydropyridine]

The procedure of reaction, treatment and purification of Example 1 were repeated except that 4.1 g (0.0101 mol) of 2-(N-diphenylmethylpiperazinyl)-1,1-dimethylethylacetoacetatewas employed instead of 2-(N-diphenylmethylpiperazinyl)ethylacetoacetate empolyed in Example 1 to give 2.4 g of the free base of the desired compound (yield: 39.0 %). Then, the procedure of reaction and treatment of Example 1 were repeated to give 2.3 g of the desired compound from the obtained free base (yield: 86.0 %).

mp: 227° - 230° C

MS (m/z): 617 ($M^+$),

($M^+$ – 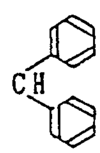 )

IR ($cm^{-1}$): 3360 (NH), 3100 - 2800 (CN), 1710 (COO)

Example 39

In a centrifugal flow system for agglomerating, glanulating and coating, 960 g of lactose (100 mesh) was sprayed and coated with 1000 mℓ of a solution in which 10 g of the compound No. 37 of the present invention and 30 g of hydroxypropyl-methylcellulose were completely dissolved in a mixture of ethanol and methylene chloride (1 : 1 by volume) to give granules according to a conventional method. After drying them for 4 hours at 40° C, the compound No. 37 was granulated to give a granule according to a conventional method.

Example 40

In a centrifugal flow system for agglomerating, glanulating and coating (CF-360 Type, made by FROINT

SANGYO Co., Ltd.,) 1590 g of lactose (100 mesh, made by DMV Co., Ltd.) was sprayed and coated with 5000 mℓ of a solution in which 100 g of the compound No. 1 of the present invention and 300 g of hydroxypropylmethylcellulose 2910 (HMPC 2910, made by SHINETSUKAGAKU Co., Ltd.) are completely dissolved in ethanol and methylene chloride (1 : 1, by volume) to give granules according to a conventional method. After drying them for 4 hours at 40°C, the compound No. 1 was granulated according to a conventional method. After 10 g of magnesium stearate was added thereto and mixed, the mixture was filled up into capsules to give a capsule.

Example 41

In 200 mℓ of ethanol were dissolved 10 g of the compound No. 4 of the present invention and 30 g of polyvinyl pyrrolidone, and then ethanol was distilled away with drying under reduced pressure. The residue was pulverized to powder. Thereto were added 20 g of lactose, 19 g of calcium carboxymethanol and 1 g of magnesium stearate. According to a conventional method, the mixture was compressed to give tablets containing 10 mg of the compound No. 4 per tablet.

Example 42

The powder obtained in Example 38, 50 g of corn starch, 60 g of lactose and gelatinized starch were mixed to give granules. Thereto was added 2 g of magnesium sterate, and the mixture was compressed according to a conventional method to give sublingual tablets containing 10 mg of the compound No. 4 per tablet.

Example 43

To the mixture solution of 50 g of microcrystalline wax and 100 g of paraffin fused with heating was added 40 g of white soft paraffin and the mixture was kneaded together and transferred into a chaser mill. Separately, isopropyl myristate solution containing 10 g of the compound No. 1 of the present invention was prepared. The prepared solution was gradually added to the mixture with stirring. The mixture was kneaded to give an ointment.

Example 44

In 150 mℓ of 90 % ethanol was dissolved the compound No. 1 of the present invention . Then the solution was added to the distilled water for injection containing 150 mℓ of propylene glycol, 2 g of sodium citrate and 0.3 g of citric acid to give total amount of 600 mℓ of an injection.

Example 45

Various components consisting of 5 g of the compound No. 1 of the present invention, 25 g of polyvinyl pyrrolidone, 5 g of polyethylene glycol 400, 25 g of magnesium alumino meta silicate, 137 g of mixture of starch and anhydrous calcium phosphate (starch : anhydrous calcium phosphate = 8 : 2) and 1 g of magnesium stearate were mixed in such proportion and compressed with shaping to give tablets containing 5 mg of the compound NO. 1 per tablet according to a conventional method.

## Claims

**1.** An ethynylphenyl derivative having the formula (I):

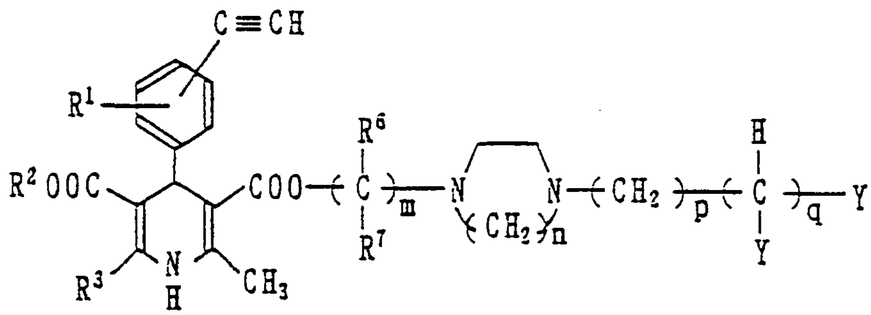

(I)

wherein $R^1$ is hydrogen atom or a halogen atom, $R^2$ is a lower alkyl group or a lower alkoxyalkyl group, $R^3$ is methyl group, amino group, cyano group, formyl group or dimethoxymethyl group, Y is the formula:

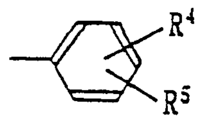

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

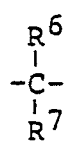

can be the same or different from each other, n is an integer of 2 or 3, p is an integer of 0 to 2 and q is an integer of 0 or 1, or a pharmaclogically acceptable salt thereof.

2. The ethynylphenyl derivative or a pharmacologically acceptable salt thereof of Claim 1, wherein ethynyl group is substituted at the 3-position (meta position).

3. The ethynylphenyl derivative or a pharmacologically acceptable salt thereof of Claim 2, wherein $R^1$ is hydrogen atom.

4. The ethynylphenyl derivative or a pharmacologically acceptable salt thereof of Claim 2 or 3, wherein $R^2$ is a lower alkyl group.

5. The ethynylphenyl derivative or a pharmacologically acceptable salt thereof of Claim 4, wherein $R^3$ is methyl group.

6. The ethynylphenyl derivative or a pharmacologically acceptable salt thereof of Claim 5, wherein $R^4$ and $R^5$ are the same and hydrogen atom.

7. The ethynylphenyl derivative or a pharmacologically acceptable salt thereof of Claim 3, wherein m is 2, n is 2, p is 0 and q is 1.

8. A process for preparing an ethynylphenyl derivative having the formula (Ia):

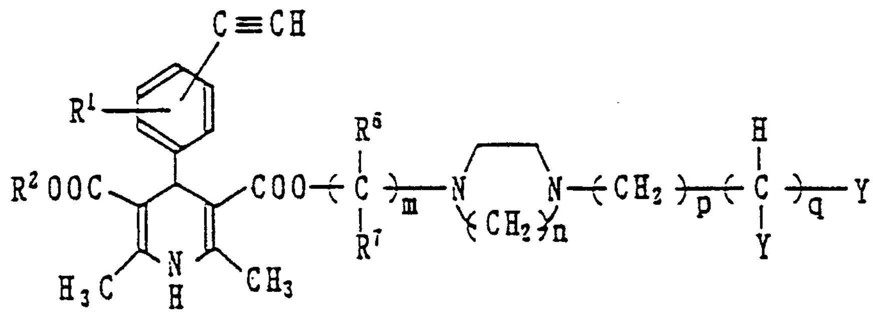 (Ia)

wherein $R^1$ is hydrogen atom or a halogen atom, $R^2$ is a lower alkyl group or a lower alkoxyalkyl group, Y is the formula:

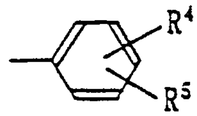

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

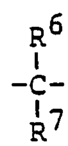

can be the same or different from each other, n is an integer of 2 or 3, p is an integer of 0 to 2 and q is an integer of 0 or 1, or a pharmacologically acceptable salt thereof, which comprises reacting an ethynylbenzaldehyde derivative having the formula (II):

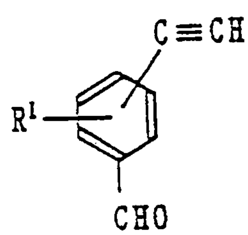 (II)

wherein $R^1$ is hydrogen atom or a halogen atom, a keto-ester derivative having the formula (III):

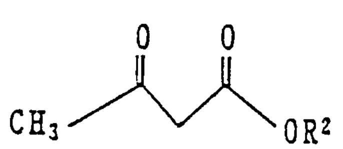 (III)

wherein $R^2$ is a lower alkyl group or a lower alkoxyalkyl group and an aminocrotonic acid derivative having the formula (IV):

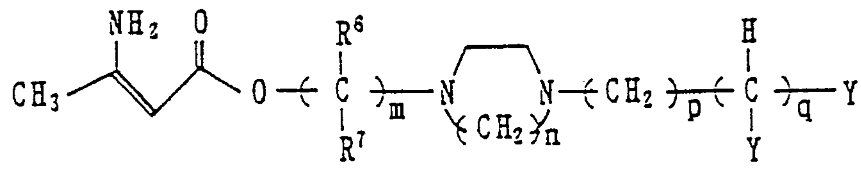

(IV)

wherein Y is the formula:

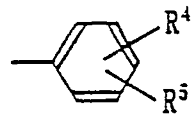

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

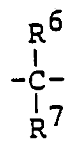

can be the same or different from each other, n is an integer of 2 or 3, p is an integer of 0 to 2 and q is an integer of 0 or 1, in an organic solvent.

**9.** A process for preparing an ethynylphenyl derivative having the formula (Ia):

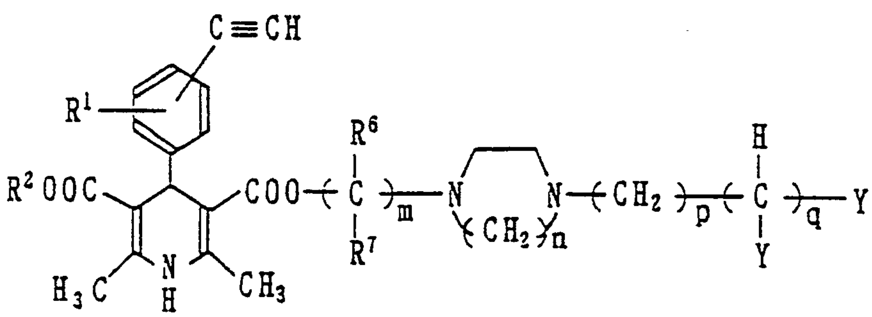

(Ia)

wherein $R^1$ is hydrogen atom or a halogen atom, $R^2$ is a lower alkyl group or a lower alkoxyalkyl group, Y is the formula:

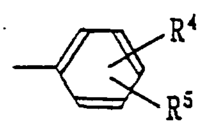

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

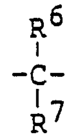

can be the same or different from each other, n is an integer of 2 or 3, p is an integer of 0 to 2 and q is an integer of 0 or 1, or a pharmacologically acceptable salt thereof, which comprises reacting an ethynylbenzaldehyde derivative having the formula (II):

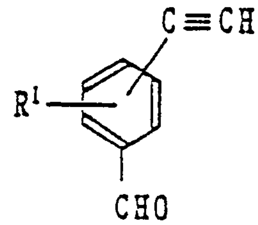

(II)

wherein $R^1$ is hydrogen atom or a halogen atom, an aminocrotonic acid derivative having the formula (V):

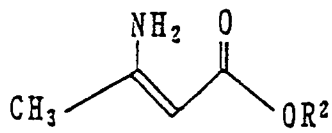

(V)

wherein $R^2$ is a lower alkyl group or a lower alkoxyalkyl group and a keto-ester derivative having the formula (VI):

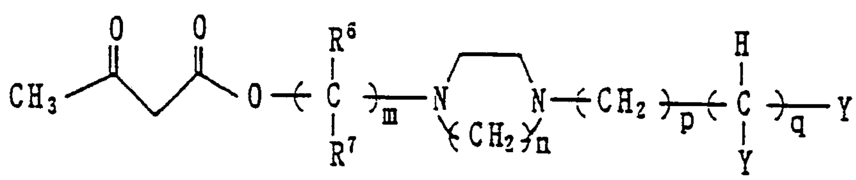

(VI)

wherein Y is the formula:

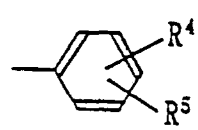

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

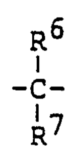

can be the same or different from each other, n is an integer of 2 or 3, p is an integer of 0 to 2 and q is an integer of 0 or 1, in an organic solvent.

**10.** A process for preparing an ethynylphenyl derivative having the formula (Ia):

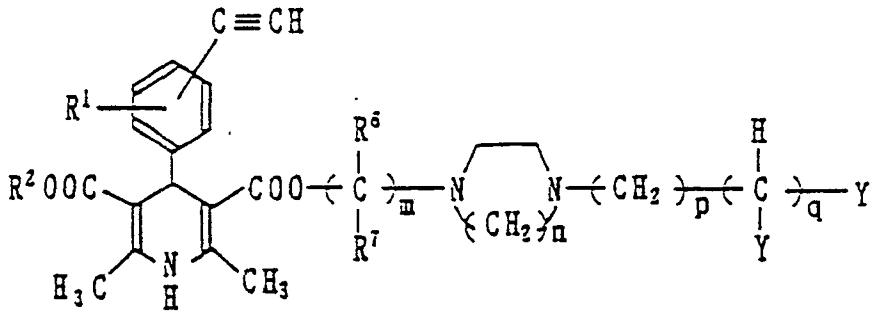

(Ia)

wherein $R^1$ is hydrogen atom or a halogen atom, $R^2$ is a lower alkyl group or a lower alkoxyalkyl group, Y is the formula:

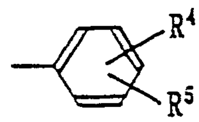

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

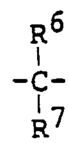

can be the same or different from each other, n is an integer of 2 or 3, p is an integer of 0 to 2 and q is an integer of 0 or 1, or a pharmacologically acceptable salt thereof, which comprises reacting a benzylidene derivative having the formula (VII):

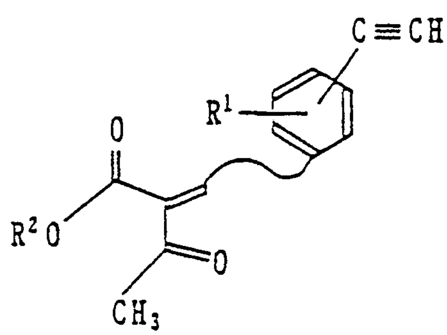

(VII)

wherein $R^1$ is hydrogen atom or a halogen atom and $R^2$ is a lower alkyl group or a lower alkoxyalkyl group and an aminocrotonic acid derivative having the formula (IV):

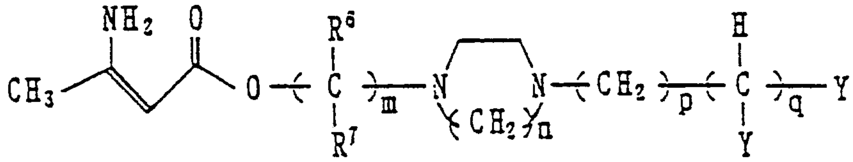 (IV)

wherein Y is the formula:

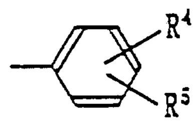

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

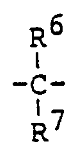

can be the same or different from each other, n is an integer of 2 or 3, p is an integer of 0 to 2 and q is an integer of 0 or 1, in a suitable organic solvent.

**11.** A process for preparing an ethynylphenyl derivative having the formula (Ia):

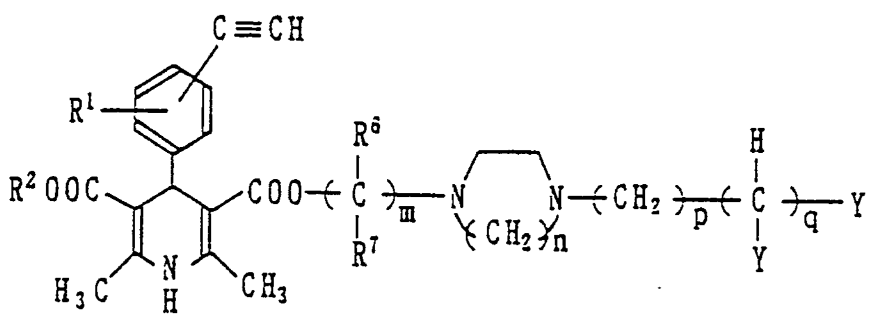 (I a)

wherein $R^1$ is hydrogen atom or a halogen atom, $R^2$ is a lower alkyl group or a lower alkoxyalkyl group, Y is the formula:

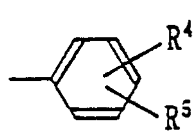

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

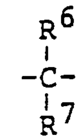

can be the same or different from each other, n is an integer of 2 or 3, p is an integer of 0 to 2 and q is an integer of 0 or 1, or a pharmacologically acceptable salt thereof, which comprises reacting an aminocrotonic acid derivative having the formula (V):

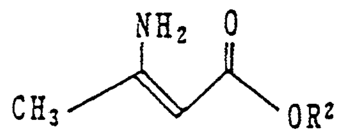

(V)

wherein $R^2$ is a lower alkyl group or a lower alkoxyalkyl group and a benzylidene derivative having the formula (VIII):

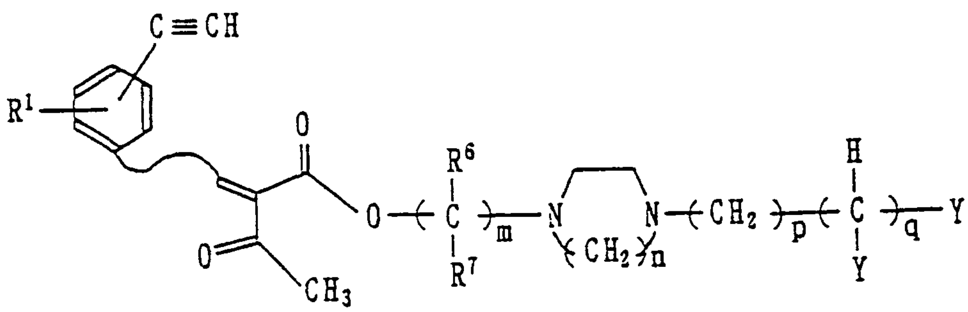

(VIII)

wherein $R^1$ is hydrogen atom or a halogen atom, Y is the formula:

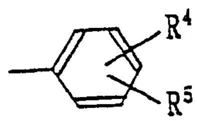

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

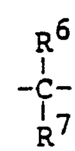

can be the same or different from each other, n is an integer of 2 or 3, p is an integer of 0 to 2 and q is an integer of 0 or 1, in a suitable organic solvent.

**12.** A process for preparing an ethynylphenyl derivative having the formula (Ib):

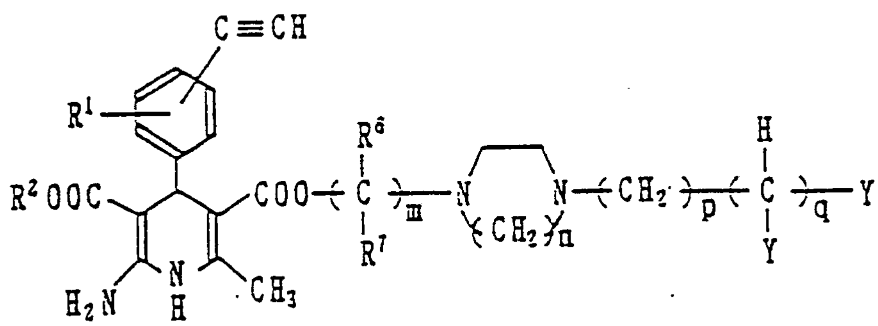

(Ib)

wherein $R^1$ is hydrogen atom or a halogen atom, $R^2$ is a lower alkyl group or a lower alkoxyalkyl group, Y is the formula:

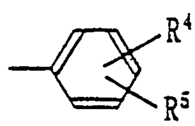

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

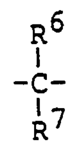

can be the same or different from each other, n is an integer of 2 or 3, p is an integer of 0 to 2 and q is an integer of 0 or 1, or a pharmacologically acceptable salt thereof, which comprises reacting an ethynylbenzaldehyde derivative having the formula (II):

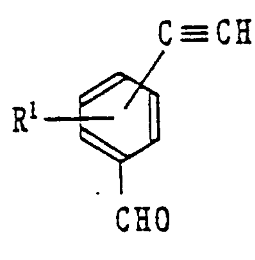

(II)

wherein $R^1$ is hydrogen atom or a halogen atom, an amidine derivative having the formula (IX):

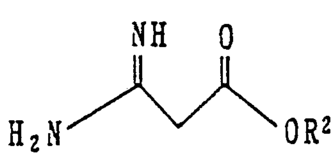

(IX)

wherein $R^2$ is a lower alkyl group or a lower alkoxyalkyl group and a keto-ester derivative having the formula (VI):

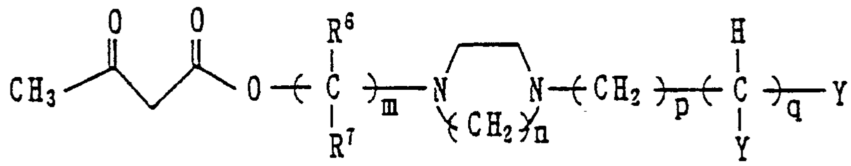 (VI)

wherein Y is the formula:

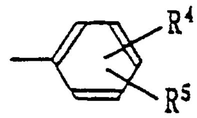

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

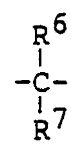

can be the same or different from each other, n is an integer of 2 or 3, p is an integer of 0 to 2 and q is an integer of 0 or 1, in an organic solvent.

**13.** A process for preparing an ethynylphenyl derivative having the formula (Ib):

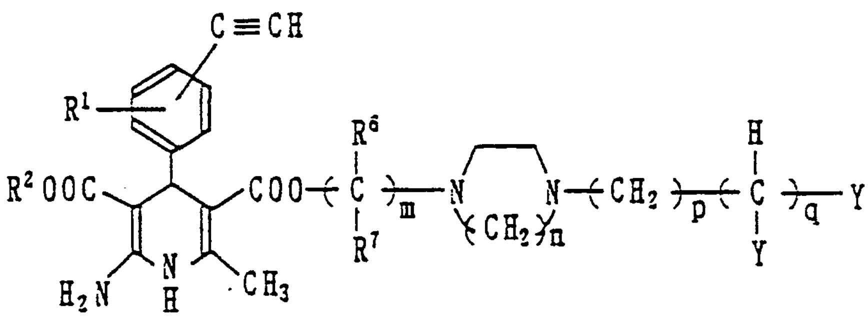 (Ib)

wherein $R^1$ is hydrogen atom or a halogen atom, $R^2$ is a lower alkyl group or a lower alkoxyalkyl group, Y is the formula:

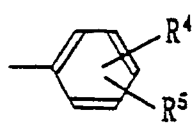

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

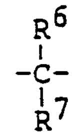

can be the same or different from each other, n is an integer of 2 or 3, p is an integer of 0 to 2 and q is an integer of 0 or 1, or a pharmacologically acceptable salt thereof, which comprises reacting an amidine derivative having the formula (IX):

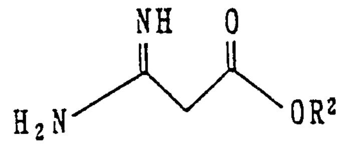

(IX)

wherein $R^2$ is a lower alkyl group or a lower alkoxyalkyl group and a benzylidene derivative having the formula (VIII):

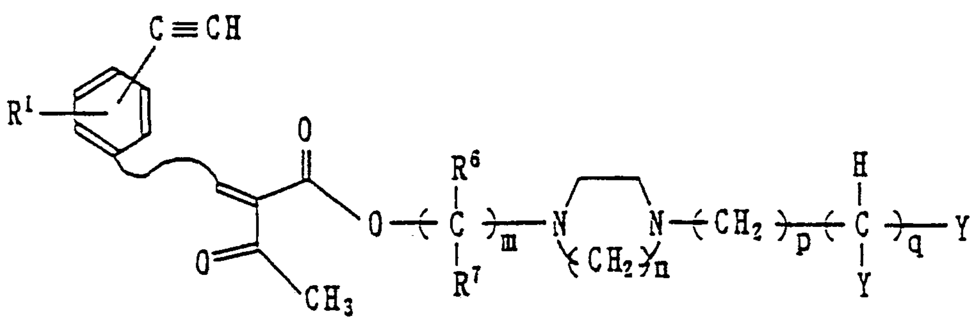

(VIII)

wherein $R^1$ is hydrogen atom or a halogen atom, Y is the formula:

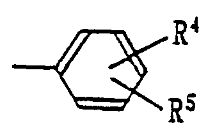

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

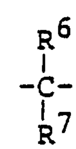

can be the same or different from each other, n is an integer of 2 or 3, p is an integer of 0 to 2 and q is an integer of 0 or 1, in a suitable organic solvent.

**14.** A process for preparing an ethynylphenyl derivative having the formula (Ic):

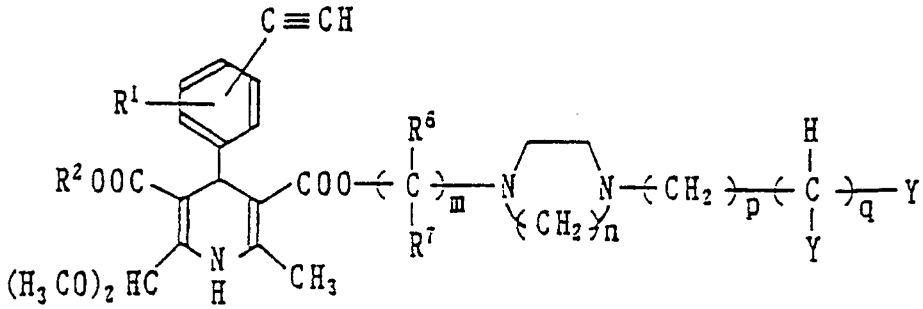

(Ic)

wherein $R^1$ is hydrogen atom or a halogen atom, $R^2$ is a lower alkyl group or a lower alkoxyalkyl group, Y is the formula:

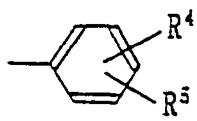

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

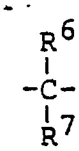

can be the same or different from each other, n is an integer of 2 or 3, p is an integer of 0 to 2 and q is an integer of 0 or 1, or a pharmacologically acceptable salt thereof, which comprises reacting an ethynylbenzaldehyde having the formula (II):

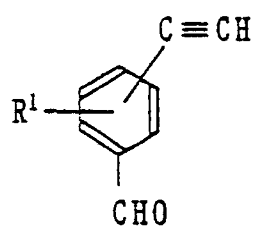

(II)

wherein $R^1$ is hydrogen atom or a halogen atom, an aminocrotonic acid derivative having the formula (IV):

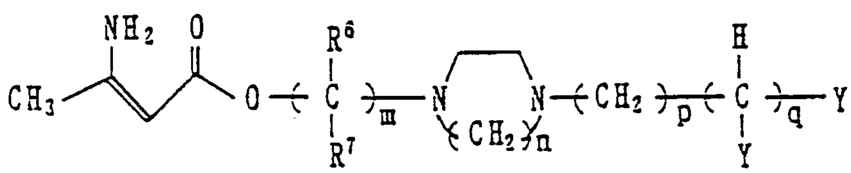

(IV)

wherein Y is the formula:

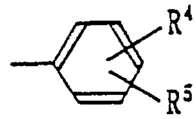

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

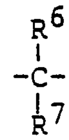

can be the same or different from each other, n is an integer of 2 or 3, p is an integer of 0 to 2 and q is an integer of 0 or 1, and an acetal keto-ester derivative having the formula (X):

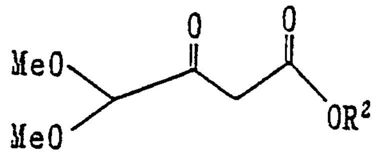

(X)

wherein $R^2$ is a lower alkyl group or a lower alkoxyalkyl group in a suitable organic solvent.

**15.** A process for preparing an ethynylphenyl derivative having the formula (Ic):

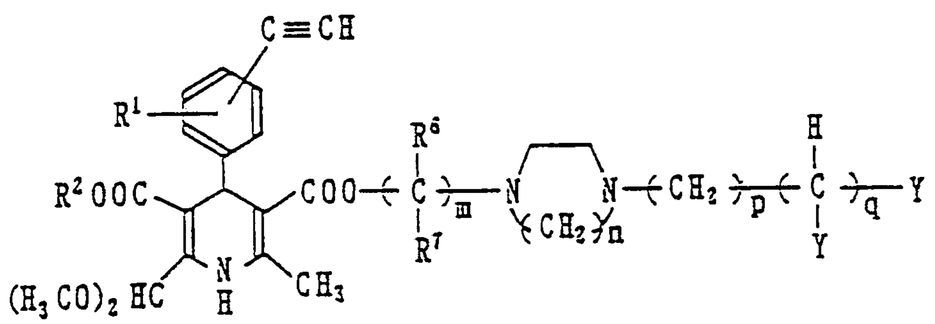

(Ic)

wherein $R^1$ is hydrogen atom or a halogen atom, $R^2$ is a lower alkyl group or a lower alkoxyalkyl group, Y is the formula:

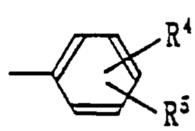

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

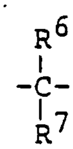

can be the same or different from each other, n is an integer of 2 or 3, p is an integer of 0 to 2 and q is an integer of 0 or 1, or a pharmacologically acceptable salt thereof, which comprises reacting a benzylidene derivative having the formula (XI):

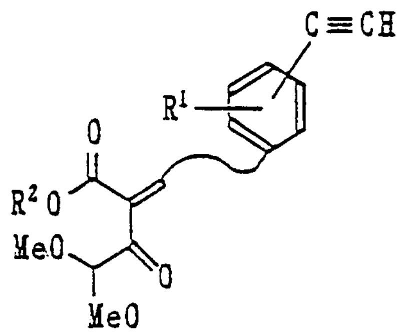

(XI)

wherein $R^1$ is hydrogen atom or a halogen atom and $R^2$ is a lower alkyl group or a lower alkoxyalkyl group and an aminocrotonic acid having the formula (IV):

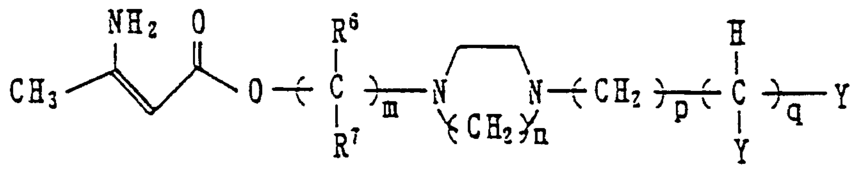

(IV)

wherein Y is the formula:

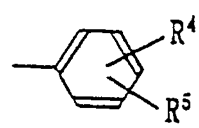

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

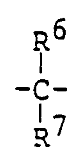

can be the same or different from each other, n is an integer of 2 or 3, p is an integer of 0 to 2 and q is an integer of 0 or 1, in a suitable organic solvent.

**16.** A process for preparing an ethynylphenyl derivative having the formula (Id):

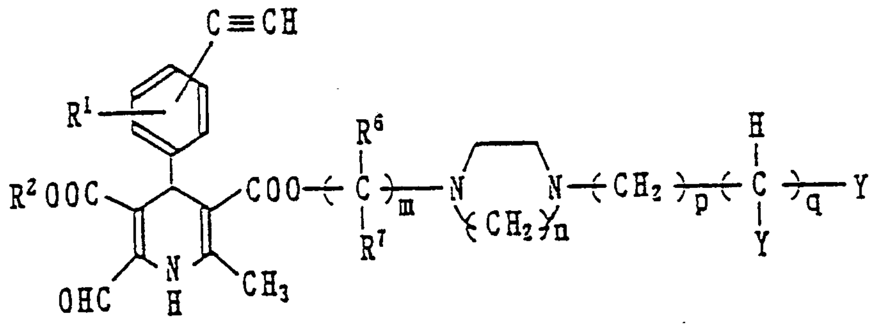

(Id)

wherein $R^1$ is hydrogen atom or a halogen atom, $R^2$ is a lower alkyl group or a lower alkoxyalkyl group, Y is the formula:

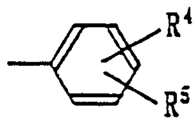

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

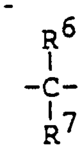

can be the same or different from each other, n is an integer of 2 or 3, p is an integer of 0 to 2 and q is an integer of 0 or 1, or a pharmacologically acceptable salt thereof, which comprises hydrolyzing an ethynylphenyl derivative having the formula (Ic):

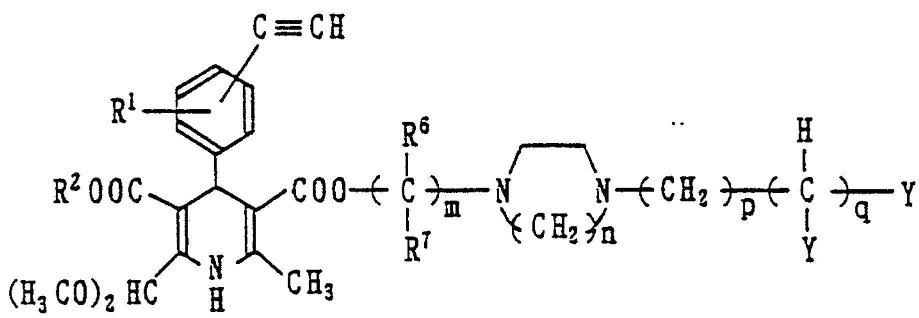

(Ic)

wherein $R^1$ is hydrogen atom or a halogen atom, $R^2$ is a lower alkyl group or a lower alkoxyalkyl group, Y is the formula:

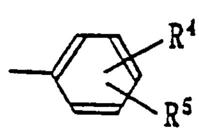

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

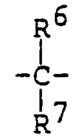

can be the same or different from each other, n is an integer of 2 or 3, p is an integer of 0 to 2 and q is an integer of 0 or 1.

**17.** A process for preparing an ethynylphenyl derivative having the formula (Ie):

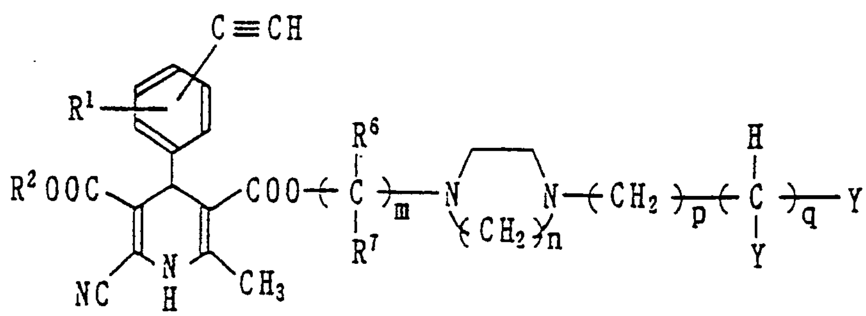

(Ie)

wherein $R^1$ is hydrogen atom or a halogen atom, $R^2$ is a lower alkyl group or a lower alkoxyalkyl groups, Y is the formula:

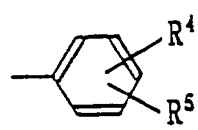

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

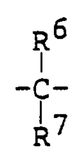

can be the same or different from each other, n is an integer of 2 or 3, p is an integer of 0 to 2 and q is an integer of 0 or 1, or a pharmacologically acceptable salt thereof, which comprises converting an ethynylphenyl derivative having the formula (Id):

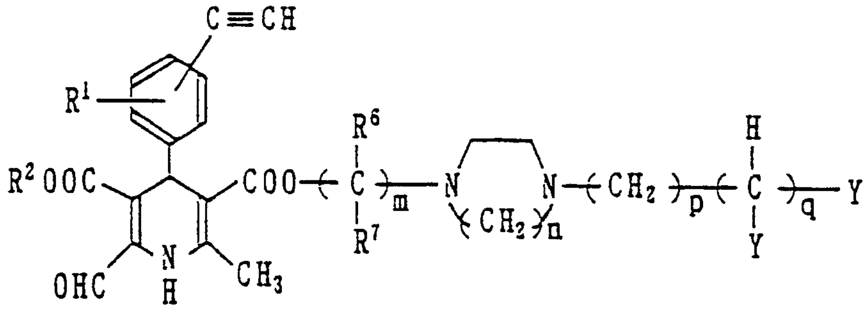

(Id)

wherein $R^1$ is hydrogen atom or a halogen atom, $R^2$ is a lower alkyl group or a lower alkoxyalkyl group, Y is the formula:

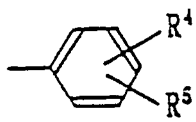

wherein $R^4$ and $R^5$ are the same or different from each other, hydrogen atom, a halogen atom, a lower alkyl group or a lower alkoxy group, $R^6$ and $R^7$ are the same or different from each other, hydrogen atom or a lower alkyl group, m is an integer of 2 to 4 provided that m groups of

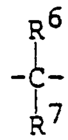

can be the same or different from each other, n is an integer of 2 or 3, p is an integer of 0 to 2 and q is an integer of 0 or 1, into an oxime and subsequently subjecting to dehydration.

**18.** A medicament for circulatory disease which comprises the ethynylphenyl derivative or a pharmacologically acceptable salt thereof of Claim 1 as an effective ingredient.

**19.** A medicament for circulatory disease which comprises an effective amount of the ethynylphenyl derivative or a pharmacologically acceptable salt thereof of Claim 1 and pharmacologically acceptable additives.

FIG. 1

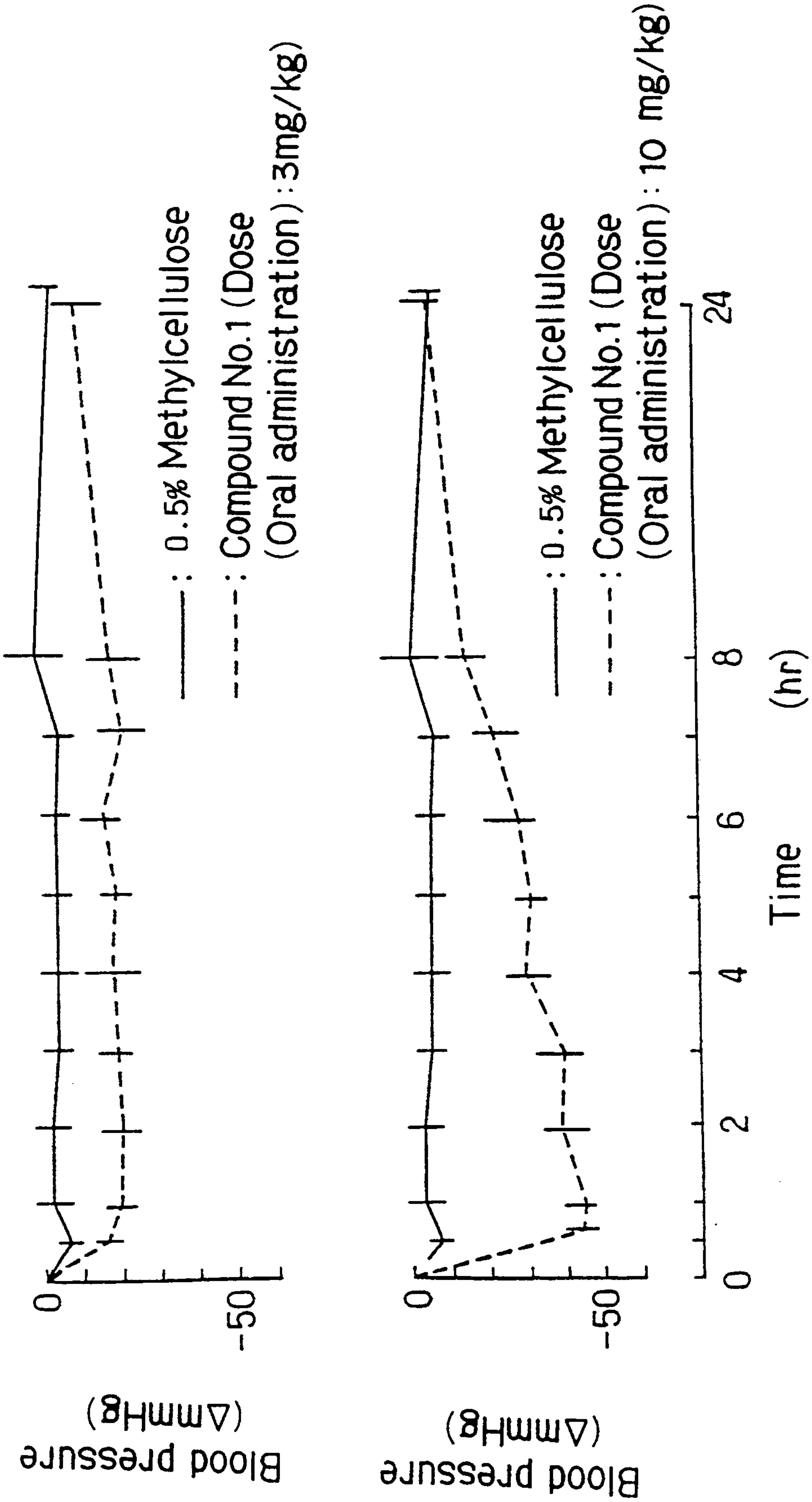

Blood pressure (ΔmmHg)
0
-50
—— : 0.5% Methylcellulose
- - - : Compound No.1 (Dose (Oral administration) : 3mg/kg)
Blood pressure (ΔmmHg)
0
-50
—— : 0.5% Methylcellulose
- - - : Compound No.1 (Dose (Oral administration) : 10 mg/kg)
0
2
4
6
8
24
Time (hr)

FIG.2

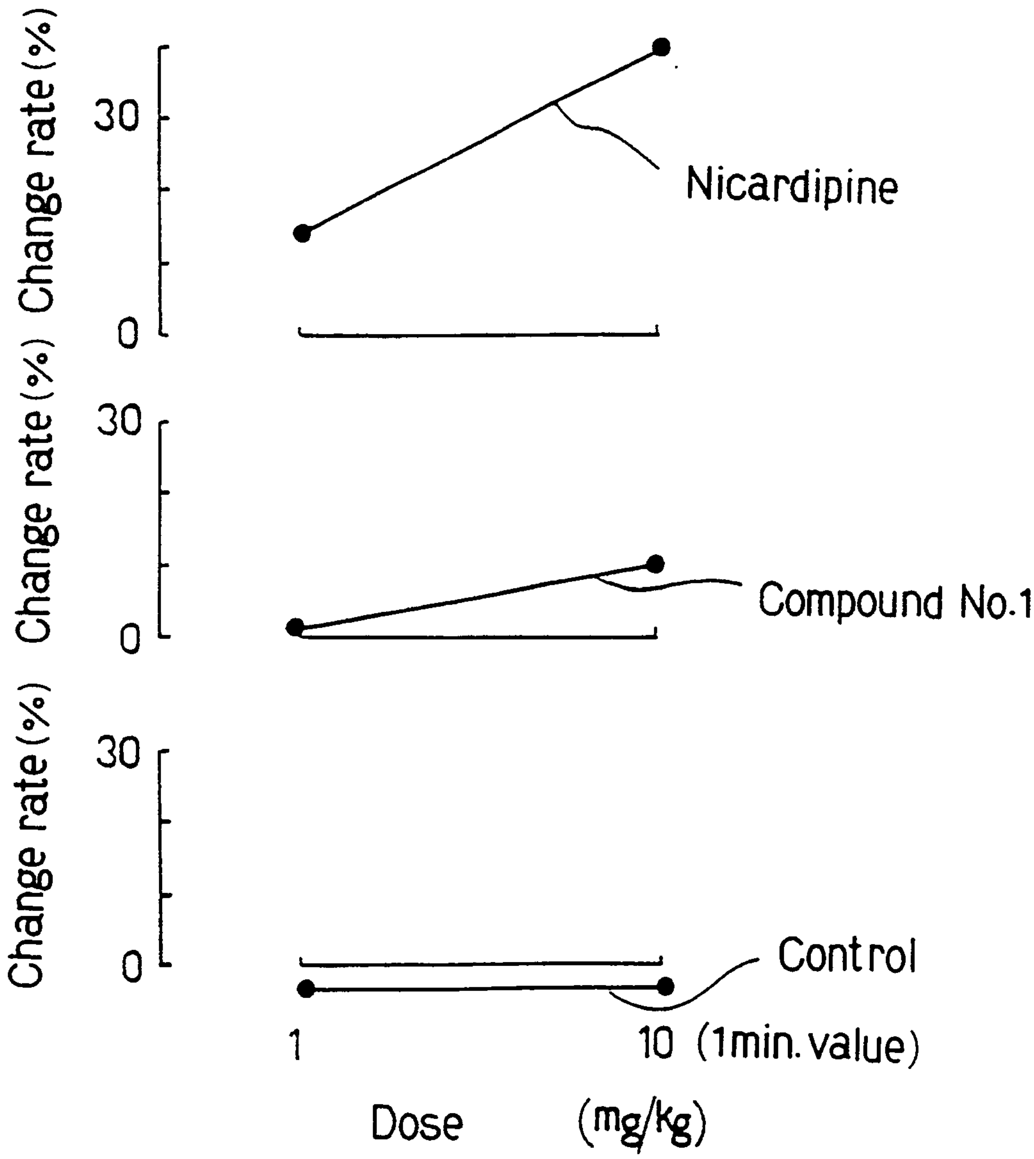
Change rate (%)
30
0
Nicardipine
Change rate (%)
30
0
Compound No.1
Change rate (%)
30
0
Control
1
10 (1min. value)
Dose (mg/kg)

FIG.3

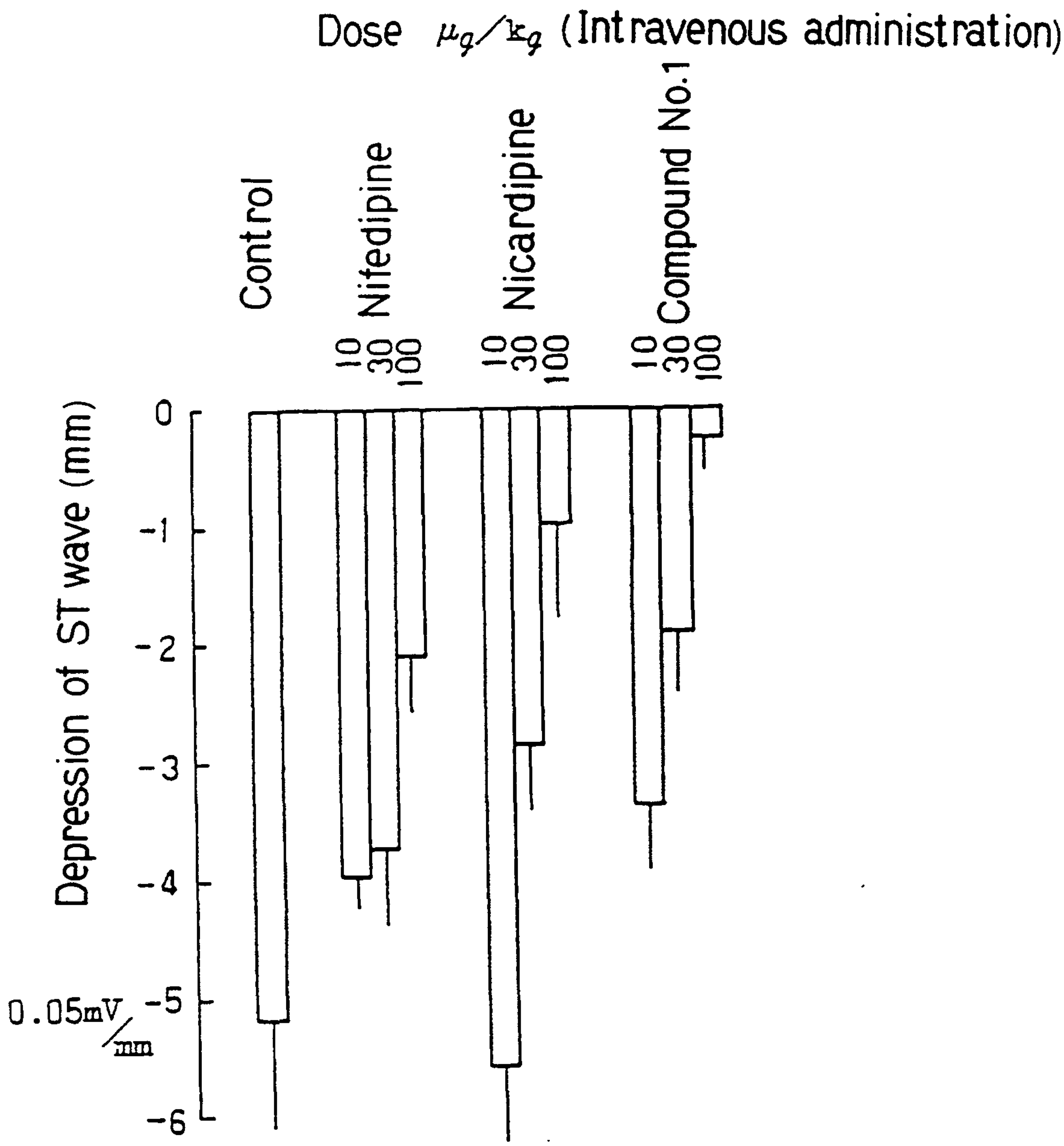
Dose μg/kg (Intravenous administration)
Control
Nifedipine
Nicardipine
Compound No.1
10
30
100
10
30
100
10
30
100
Depression of ST wave (mm)
0
-1
-2
-3
-4
-5
-6
0.05mV/mm

FIG. 4

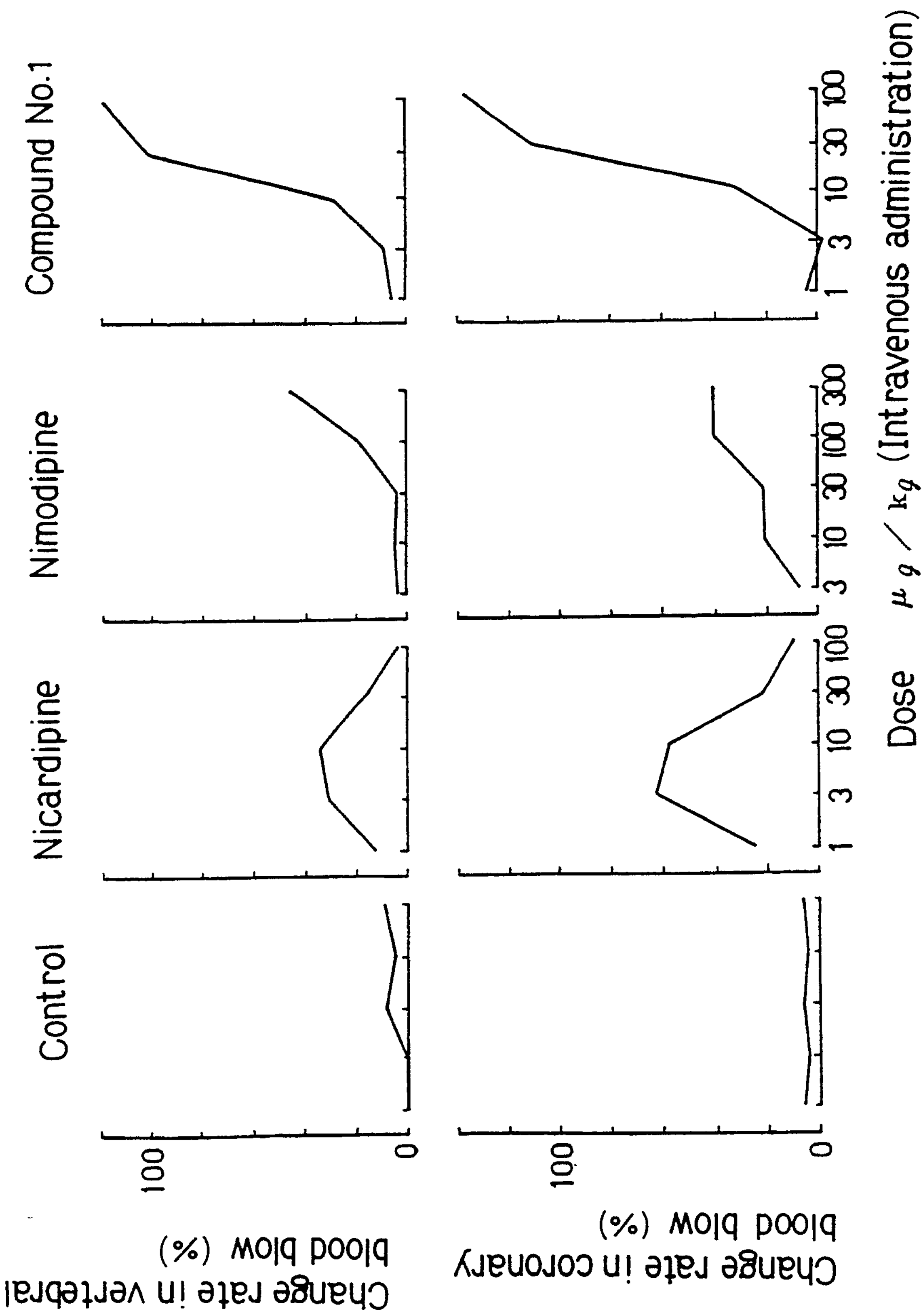
Control
Nicardipine
Nimodipine
Compound No.1
Change rate in vertebral blood blow (%)
Change rate in coronary blood blow (%)
100
0
1 3 10 30 100
3 10 30 100 300
Dose μg / kg (Intravenous administration)

# INTERNATIONAL SEARCH REPORT

International Application No PCT/JP90/01682

**I. CLASSIFICATION OF SUBJECT MATTER** (if several classification symbols apply, indicate all) [6]

According to International Patent Classification (IPC) or to both National Classification and IPC

Int. $Cl^5$ C07D211/90, 401/12, A61K31/455, 31/55

**II. FIELDS SEARCHED**

Minimum Documentation Searched [7]

| Classification System [8] | Classification Symbols |
| --- | --- |
| IPC | C07D211/90, 401/12, A61K31/435-31/455, 31/55 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
| --- | --- | --- |
| A | JP, A, 50-40576 (Science Union et Cie), April 14, 1975 (14. 04. 75) & GB, A, 1,409,865 | 1-19 |
| A | JP, A, 58-159462 (Yoshitomi Pharmaceutical Industries, Ltd.), September 21, 1983 (21. 09. 83) & EP, A, 88903 | 1-19 |
| A | JP, A, 58-201765 (Takeda Chemical Industries, Ltd.), November 24, 1983 (24. 11. 83) & EP, A, 94159 | 1-19 |
| A | JP, A, 59-16876 (Pierrel S.p.A.), January 28, 1984 (28. 01. 84) & EP, A, 97821 | 1-19 |
| A | JP, A, 60-48970 (Takeda Chemical Industries, Ltd.), March 16, 1985 (16. 03. 85), (Family: none) | 1-19 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
| --- | --- |
| March 13, 1991 (13. 03. 91) | March 25, 1991 (25. 03. 91) |
| **International Searching Authority** | **Signature of Authorized Officer** |
| Japanese Patent Office | |

International Application No PCT/JP90/01682

**FURTHER INFORMATION CONTINUED FROM THE SECOND SHEET**

| | | |
|---|---|---|
| A | JP, A, 61-17562 (Bristol-Myers Co.) January 25, 1986 (25. 01. 86) & DE, A 3,512,995 & GB, A, 2,158,065 | 1-19 |
| A | JP, A, 61-140567 (Otuka Pharmaceutical Co., Ltd.) June 27, 1986 (27. 06. 86), (Family: none) | 1-19 |
| A | JP, A, 62-252768 (Glaxo S.p.A.), November 4, 1987 (04. 11. 87) & EP, A, 245918 | 1-19 |
| A | JP, A, 63-91366 (Kyoto Pharmaceutical Industries, Ltd.) | 1-19 |

**V.☐ OBSERVATIONS WHERE CERTAIN CLAIMS WERE FOUND UNSEARCHABLE [1]**

This international search report has not been established in respect of certain claims under Article 17(2) (a) for the following reasons.

1.☐ Claim numbers because they relate to subject matter not required to be searched by this Authority, namely

2.☐ Claim numbers , because they relate to parts of the international application that do not comply with the prescribed requirements to such an extent that no meaningful international search can be carried out, specifically

3.☐ Claim numbers , because they are dependent claims and are not drafted in accordance with the second and third sentences of PCT Rule 6.4(a)

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application

2.☐ As only some of the required additional search fees were timely paid by the applicant, this international search report covers only those claims of the international application for which fees were paid, specifically claims

3.☐ No required additional search fees were timely paid by the applicant. Consequently, this international search report is restricted to the invention first mentioned in the claims; it is covered by claim numbers

4.☐ As all searchable claims could be searched without effort justifying an additional fee, the International Searching Authority did not invite payment of any additional fee.

Remark on Protest

☐ The additional search fees were accompanied by applicant's protest

☐ No protest accompanied the payment of additional search fees

International Application No. PCT/JP90/01682

**FURTHER INFORMATION CONTINUED FROM THE SECOND SHEET**

| | | |
|---|---|---|
| | April 22, 1988 (22. 04. 88), (Family: none) | |
| A | JP, A, 1-238569 (The Green Cross Corp.), September 22, 1989 (22. 09. 89), (Family: none) | 1-19 |

**V.☐ OBSERVATIONS WHERE CERTAIN CLAIMS WERE FOUND UNSEARCHABLE [1]**

This international search report has not been established in respect of certain claims under Article 17(2) (a) for the following reasons.

1.☐ Claim numbers because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.